# EUROPEAN PATENT SPECIFICATION

(11) **EP 1 235 595 B1**
(45) Date of publication and mention of the grant of the patent: **05.08.2009**
(21) Application number: 00965901.2
(22) Date of filing: 28.08.2000
(51) Int. Cl.: A61K 47/48

(54) **INTEGRIN-MEDIATED DRUG TARGETING**
INTEGRIN-VERMITTELTE ARZNEIMITTEL-ZIELRICHTUNG
CIBLAGE DE MEDICAMENT INDUIT PAR L'INTEGRINE

(30) Priority: 08.09.1999 US 392167; 29.06.2000 US 606772
(43) Date of publication of application: 04.09.2002
(73) Proprietor: Albers, Markus, 51375 Leverkusen (DE); Baumgarten, Jörg, 42115 Wuppertal (DE); Brüggemeier, Ulf, 42799 Leichlingen (DE); Lerchen, Hans-Georg, 51375 Leverkusen (DE)
(72) Inventor: LERCHEN, Hans-Georg, 51375 Leverkusen (DE); BAUMGARTEN, Jörg, 42115 Wuppertal (DE); BRÜGGEMEIER, Ulf, 42799 Leichlingen (DE); ALBERS, Markus, 51375 Leverkusen (DE); SCHOOP, Andreas, AT/1150 Wien (AT); SCHULZE, Thomas-J, Thousands Oaks, CA 91360 (US)
(86) International application number: PCT/EP2000/008361
(87) International publication number: WO 2001/017563

(56) References cited:
- WO-A-93/20229
- WO-A-97/39021
- KERR J S ET AL: "NOVEL SMALL MOLECULE ALPHAV INTEGRIN ANTAGONISTS: COMPARATIVE ANTI-CANCER EFFICACY WITH KNOWN ANGIOGENESIS INHIBITORS" ANTICANCER RESEARCH,HELENIC ANTICANCER INSTITUTE, ATHENS,,GR, vol. 19, no. 2A, 1999, pages 959-968, XP000924849 ISSN: 0250-7005
- FIELDS ET AL: "Integrins: cell adhesion molecules in cancer" EXPERT OPINION ON THERAPEUTIC PATENTS,GB,ASHLEY PUBLICATIONS, vol. 8, no. 6, August 1998 (1998-08), pages 633-644, XP002105988 ISSN: 1354-3776
- CARRON C P ET AL: "A PEPTIDOMIMETIC ANTAGONIST OF THE INTEGRIN ALPHA V BETA 3 INHIBITS LEYDIG CELL TUMOR GROWTH AND THE DEVELOPMENT OF HYPERCALCEMIA OF MALIGNANCY" CANCER RESEARCH,US,AMERICAN ASSOCIATION FOR CANCER RESEARCH, BALTIMORE, MD, vol. 58, May 1998 (1998-05), pages 1930-1935, XP000915219 ISSN: 0008-5472
- NICKOLS A ET AL: "ANTIANGIOGENIC AND ANTICANCER ACTIVITIES OF ANTAGONISTS OF INTEGRIN ALPHAVBETA3" PROCEEDINGS OF THE ANNUAL MEETING OF THE AMERICAN ASSOCIATION FOR CANCER RESEARCH,US,PHILADELPHIA, AACR, vol. 38, 1 March 1997 (1997-03-01), page 206, abstract no. 1389 XP002071773
- T J MACDONALD ET AL: "Migration of human brain tumor cells and human brain endothelial cells on tenascin requires the integrin alphaVbeta3;a unifying model for brain tumor invasion and angiogenesis" PROC. ANNU MEET. AACR, vol. 39, 28 March 1998 (1998-03-28), page 497, abstract no. 3382 XP002141915 NEW ORLEANS, LA, MARCH 28 - APRIL 1, 1998,PHILADELPHIA, US
- P C BROOKS ET AL: "Requirement of vascular integrin alphaV beta3 for angiogenesis" SCIENCE ,AAAS, LANCASTER, PA,US, vol. 264, 22 April 1994 (1994-04-22), pages 569-571, XP002138524 ISSN: 0036-8075
- BROOKS P C ET AL: "INTEGRIN ALPHAVBETA3 ANTAGONISTS PROMOTE TUMOR REGRESSION BY INDUCING APOPTOSIS OF ANGIOGENIC BLOOD VESSELS" CELL,US,CELL PRESS, CAMBRIDGE, NA, vol. 79, no. 7, 30 December 1994 (1994-12-30), pages 1157-1164, XP000652129 ISSN: 0092-8674
- VARNER J A ET AL: "TUMOR ANGIOGENESIS AND THE ROLE OF VASCULAR CELL INTEGRIN ALPHAVBETA3" IMPORTANT ADVANCES IN ONCOLOGY,LIPPINCOT-RAVEN, PHILADELPHIA,US, 1996, pages 69-87, XP000857371 ISSN: 0883-5896
- VARNER J A ET AL: "INTEGRINS AND CANCER" CURRENT OPINION IN CELL BIOLOGY,GB,CURRENT SCIENCE, LONDON, vol. 8, no. 5, October 1996 (1996-10), pages 724-730, XP000857374 ISSN: 0955-0674
- VARNER J A ET AL: "REVIEW: THE INTEGRIN ALPHAVBETA3: ANGIOGENESIS AND APOPTOSIS" CELL ADHESION AND COMMUNICATION,US,HARWOOD ACADEMIC PUBLISHERS, GMBH, YVERDON, vol. 3, no. 4, 1995, pages 367-374, XP000857373 ISSN: 1061-5385
- GASPARINI G.: "The rationale and future potential of angiogenesis inhibitors in neoplasia" DRUGS, vol. 58, no. 1, July 1999 (1999-07), pages 17-38, XP001002355
- DAMIANO JS ET AL: "Cell adhesion mediated drug resistance (CAM-DR): role of integrins and resistance to apoptosis in human myeloma cell lines." BLOOD, vol. 93, no. 5, 1 March 1999 (1999-03-01), pages 1658-1667, XP002168941
- UHM, JOON H. ET AL: "Vitronectin, a glioma-derived extracellular matrix protein, protects tumor cells from apoptotic death" CLIN. CANCER RES., 06-1999, VOL. 5, NO. 6, PAGE(S) 1587-1594, XP002168942
- MUELLER, B. M. ET AL: "Pre-clinical therapy of human melanoma with morpholino-doxorubicin conjugated to a monoclonal antibody directed against an integrin on melanoma cells" ANTIBODY, IMMUNOCONJUGATES, RADIOPHARM., 1991, VOL. 4, NO. 2, PAGE(S) 99-106, XP001002266
- BITAN GAL ET AL: "Mapping the integrin alphaVbeta3-ligand interface by photoaffinity cross-linking." BIOCHEMISTRY, vol. 38, no. 11, 16 March 1999 (1999-03-16), pages 3414-3420, XP002168943 ISSN: 0006-2960
- SHEU JOEN R ET AL: "Triflavin, an arg-gly-asp-containing peptide, inhibits the adhesion of tumor cells to matrix proteins via binding to multiple integrin receptors expressed on human hepatoma cells." PROCEEDINGS OF THE SOCIETY FOR EXPERIMENTAL BIOLOGY AND MEDICINE, vol. 213, no. 1, 1996, pages 71-79, XP001002230 ISSN: 0037-9727
- Hart et al. (1994) JBC 269, 12468-12474.

## Description

The marked lectin pattern on tumour cell surfaces (Gabius; Onkologie 12, (1989), 175) opens up the fundamental possibility of addressing these specifically on tumour cells by linkage of appropriate carbohydrate units to cytostatics. This prospect is restricted by the fact that, even in other tissues, in particular in the liver, lectins having similar carbohydrate specificities (galactose, lactose, mannose, N-acetylglucosamine, fucose etc.) occur (Ashwell et al., Annu. Rev. Biochem. 46 (1982), 531; Stahl et al. Proc. Natl. Acad. Sci. USA 74 (1977), 1521; Hill et al., J. Biol. Chem. 262 (1986), 7433; Jansen et al., J. Biol. Chem. 266 (1991), 3343). Accordingly, a marked concentration of active compound-containing glycoconjugates in the liver and other lectin-rich organs must be expected if, in this approach, carbohydrates are used without particular modification establishing a selectivity to tumour tissue.

The heterocyclic amine batracylin (1) shows a good anti tumour action in various stomach cancer models (US-4 757 072).

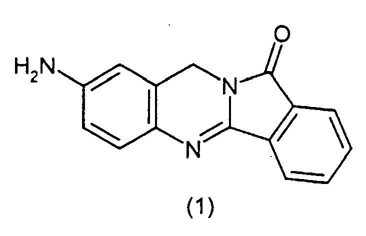

Peptide conjugates of (1) having good in-vitro action and more favourable solubility properties (US-4 180 343) are more poorly tolerable in animal experiments than free batracylin. The fucose conjugates of batracylin (1) described in EP-A-0 501 250 disadvantageously concentrate very strongly in the liver.

Quinolone-a (2), 7-[(3a-R,S, 4-R,S, 7a-S,R)-4-amino-1,3,3a,4,7,7a-hexahydro-iso-in-dol-2-yl]-8-chloro-1-cyclopropyl-6-fluoro-1,4-dihydro-4-oxo-3-quinolinecarboxylic acid, also shows, in addition to its outstanding antibacterial activity, a very good activity against various tumour cell lines (EP-A-0 520 240, JP-4 253 973). However, considerable toxicological problems face it (e.g. genotoxicity, bone marrow toxicity, high acute toxicity in vivo etc.).

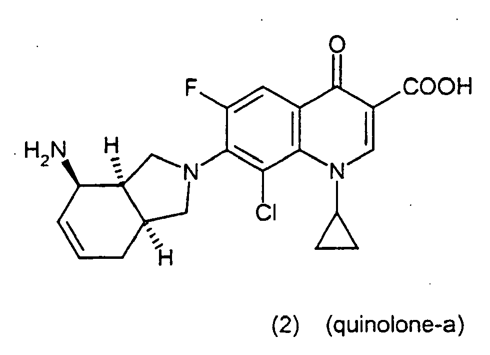

20(S)-Camptothecin is a pentacyclic alkaloid which was isolated in 1966 by Wall et al. (J. Am. Chem. Soc. 88, 3888 (1966)). It has a high active antitumour potential in numerous in-vitro and in-vivo tests. Unfortunately, however, the realization of the promising potential in the clinical investigation phase failed because of toxicity and solubility problems.

By opening of the E ring lactone and formation of the sodium salt, a water-soluble compound was obtained which is in a pH-dependent equilibrium with the ring-closed form. Here too, clinical studies have not led to success as yet.

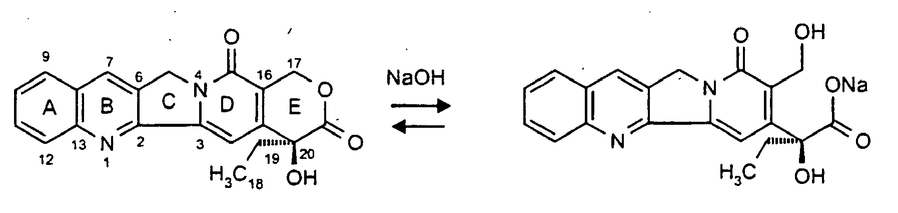

About 20 years later, it was found that the biological activity is to be attributed to enzyme inhibition of topoisomerase I. Since then, the research activities have again been increased in order to find a camptothecin derivative which is more tolerable and which is active in vivo.

For improvement of the water solubility, salts of A ring- and B ring-modified camptothecin derivatives and of 20-O-acyl derivatives with ionizable groups have been described (Vishnuvajjala et al. US 4 943 579). The latter prodrug concept was later also transferred to modified camptothecin derivatives (Wani et al. WO 9602546).
The described 20-O-acyl prodrugs, however, have a very short half-life in vivo and are very rapidly cleaved to give the parent structure.

WO 96/31532 describes carbohydrate-modified cytostatics in which both serum stability and release of the cytostatic within the tumour cells and a specific concentration of the cytostatic in tumour tissue is achieved by a novel linkage of selectively modified carbohydrates to cytostatics (for example batracylin, quinolone-a, camptothecin) via preferred spacer and linker groups.

Integrins are heterodimeric transmembrane proteins found on the surface of cells, which play an important part in the adhesion of the cells to an extracellular matrix.
They recognize extracellular glycoproteins such as fibronectin or vitronectin on the extracellular matrix via the RGD sequence occurring in these proteins (RGD is the single-letter code for the amino acid sequence arginine-glycine-aspartate).

In general, integrins such as, for example, the vitronectin receptor, which is also called the αᵥβ₃ receptor, or alternatively the αᵥβ₅ receptor or the GpIIb/IIIa receptor play an important part in biological processes such as cell migration, angiogenesis and cell-matrix adhesion and thus for diseases in which these processes are crucial steps. Cancer, osteoporosis, arteriosclerosis, restenosis and ophthalmia may be mentioned by way of example.

The αᵥB₃ receptor occurs, for example, in large amounts on growing endothelial cells and makes possible their adhesion to an extracellular matrix. The _{v 3} receptor thus plays an important part in angiogenesis, i.e. the formation of new blood vessels, which is a crucial prerequisite for tumour growth and metastasis formation in carcinomatous disorders.

It was possible to show that the blockade of the abovementioned receptors is an important starting point for the treatment of disorders of this type. If the adhesion of growing endothelial cells to an extracellular matrix is suppressed by blocking their corresponding integrin receptors, for example, by a cyclic peptide or a monoclonal antibody, the endothelial cells die. Angiogenesis therefore does not occur, which leads to a stoppage or regression of tumour growth (cf., for example, Brooks et al., Cell, Volume 79, 1157-1164, 1994).

Moreover, the invasive properties of tumour cells and thus their capability to form metastases markedly decrease when their αᵥβ₃ receptor is blocked by an antibody (Brooks et al., J. Clin. Invest., Volume 96, 1815, 1995).

WO 98/10795 describes conjugates in which a molecule adding to tumours is linked to a functional unit such as, for example, a cytostatic or a detectable label such as, for example, a radioactive nuclide. Inter alia, integrin antagonists such as, for example, peptides having the RGD sequence described above are described as molecules adding to tumours. Doxorubicin is described as an example of a cytostatic which is linked to a molecule of this type addressing tumours.

In the case of the compounds of WO 98/10795, the linkage is carried out such that the molecule addressing a tumour and the functional unit are directly bonded to one another with retention of their respective properties (cf., for example, p. 56, 1. 17, to p. 58, 1. 10, and Ex. 6). This has the result that these compounds are indeed selectively concentrated in the immediate vicinity of tumour cells by binding of the entity addressing a tumour (in the case of a radical having αᵥß₃ integrin-antagonistic action by binding to the αᵥß₃ integrin receptor which, in particular, is expressed on endothelial cells newly formed by angiogenesis), but on account of the direct combination the functional unit such as, for example, a cytostatic cannot be released into the intracellular space of the tumour tissue.

Fundamentally, the conjugate which on the one hand is selectively concentrated in tumour tissue by the effect of a part addressing αᵥβ₃ or αᵥβ₅ integrin receptors found in the conjugate, but on the other hand comprises a cytostatic which can be released from the conjugate, should have an increased toxophoric effect on tumour tissue due to the possibility of the more direct action of the cytostatic on the tumour cells compared with the conjugates described in WO 98/10795.

It was therefore the object of the present invention to develop conjugates which comprise a moiety addressing αᵥβ₃ or αᵥβ₅ integrin receptors and a cytostatic which can be released from the conjugate, where the moiety in the conjugate addressing αᵥβ₃ or αᵥβ₅ integrin receptors retains its ability to bind to the αᵥβ₃ or αᵥβ₅ integrin receptor.

The above object is achieved by conjugates which comprise a non-peptide moiety addressing αᵥβ₃ or αᵥβ₅ integrin receptors, a cytostatic and a linking unit which is enzymatically or hydrolytically cleavable with release of the cytostatic. Conjugates having a non-peptide moiety addressing αᵥβ₃ integrin receptors are particularly preferred here.

In principle, medicament-containing conjugates are complex, difficult-to-prepare compounds, as is explained, for example, in Anti-Cancer Drug Design 10 (1995), 1-9, in particular p. 1. In this article, conjugates of the cytostatic methotrexate, an oligopeptide spacer and a protein (human serum albumin) are described. However, it is also pointed out (cf. p. 7, first paragraph) that the nature of the linking unit and the type of linkage of this unit to the toxophore and the carrier (for example an antibody) can affect the cleavage of the linking unit. This article therefore teaches that the linkage presented there cannot be transferred to other conjugate systems without difficulty. In particular, nothing is said about whether moieties addressed also to αᵥβ₃ or αᵥβ₅ integrin receptors in this manner can be linked to toxophores without the moiety addressing aᵥβ₃ or aᵥβ₅ integrin receptors by this means losing its ability to bind to αᵥβ₃ or αᵥβ₅ integrin receptors.

The linking units disclosed in WO 96/31532 are used specifically for the linkage of a toxophore to an oligosaccharide radical. Nothing is said about whether moieties addressed also to αᵥβ₃ or αᵥβ₅ integrin receptors can be linked to toxophores in this manner, without, by this means, the moiety addressing αᵥβ₃ or αᵥβ₅ integrin receptors losing its ability to bind to αᵥβ₃ or αᵥβ₅ integrin receptors.

According to a preferred embodiment of the present invention, the linking unit can be cleaved by tumour-associated enzymes. This leads to a further increase in the tissue specificity of the conjugates according to the invention and thus to an additional decrease of the conjugates according to the invention in other tissue types.

According to a further preferred embodiment of the invention, the linking unit can be cleaved by enzymes which are coupled to antibodies with selectivity for tumour tissue and are thus addressed to tumour tissue. This is also called the ADEPT approach. This likewise leads to a further increase in the tissue specificity of the conjugates according to the invention and thus to an additional decrease of the conjugates according to the invention in other tissue types.

Particularly preferred conjugates according to the present invention are those of the general formula (I)

CT - AA1 - AA2 - AA3 - AA4 - Sp - IA (I)

in which
CT denotes a cytotoxic radical or a radical of a cytostatic or of a cytostatic derivative, which can additionally carry a hydroxyl, carboxyl or amino group,
AA1 is absent or is an amino acid in the D or L configuration, which can optionally carry protective groups or a radical Sp',
AA2 is absent or is an amino acid in the D or L configuration, which can optionally carry protective groups or a radical Sp',
AA3 is absent or is an amino acid in the D or L configuration, which can optionally carry protective groups or a radical Sp',
A4 is absent or is an amino acid in the D or L configuration, which can optionally carry protective groups or a radical Sp',
in which
Sp' is an arylaminocarbonyl or an arylaminothiocarbonyl radical having 7-11 carbon atoms,
Sp is absent, is an arylaminocarbonyl or an arylaminothiocarbonyl radical having 7-11 carbon atoms or is an alkanedicarboxylic acid radical having 3 to 8 carbon atoms or a carbonyl or a thiocarbonyl radical,
with the proviso that at least one of the radicals AA1 to AA4 and/or Sp is present,
IA is a non-peptide radical addressing an αᵥß₃ integrin receptor, which
is a radical of the formula (III) 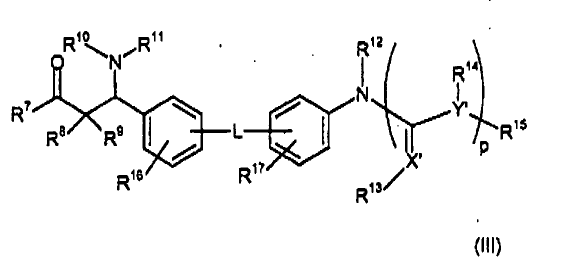 in which
- R⁷: is OH,
- R⁸: is hydrogen,
- R⁹: is hydrogen,
- R¹⁰: is -SO₂R^{10'}, -COOR^{10"}, -COR^{10'}, -CONR^{10'} or -CS-NR^{10'}, or represents a direct bond via which the radical of the formula (III) is optionally bonded to the rest of the conjugate;
- R^{10'}: independently of one another is hydrogen, a,substituted or unsubstituted alkyl or cycloalkyl radical, a substituted or unsubstituted aryl radical or a saturated or unsaturated, optionally substituted heterocyclic radical, via which the radical of the formula (III) is optionally bonded to the rest of the conjugate;
- R^{10"}: is a substituted or unsubstituted alkyl or cycloalkyl radical, a substituted or unsubstituted aryl radical or a saturated or unsaturated, optionally substituted heterocyclic radical, via which the radical of the formula (III) is optionally bonded to the rest of the conjugate;
- R¹¹: is hydrogen, a substituted or unsubstituted alkyl or cycloalkyl radical or a substituted or unsubstituted aryl radical,
- R¹⁶: is hydrogen, CN, a substituted or unsubstituted alkyl or cycloalkyl radical, a substituted or unsubstituted alkoxy radical or a halogen atom;
- R¹⁷: is hydrogen, CN, a substituted or unsubstituted alkyl or cycloalkyl radical, a substituted or unsubstituted alkoxy radical or a halogen atom;
- L: is -NHSO₂-
- R¹²: is hydrogen, a substituted or unsubstituted alkyl or cycloalkyl radical, a substituted or unsubstituted aryl radical, a saturated or unsaturated, optionally substituted heterocyclic radical
- X': is N, O or S;
- p: is 1;
- R¹³: is absent, is -H, a substituted or unsubstituted alkyl or cycloalkyl radical, -NO₂, -CN, -COR^{13'}, -COOR^{13'}.
- R^{13'}: is hydrogen, a substituted or unsubstituted alkyl or cycloalkyl radical, a substituted or unsubstituted aryl radical or a saturated or unsaturated, optionally substituted heterocyclic radical which can be saturated or unsaturated and/or can contain further heteroatoms;
- Y': is N
- R¹⁴: is hydrogen, a substituted or substituted alkyl or cycloalkyl radical, a substituted or unsubstituted aryl radical, a saturated or unsaturated, optionally substituted heterocyclic radical
- R¹⁵: is hydrogen, a substituted or unsubstituted alkyl or cycloalkyl radical, a substituted or unsubstituted aryl radical ,a saturated or unsaturated, optionally substituted heterocyclic radical;
and their physiologically acceptable salts and stereoisomers.

Of the conjugates of the formula (I), according to yet a further preferred embodiment those conjugates are particularly preferred in which
- CT: is camptothecin or 9-aminocamptothecin, which can be linked to the rest of the conjugate via the C20-OH group or, in the case of 9-aminocamptothecin, via the free amino group;
- AA1: is absent or is a naturally occurring amino acid in the D or L configuration, which is selected from the group consisting of glycine, alanine, valine, leucine, isoleucine, and phenylalanine;
- AA2: is absent or is a naturally occurring amino acid in the D or L configuration, which is selected from the group consisting of lysine, glutamate, histidine, glycine, arginine, ornithine and leucine, and can optionally carry protective groups or a radical Sp',
- AA3: is absent or is a naturally occurring amino acid in the D or L configuration, which is selected from the group consisting of glycine, alanine, valise, leucine, isoleucine and phenylalanine;
- AA4: is absent or is a naturally occurring amino acid in the D or L configuration, which can optionally carry protective groups or a radical Sp',
in which
Sp' is a phenylaminocarbonyl or a phenylaminothiocarbonyl radical,
- Sp: is absent, is a phenylaminocarbonyl or a phenylaminothiocarbonyl radical or is an alkanedicarboxylic acid radical having 3 to 6 carbon atoms or a carbonyl or a thiocarbonyl radical,
with the proviso that at least one of the radicals AA1 to AA4 and/or Sp is present,
- IA: is a non-peptide radical of the formula (III) addressing an αᵥß₃ integrin receptor,
in which
R⁷ is OH,
R⁸ is hydrogen,
R⁹ is hydrogen,
R¹⁰ is SO₂R^{10'}, -COOR^{10''}, -COR^{10'}, -CONR^{10'}₂ or -CS-NR^{10'}2 or represents a direct bond, via which the radical of the formula (III) is optionally bonded to the rest of the conjugate;
R^{10'} is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -C₆H₂(CH₃)₃, -C₆(CH₃)₅, -CH₂C₆H₂(CH₃)₃, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 4-chlorophenylmethyl, 2,4-dichlorophenylmethyl, 2,6-dichlorophenylmethyl, 3-aminophenyl, 4-aminophenyl, 2-methoxycarbonylphenylmethyl, 3-trifluoromethylphenyl, 4-trifluoromethyl-phenyl, 3,5-bis(trifluoromethyl)phenyl, 4-trifluo-ro-methoxyphenyl, phenylmethyl, 2-acetamido-4-meth-ylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimeth-oxyphenyl, 3-methylphenyl, 4-methylphenyl, 4-t-butylphenyl, 4-propylphenyl, 2,5-dimethylphenyl, 2-meth-oxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluoro-phenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methylbenzothiazol-2-yl, N-methoxycarbonyl-piperidin-3-yl, thiophen-2-yl, isoxazol-5-yl, ethoxy, 2-chloropyridin-3-yl, pyridin-3-yl, benzyloxy, 5-methylisoxazol-3-yl, 1-adamantyl, 4-chlorophenoxymethyl, 2,2-dimethylethenyl, 2-chloropyridine-5-methyl, 5,7-dimethyl-1,3,4-triazaindolizin-2-yl, (S)-camphan-1-yl, (R)-camphan-1-yl or 8-quinolinyl;
R^{10"} is a C₁₋₆-alkyl radical, a C₃₋₇-cycloalkyl radical, a substituted or unsubstituted aryl radical or a saturated or unsaturated, optionally substituted heterocyclic radical, via which the radical of the formula (III) is optionally bonded to the rest of the conjugate;
R¹¹ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, dialkyl-amino-C₁₋₄-alkyl, amino-C₁₋₄-alkyl, C₁₋₄-alkyloxy-C₁₋₄-alkyl or 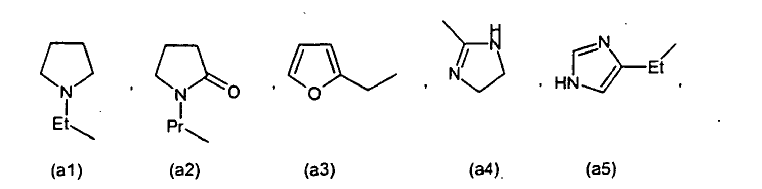 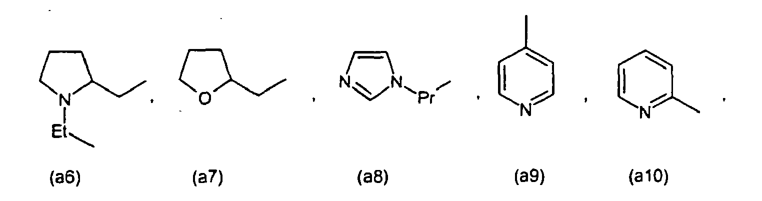 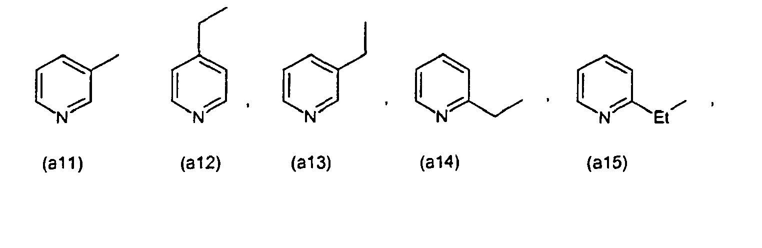 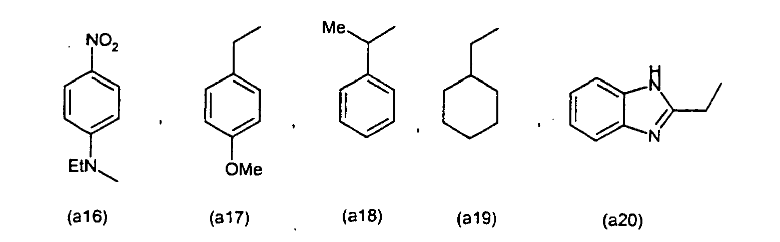 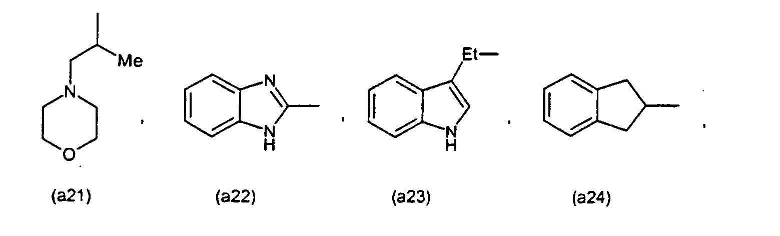 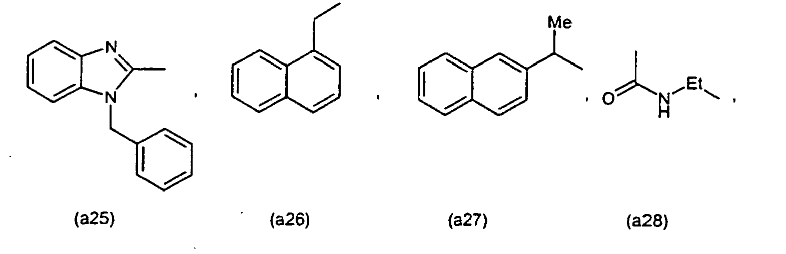
R¹⁶ is hydrogen, CN, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, methoxy, trifluoromethoxy, ethoxy, propoxy, butoxy, pentoxy or hexoxy, fluorine, chlorine, bromine or iodine;
R¹⁷ is hydrogen, CN, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, methoxy, ethoxy, trifluoromethoxy, propoxy, butoxy, pentoxy or hexoxy, fluorine, chlorine, bromine or iodine;
L is -NHSO₂-,
R¹² is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28)
X' is N, O or S;
p 1;
R¹³ is absent, is -H, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, -NO₂, -CN, -COR^{7'}, -COOR^{7'},
R^{13'} is hydrogen, methyl, ethyl, propyl, isopropyl, butyl, isobutyl, t-butyl, pentyl, isopentyl, neopontyl, hexyl, cyclopropyl, cyclobutyl, cyclopentyl, cyclohexyl, cycloheptyl, phenyl, benzyl, tolyl or a substituted derivative thereof;
Y' is N
R¹⁴ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethyleyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28),
and
R¹⁵ is hydrogen methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, C₁₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl, one of the radicals (a1) to (a28),

Likewise particularly preferred conjugates of the formula (I) in this further preferred embodiment are those in which the radical of the formula (III) is linked to the rest of the conjugate via a radical in the β-position relative to the carboxyl group, and the other radicals of the formula (III) are as defined above.

The compounds of the formula (I) according to the invention can also be present in the form of their salts. In general salts with organic or inorganic bases or acids may be mentioned here.

In particular, the compounds of the formula (I) according to the invention can be employed in the form of their physiologically acceptable salts. Physiologically acceptable salts are understood according to the invention as meaning non-toxic salts which in general are accessible by reaction of the compounds of the formula (I) according to the invention with an inorganic or organic base or acid conventionally used for this purpose. Examples of preferred salts of the compounds of the formula (I) according to the invention are the corresponding alkali metal salt, e.g. lithium, potassium or sodium salt, the corresponding alkaline earth metal salt such as the magnesium or calcium salt, a quaternary ammonium salt such as, for example, the triethylammonium salt, acetate, benzenesulphonate, benzoate, dicarbonate, disulphate, di-tartrate, borate, bromide, carbonate, chloride, citrate, dihydrochloride, fumarate, gluconate, glutamate, hexylresorcinate, hydrobromide, hydrochloride, hydroxynaph-thoate, iodide, isethionate, lactate, laurate, malate, maleate, mandelate, mesylate, methylbromide, methylnitrate, methylsulphate, nitrate, oleate, oxalate, palmitate, pantothenate, phosphate, diphosphate, polygalacturonate, salicylate, stearate, sulphate, succinate, tartrate, tosylate and valerate and other salts used for medicinal purposes.

The present invention includes both the individual enantiomers or diastereomers and the corresponding racemates, diastereomer mixtures and salts of the compounds according to the invention. In addition, all possible tautomeric forms of the compounds described above are included according to the present invention. Furthermore, the present invention includes both the pure E and Z isomers of the compounds of the formula (I) and their E/Z mixtures in all ratios. The diastereomer mixtures or E/Z mixtures can be separated into the individual isomers by chromatographic processes. The racemates can be resolved into the respective enantiomers either by chromatographic processes on chiral phases or by resolution.

In the context of the present invention, the substituents, if not stated otherwise, in general have the following meaning:
Alkyl in general represents a straight-chain or branched hydrocarbon radical having 1 to 20 carbon atoms. Examples which may be mentioned are methyl, ethyl, propyl, isopropyl, butyl, isobutyl, pentyl, isopentyl, hexyl, isohexyl, heptyl, isoheptyl, octyl and isooctyl, nonyl, decyl, dodeyl, eicosyl.
Alkenyl in general represents a straight-chain or branched hydrocarbon radical having 2 to 20 carbon atoms and one or more, preferably having one or two, double bonds. Examples which may be mentioned are allyl, propenyl, isopropenyl, butenyl, isobutenyl, pentenyl, isopentenyl, hexenyl, isohexenyl, heptenyl, isoheptenyl, octenyl, isooctenyl.
Alkinyl in general represents a straight-chain or branched hydrocarbon radical having 2 to 20 carbon atoms and one or more, preferably having one or two, triple bonds. Examples which may be mentioned are ethinyl, 2-butinyl, 2-pentinyl and 2-hexinyl.
Acyl in general represents straight-chain or branched lower alkyl having 1 to 9 carbon atoms, which is bonded via a carbonyl group. Examples which may be mentioned are: acetyl, ethylcarbonyl, propylcarbonyl, isopropylcarbonyl, butylcarbonyl and isobutylcarbonyl.
Alkoxy in general represents a straight-chain or branched hydrocarbon radical having 1 to 14 carbon atoms and bonded via an oxygen atom. Examples which may be mentioned are methoxy, ethoxy, propoxy, isopropoxy, butoxy, isobutoxy, pentoxy, isopentoxy, hexoxy, isohexoxy, heptoxy, isoheptoxy, octoxy or isooctoxy, The terms "alkoxy" and "alkyloxy" are used synonymously.
Alkoxyalkyl in general represents an alkyl radical having up to 8 carbon atoms, which is substituted by an alkoxy radical having up to 8 carbon atoms.
Alkoxycarbonyl can be represented, for example, by the formula 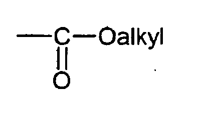
Alkyl here in general represents a straight-chain or branched hydrocarbon radical having 1 to 13 carbon atoms. Examples which may be mentioned are the following alkoxycarbonyl radicals: methoxycarbonyl, ethoxycarbonyl, propoxycarbonyl, isopropoxycarbonyl, butoxycarbonyl or isobutoxycarbonyl.
Cycloalkyl in general represents a cyclic hydrocarbon radical having 3 to 8 carbon atoms. Cyclopropyl, cyclopentyl and cyclohexyl are preferred. Examples which may be mentioned are cyclopentyl, cyclohexyl, cycloheptyl and cyclooctyl.
Cycloalkoxy in the context of the invention represents an alkoxy radical whose hydrocarbon radical is a cycloalkyl radical. The cycloalkyl radical in general has up to 8 carbon atoms. Examples which may be mentioned are: cyclopropyloxy and cyclo-hexyloxy. The terms "cycloalkoxy" and "cycloalkyloxy" are used synonymously.
Aryl in general represents an aromatic radical having 6 to 10 carbon atoms. Preferred aryl radicals are phenyl, benzyl and naphthyl.
Halogen in the context of the invention represents fluorine, chlorine, bromine and iodine.
Heterocycle in the context of the invention in general represents a saturated, unsaturated or aromatic 3- to 10-membered, for example 5- or 6-membered, heterocycle which can contain up to 3 heteroatoms from the group consisting of S, N and/or O and which, in the case of a nitrogen atom, can also be bonded via this. Examples which may be mentioned are: oxadiazolyl, thiadiazolyl, pyrazolyl, pyridyl, pyrimidinyl, pyridazinyl, pyrazinyl, thienyl, furyl, pyrrolyl, pyrrolidinyl, piperazinyl, tetrahydropyranyl, tetrahydrofuranyl, 1,2,3-triazolyl, thiazolyl, oxazolyl, imidazolyl, morpholinyl or piperidyl. Thiazolyl, furyl, oxazolyl, pyrazolyl, triazolyl, pyridyl, pyrimidinyl, pyridazinyl and tetrahydropyranyl are preferred. The term "heteroaryl" (or "hetaryl") represents an aromatic heterocyclic radical.

The conjugates according to the invention are characterized in that a cytotoxic radical or a radical of a cytostatic or of a cytostatic derivative is bonded via a linking unit to a non-peptide moiety addressing αᵥβ₃ or αᵥβ₅ integrin receptors.

The non-peptide moiety of the conjugate addressing αᵥβ₃ or αᵥβ₅ integrin receptors serves to bring the toxophoric part of the conjugate into or into the vicinity of tumour cells and thus to achieve tissue selectivity. Growing tumour tissue stimulates the formation of new blood vessels, i.e. angiogenesis, to a considerable extent in order to cover its increasing nutritional need. The blood vessels newly formed by angiogenesis differ from conventional tissue by specific markers on the surfaces of the endothelial cells formed. Moreover, the αᵥβ₃ or αᵥβ₃ integrin receptor is expressed by many human tumours (cf. WO 98/10795 and the references indicated there). Thus the conjugate is brought selectively into or into the vicinity of the tumour tissue to be treated by the interaction of its non-peptide part addressing αᵥβ₃ or αᵥβ₅ integrin receptors with αᵥβ₃ or αᵥβ₅ integin receptors found on endothelial cells or on tumour cells formed by angiogenesis.

Unlike peptide radicals addressing αᵥβ₃ or αᵥβ₃ integrin receptors (such as disclosed, for example, in WO 98/10795), the non-peptide moieties according to the invention addressing αᵥβ₃ or αᵥβ₅ integrin receptors are distinguished by an increased serum stability, whereby the transport of the toxophore in the conjugate to the tumour tissue is ensured to an increased extent.

The conjugate according to the invention can release its toxophoric radical at its target site and this can thus make possible penetration into the tumour tissue. This is carried out by the specific choice of a unit linking the toxophoric radical to the moiety addressing αᵥβ₃ or αᵥβ₅ integrin receptors. In order to be able to release the toxophoric radical, the linking unit must be cleavable under physiological conditions. This means that the linking unit must be cleavable either hydrolytically or by endogenous enzymes.

It is particularly preferred if the linking unit is cleaved by tumour-associated enzymes.
This leads to a further increase in the tissue selectivity of the action of the conjugates according to the invention.

A further suitable starting point for promoting the tissue selectivity of the action of the conjugates according to the invention consists in the so-called ADEPT approach. In this, conjugates are cleaved by certain enzymes. These enzymes are introduced into the body coupled to antibodies together with the conjugates according to the invention, the antibodies serving as vehicles specifically addressing tumour tissue. This leads to a selective concentration both of the conjugate and of the enzyme/antibody system in the tumour tissue, whereby the toxophore is released in the tumour tissue with even greater selectivity and can display its action there.

Suitable linking units according to the invention are all linking units which fulfil at least one of the abovementioned criteria and can be linked to the moiety addressing αᵥβ₃ or αᵥβ₅ integrin receptors in such a way that this retains its binding action to αᵥβ₃ or αᵥβ₅ integrin receptors.

In the conjugates according to the invention, toxophores used can be all cytotoxic radicals or radicals of a cytostatic or of a cytostatic derivative which are conventionally employed in tumour therapy.

According to a preferred embodiment, conjugates according to the invention which can be employed are compounds of the formula (I) in which a toxophore is linked via a linking unit consisting of 0 to 4 amino acids, preferably 1 to 3 amino acids and particularly preferably 2 amino acids, and, if appropriate, of a non-peptide spacer group, to a non-peptide moiety addressing αᵥß₃ integrin receptors of the formulae (111) 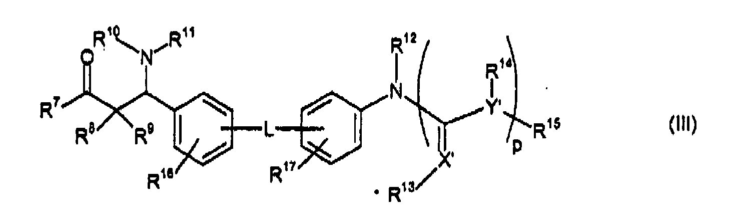 where the radicals in the formulae have the meanings indicated above.

In the conjugates of the formula (I) according to the invention, the toxophore used can be cytostatic radicals or radicals of a cytostatic or of a cytostatic derivative which are conventionally employed in tumour therapy. Camptothecin or derivatives of camptothecin such as 9-aminocamptothecin are preferred here, which can be linked to the rest of the conjugate via the C₂₀-OH group or via a functional group which is optionally present in the molecule, such as the amino group in the case of 9-aminocamptothecin.
According to this preferred embodiment, the camptothecin unit used as a starting compound can be present in the 20(R) or in the 20(S) configuration or as a mixture of these two stereoisomeric forms. The 20(S) configuration is preferred.

In the conjugates of the formula (I), the linking unit preferably consists of a unit of the formula

-AA1-AA2-AA3-AA4-Sp.

The radicals AA1 to AA4, if they are present, each represent an amino acid in the D or L configuration, which can optionally carry protective groups or a radical Sp'. In this context, they are particularly preferably one of the naturally occurring amino acids glycine, alanine, valine, leucine, isoleucine, serine, threonine, cysteine, methionine, aspartate, glutamate, asparagine, glutamine, arginine, lysine, histidine, tryptophan, phenylalanine, tyrosine or proline. The amino acids used in the process according to the invention can occur in the L or in the D configuration or alternatively as a mixture of D and L form.

The term "amino acids" refers, according to the invention, in particular to the α-amino acids occurring in nature, but moreover also includes their homologues, isomers and derivatives. An example of isomers which can be mentioned is enantiomers. Derivatives can be, for example, amino acids provided with protective groups.

According to the present invention, the amino acids can each be linked to one another and to the toxophore or to the moiety addressing αᵥβ₃ or αᵥβ₅ integrin receptors via their α-carboxyl or α-amino functions, but also via functional groups optionally present in side chains, such as, for example, amino functions.

In the case of amino acids having functional groups in the side chains, these functional groups can be either deblocked or protected by conventional protective groups used in peptide chemistry. Protective groups employed for these functional groups of the amino acids can be the protective groups known in peptide chemistry, for example of the urethane, alkyl, acyl, ester or amide type.

Amino protective groups in the context of the invention are the customary amino protective groups used in peptide chemistry. These preferably include: benzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl; 4-nitrobenzyloxy-carbonyl, 2-nitrobenzyloxycarbonyl, 2-nitro-4,5-dimethoxybenzyloxycarbonyl, methoxycarbonyl, ethoxycarbonyl, tert-butoxycarbonyl (Boc), allyloxycarbonyl, vinyloxycarbonyl, 3,4,5-trimethoxybenzyloxycarbonyl, phthaloyl, 2,2,2-trichloroethoxycarbonyl, 2,2,2-trichloro-tert-butoxycarbonyl, menthyloxycarbonyl, 4-nitro-phenoxycarbonyl, fluorenyl-9-methoxycarbonyl (Fmoc), formyl, acetyl, propionyl, pivaloyl, 2-chloroacetyl, 2-bromoacetyl, 2,2,2-trifluoroacetyl, 2,2,2-trichloroacetyl, benzoyl, benzyl, 4-chlorobenzoyl, 4-bromobenzoyl, 4-nitrobenzoyl, phthalimido, isovaleroyl or benzyloxymethylene, 4-nitrobenzyl, 2,4-dinitrobenzyl, 4-nitrophenyl or 2-nitrophenylsulphenyl. The Fmoc group and the Boc group are particularly pure-ferred.

The removal of protective groups in appropriate reaction steps can be carried out, for example, by the action of acid or base, hydrogenolytically or reductively in another manner.

Furthermore, each of the amino acids AA1 to AA4 can carry a radical Sp', where Sp' represents an arylaminocarbonyl or an arylaminothiocarbonyl radical having 7-11 carbon atoms. Preferably, this radical Sp' is a phenylaminocarbonyl or a phenylaminothiocarbonyl radical.

The radical Sp' is preferably bonded to the side chain of the corresponding amino acid via the functional group. If, however, the linkage of the toxophore to the moiety addressing αᵥβ₃ or αᵥβ₅ integrin receptors in an amino acid takes place via the functional group in the side chain, the radical Sp' can also be linked to the carboxyl or α-amino function of the corresponding amino acid.

According to a preferred embodiment, AA1, if present, is selected from amino acids having sterically demanding or non-polar side chains. Examples which may be mentioned are glycine, alanine, valine, leucine, isoleucine, phenylalanine, proline, tryptophan and methionine. Valine, leucine and isoleucine are particularly preferred.

According to a preferred embodiment, AA2, if present, is selected from amino acids having basic side chains. Examples which may be mentioned are lysine, arginine, glutamate, histidine, ornithine, glycine, leucine or diaminobutyric acid. However, amino acids having non-polar side chains can also be used. Lysine, glutamate, histidine, leucine and glycine are particularly preferred.

According to a preferred embodiment, AA3, if present, is selected from amino acids having non-polar side chains. Examples which may be mentioned are glycine, alanine, valine, leucine, phenylalanine and isoleucine. Glycine, valine and leucine are particularly preferred.

According to a preferred embodiment, AA4, if present, is selected from amino acids having non-polar side chains. Examples which may be mentioned are alanine, valine, leucine, isoleucine, proline, tryptophan, phenylalanine and methionine. Alanine, valine and proline are particularly preferred.

According to the preferred embodiment according to the invention, the spacer unit Sp is an arylaminocarbonyl or an arylaminothiocarbonyl radical having 7-11 carbon atoms or an alkanedicarboxylic acid radical having 3 to 8 carbon atoms or a carbonyl or a thiocarbonyl radical. Particularly preferably, Sp is a phenylaminocarbonyl or a phenylaminothiocarbonyl radical or an alkanedicarboxylic acid radical having 3 to 6 carbon atoms or a carbonyl or a thiocarbonyl radical. In particular, a carbonyl or a thiocarbonyl radical and a succinic acid or glutaric acid radical are preferred.

It is preferred according to the invention that the linking unit consists of two amino acids AA1 and AA2 and the spacer unit Sp, it being possible, in particular, for the unit AA2 to be modified on the side chain by protective groups or the radical Sp'. However, it is also possible for the linking unit to consist of one, three or four amino acids AA1 to AA4 and a spacer unit Sp. In these cases, the linkage to the toxophore as a rule takes place via the carboxyl function of the amino acid AA1 and the linkage to the moiety addressing αᵥβ₃ or αᵥβ₅ integrin receptors via the spacer unit Sp takes place using an amino group or hydroxyl group of the moiety addressing αᵥβ₃ or αᵥβ₅ integrin receptors. In the case in which the linkage is to take place via a carboxyl function of the moiety addressing αᵥβ₃ or αᵥβ₅ integrin receptors, it is preferred, however, to use linking units without the spacer unit Sp. In this case, the linkage between the linking unit and the moiety addressing αᵥβ₃ or αᵥβ₅ integrin receptors takes place via an amino function of an amino acid. In this case, a linking unit consisting of two amino acids AA1 and AA2 is particularly preferred,

If the toxophore contains an amino function, for example 9-aminocamptothecin, the linkage to the moiety addressing αᵥβ₃ or αᵥβ₃ integrin receptors can take place directly via a spacer unit Sp without any amino acids AA1 to AA4 being contained in the linking unit. It is particularly preferred in this case for Sp to represent a carbonyl or thiocarbonyl function, in particular a thiocarbonyl function.

The moiety addressing αᵥβ₃ integrin receptors can furthermore be a radical of the formula (III): 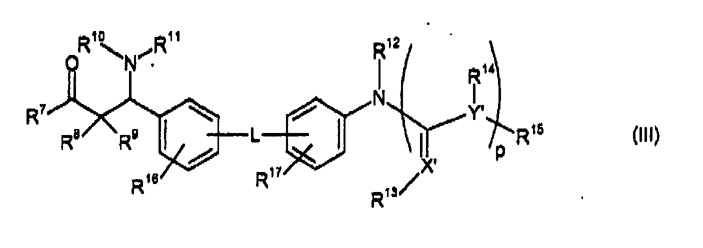 where the radicals in the formula (III) have the meaning defined above.

In the description below, bivalent substituents are indicated such that their respective left end is connected to the group indicated left of the corresponding substituent in formula (III) and their respective right end is connected to the group indicated right of the corresponding substituent in formula (III). If, for example, the radical L is equal to -NHSO₂- in formula (III), the nitrogen atom is connected to the phenylene group found left of the radical L in formula (III). The following details additionally relate to the radical of the formula (III) in the un-linked state. The linkage of the radical of the formula (III) to the toxophore via the linking unit can take place via a functional group in the side chain of the radical of the formula (III), i.e. via the amino group or a substituent attached thereon in the ß-position relative to the terminal carboxyl group.

The radicals of the formula (III) according to the invention are characterized in that they have, as a main structural element, two phenyl units connected via a linker group L, one phenylene group of which has a radical derived from a β-amino acid, while the other phenylene group has an amino group, urea group, thiourea group or guanidine group. The phenylene units connected via a linker group L can moreover carry further substituents in addition to the abovementioned radicals,

Preferably, the radicals of the formula (III) according to the invention are used in a form in which the terminal carboxyl unit is present as a free carboxylic acid.

The radical bonded to one of the two central phenylene units and derived from a β-amino acid can alternatively carry one or two additional substituents in the α-position relative to the carboxyl group. These substituents can each be selected from the group which consists of hydrogen, a substituted or unsubstituted alkyl or cycloalkyl radical, a substituted or unsubstituted aryl radical, a saturated or unsaturated, optionally substituted heterocyclic radical, an optionally substituted alkenyl radical, an optionally substituted alkinyl radical, a hydroxyl radical or an alkoxy radical. The alkyl

According to the invention, the amino group included in the radical derived from a β-amino acid is particularly preferably substituted by -SO₂R^{10'}, -COOR^{10"}, -CONR^{10'}₂ or -COR^{10'}, where R^{10'} and R^{10"} are as defined above. In particular, radicals of the formula (III) are preferred here in which the radical derived from a β-amino acid has no substituent in the α-position relative to the carboxyl unit and the amino group included in this radical is substituted by -SO₂R^{10'}, -CONR^{10'}₂ or -COR^{10'}, where R^{10'} is as defined above.

In addition to one of the abovementioned radicals, the nitrogen atom of the amino group found in the β-position can have a substituent which is selected from the group consisting of hydrogen, a substituted or unsubstituted alkyl or cycloalkyl radical, a substituted or unsubstituted aryl radical, a saturated or unsaturated, optionally substituted heterocyclic radical or are bonded to one another and thus, together with the nitrogen atom to which they are bonded, form a heterocyclic ring system. Preferred substituents here are those which can be selected from the group consisting of hydrogen, a C₁₋₆-alkyl such as, for example, methyl, ethyl, propyl, isopropyl, butyl, isobutyl, t-butyl, pentyl, isopentyl, neopentyl or hexyl, a C₃₋₇cycloalkyl such as, for example, cyclopropyl, cyclopropylmethyl, cyclobutyl, cyclopentyl, cyclohexyl or cycloheptyl, an aryl such as, for example, phenyl, benzyl or tolyl, a heterocyclic radical such as, for example, pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benxothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine and can alternatively be substituted by one or more C₁₋₆-alkyl radicals such as methyl, ethyl, propyl, isopropyl, butyl, isobutyl, t-butyl, pentyl, isopentyl, neopentyl or hexyl, C₃₋₇-cycloalkyl radicals such as cyclopropyl, cyclo-propylmethyl, cyclobutyl, cyclopentyl or cyclohexyl, aryl radicals such as phenyl, benzyl, tolyl, naphthyl, indolyl, heterocyclic radicals such as pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, indolyl, benzofuranyl, benzoxazolyl, benzothiazolyl, benzimidazolyl, quinolinyl or isoquinolinyl unit or a partially or completely hydrogenated analogue thereof. The additional substituent on the nitrogen atom of the β**-**amino group is particularly preferably hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methyl-cyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl,

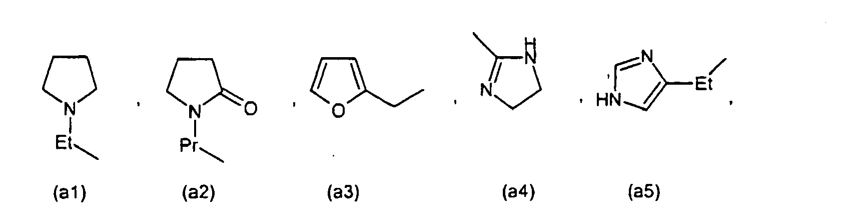

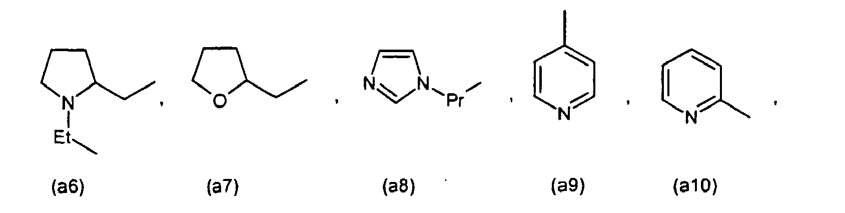

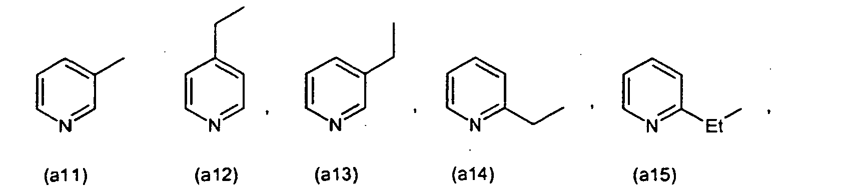

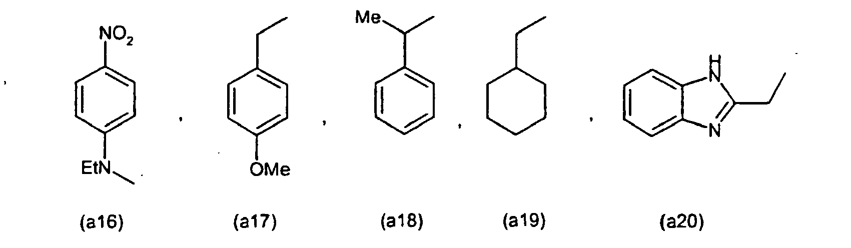

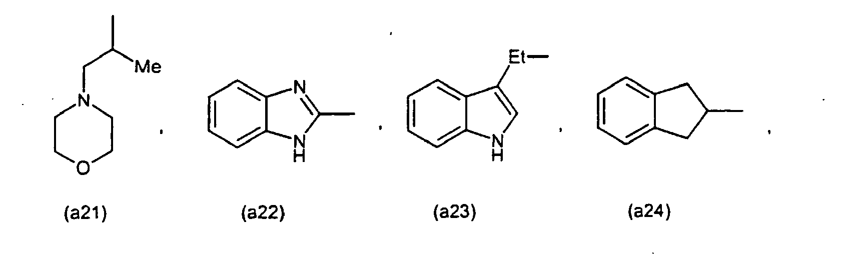

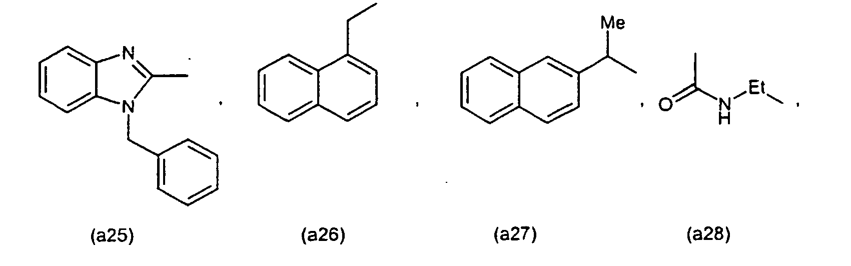

The radical derived from a β-amino acid is bonded to one of the two central phenylene units connected via a linker group L, which is to be designated here as phenylene unit A. In addition to the radical derived from a β-amino acid and the linker group L, the phenylene unit A preferably carries no further substituents, but can have one or more radicals which are selected from the group consisting of hydrogen, CN, a substituted or unsubstituted alkyl or cycloalkyl radical, a substituted or unsubstituted alkoxy radical or a halogen atom. The alkyl radical(s) is/are preferably C₁₋₆-alkyl radicals such as methyl, ethyl, propyl, isopropyl, butyl, isobutyl, t-butyl, pentyl, isopentyl, neopentyl, hexyl. The cycloalkyl radical(s) is/are preferably C₃₋₇-cycloalkyl radicals such as cyclopropyl, cyclobutyl, cyclopentyl, cyclohexyl, cycloheptyl.
The alkoxy radical(s) is/are preferably C₁₋₆-alkoxy radicals such as methoxy, trifluoromethoxy, ethoxy, propoxy, butoxy, pentoxy or hexoxy, and the halogen atom(s) is/are preferably F, Cl, Br or I.

Particularly preferred according to the present invention are radicals of the formula (III) whose central phenylene A-linker L-phenylene B unit consists according to the present definition of an m-substituted phenylene unit A and an m-substituted phenylene unit B.

According to the invention, the linker group L is preferably -NHSO₂-,

The central phenylene unit B carries as a substituent a radical which, is selected from the group consisting of a guanidine, urea or thiourea unit. This guanidine, urea or thiourea unit can be open-chain. The nitrogen atoms of the respective unit, which are optionally both present and bonded only via single bonds, can carry additional substituents R¹², R¹⁴ and R¹⁵. These substituents can independently of one another or simultaneously be hydrogen, a substituted or unsubstituted alkyl or cycloalkyl radical, a substituted or unsubstituted aryl radical, a saturated or unsaturated, optionally substituted heterocyclic radical Preferred substituents here are those which are selected from the group consisting of hydrogen, a C₁₋₆-alkyl such as, for example, methyl, ethyl, propyl, isopropyl, butyl, isobutyl, t-butyl, pentyl, isopentyl, neopentyl or hexyl, a C₃₋₇-cycloalkyl such as, for example, cyclopropyl, cyclopropylmethyl, cyclobutyl, cyclopentyl, cyclohexyl or cycloheptyl, an aryl such as, for example, phenyl, benzyl or tolyl, a heterocyclic radical such as, for example, pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydro-quinoline, tetrahydroisoquinoline, triazole, tetrazole, pyrimidine, purine, cytosine, thymine, uracil, adenine, guanine or xanthine and can alternatively be substituted by one or more C₁₋₆-alkyl radicals such as methyl, ethyl, propyl, isopropyl, butyl, isobutyl, t-butyl, pentyl, isopentyl, neopentyl or hexyl, C₃₋₇-cycloalkyl radicals such as cyclopropyl, cyclopropylmethyl, cyclobutyl, cyclopentyl or cyclohexyl, aryl radicals such as phenyl, benzyl, tolyl, naphthyl, indolyl, heterocyclic radicals such as pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydro-quinoline, tetrahydroisoquinoline, triazole, tetrazole, pyrimidine, purine, cytosine, thymine, uracil, adenine, guanine or xanthine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, indolyl, benzofuranyl, benzoxazolyl, benzothiazolyl, benzimidazolyl, quinolinyl or isoquinolinyl unit or a partially or completely hydrogenated analogue thereof.
Particularly preferred substituents are those such as hydrogen, methyl, ethyl, propyl, isopropyl, butyl, isobutyl, t-butyl, pentyl, isopentyl, neopentyl, hexyl, cyclopropyl, cyclo-propylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkylamino-C₁₋₄-alkyl, amino-C₁₋₄-alkyl, C₁₋₄-alkyloxy-C₁₋₄-alkyl or one of the abovementioned radicals (a1) to (a28).

As mentioned above, the urea, thiourea or guanidine unit can be open-chain and thus be a constituent of one of the following preferred functional units:

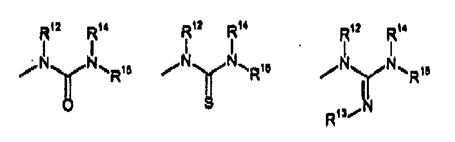

In the structural units shown above, R¹², R¹⁴ and R¹⁵ are as defined above.

Furthermore, in the above structural units R¹³ can be absent, hydrogen, a substituted or unsubstituted alkyl or cycloalkyl radical such as, for example, a C₁₋₆-alkyl such as methyl, ethyl, propyl, isopropyl, butyl, isobutyl, t-butyl, pentyl, isopentyl, neopentyl, hexyl or a C₃₋₇-cycloalkyl such as cyclopropyl, cyclobutyl, cyclopentyl, cyclohexyl, -NO₂, -CN, -COR^{13'} or -COOR^{13'}, where R^{13'} can be hydrogen, a substituted or unsubstituted alkyl or cycloalkyl radical, a substituted or unsubstituted aryl radical or a saturated or unsaturated, optionally substituted heterocyclic radical, which can be saturated or unsaturated and/or can contain further heteroatoms, and is preferably a C₁₋₆-alkyl such as, for example, methyl, ethyl, propyl, isopropyl, butyl, isobutyl, t-butyl, pentyl, isopentyl, neopentyl, hexyl, a C₃₋₇-cycloalkyl such as, for example, cyclopropyl, cyclobutyl, cyclopentyl, cyclohexyl, an aryl such as, for example, phenyl, benzyl, tolyl or a substituted derivative.

According to the invention, particularly preferred radicals of the formula (III) are those in which the amino group included in the radical derived from a β-amino acid carries a radical-SO₂R^{10'}, where R^{10'} is preferably methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -C₆H₂(CH₃)₃, -C₆(CH₃)₅, -CH₂C₆H₂(CH₃)₃, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 3-aminophenyl, 4-aminophenyl, 4-chlorophenylmethyl, 2,4-dichlorophenylmethyl, 2,6-dichlorophenylmethyl, 2-methoxycarbonylphenylmethyl, 3-trifluoro-methyl-phenyl, 4-trifluoromethylphenyl, 3,5-bis(trifluoromethyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propyl-phenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methylbenzothiazol-2-yl, N-methoxycarbonylpiperidin-3-yl, thio-phen-2-yl, isoxazol-5-yl, ethoxy, 2-chloropyridin-3-yl, pyridin-3-yl, benzyloxy, 5-methylisox-azol-3-yl, 1-adamantyl, 4-chlorophenoxymethyl, 2,2-dimethylethenyl, 2-chloropyri-din-5-methyl, 5,7-dimethyl-1,3,4-triazaindolizin-2-yl, (S)-camphan-1-yl, (R)-camphan-1-yl or 8-quinolinyl, the linker group L is -NHSO₂-, -CH₂NHSO₂-, -NHSO₂CH₂-, and the radical found on the phenylene unit is an open-chain or cyclic guanidine unit, a cyclic guanidine unit such as, for example, a 4,5-dihydro-1H-imidazol-2-ylamino unit being particularly preferred.

Furthermore, according to the present invention radicals of the formula (III) are particularly preferred in which the amino group included in the radical derived from a β-amino acid carries a radical-SO₂R^{10'} or a radical -COOR^{10"}, where R^{10'} or R^{10"} is preferably methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -C₆H₂(CH₃)₃, -C₆(CH₃)₅, -CH₂C₆H₂(CH₃)₃, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 3-aminophenyl, 4-aminophenyl, 4-chloro-phenyl-methyl, 2,4-dichlorophenylmethyl, 2,6-dichlorophenylmethyl, 2-methoxycarbonyl-phenyl-methyl, 3-trifluoromethylphenyl, 4-trifluoromethylphenyl, 3,5-bis(trifluoro-methyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methyl-phenyl, 4-methylphenyl, 4-t-butylphenyl, 4-propylphenyl, 2,5-dimethylphenyl, 2-methoxy-5-methylphenyl, 2,3,5,6-tetra-methylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methyl-benzo-thi-azol-2-yl, N-methoxycarbonyl-piperidin-3-yl, thiophen-2-yl, isoxazol-5-yl, ethoxy, 2-chloro-pyridin-3-yl, pyridin-3-yl, benzyloxy, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl or 8-quinolinyl, the linker group L is -NHSO₂-, -CH₂NH SO₂-, -NHSO₂CH₂- or -OCH₂-, -CH₂O-, -CH₂OCH₂-, -CH₂CH₂O-, -OCH₂CH₂-, and the radical found on the phenylene unit is an open-chain or cyclic guanidine unit, a cyclic guanidine unit such as, for example, a 4,5-dihydro-1H-imidazol-2-ylamino unit being particularly preferred.

Moreover, according to the present invention radicals of the formula (III) are particularly preferred in which the amino group included in the radical derived from a β-amino acid carries a radical-COR^{10'}, where R^{10'} is preferably hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -C₆H₂(CH₃)₃, -C₆(CH₃)₅, -CH₂C₆H₂(CH₃)₃, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 3-aminophenyl, 4-aminophenyl, 4-chlorophenylmethyl, 2,4-dichlorophenylmethyl, 2,6-dichlorophenylmethyl, 2-methoxycarbonylphenylmethyl, 3-trifluoromethylphenyl, 4-trifluoromethylphenyl, 3,5-bis(trifluoromethyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoro-methyl-phenyl, 2-alkylsulphonylphenyl, 2-arylsulphonylphenyl, 3-(N-acetyl-6-methoxy)-aniline, 4-acetamidophenyl, 2,2,2-trifluoroethyl, 5-chloro-3-methylbenzothiazol-2-yl, N-methoxycarbonyl-piperidin-3-yl, thiophen-2-yl, isoxazol-5-yl, ethoxy, 2-chloro-pyridin-3-yl, pyridin-3-yl, benzyloxy, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl or 8-quinolinyl, the linker group L is -NHSO₂-, -CH₂NHSO₂-, -NHSO₂CH₂-, and the radical found on the phenylene unit is an open-chain or cyclic guanidine unit, a cyclic guanidine unit such as, for example, a 4,5-dihydro-1H-imidazol-2-ylamino unit being particularly preferred.

Moreover, according to the present invention radicals of the formula (III) are particularly preferred in which the amino group included in the radical derived from a β-amino acid carries a radical -COR^{10'}, where R^{10'} is preferably hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -C₆H₂(CH₃)₃, -C₆(CH₃)₅, -CH₂C₆H₂(CH₃)₃, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 3-aminophenyl, 4-aminophenyl, 4-chlorophenylmethyl, 2,4-dichlorophenylmethyl, 2,6-dichlorophenylmethyl, 2-methoxycarbonylphenylmethyl, 3-trifluoromethylphenyl, 4-trifluoromethylphenyl, 3,5-bis(trifluoromethyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenyl-othenyl, 2-thiophenyl, 4-methoxyphenyl, 3,5-dimethoxyphenyl, 3-methylphenyl, 4-methylphenyl, 4-t-butylphenyl, 4-propylphenyl, 2,5"dimethylphenyl, 2-methoxy-5-methylphenyl, 2,3,5,6-tetramethylphenyl 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoro-methyl-phenyl, 2-alkylsulphonylphenyl, 2-arylsulphonylphenyl, 3-(N-acetyl-6-methoxy)-aniline, 4-acetamidophenyl, 2,2,2-trifluoroethyl, 5-chloro-3-methylbenzothiazol-2-yl, N-methoxycarbonyl-piperidin-3-yl, thiophen-2-yl, isoxazol-5-yl, ethoxy, 2-chloro-pyridin-3-yl, pyridin-3-yl, benzyloxy, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl or 8-quinolinyl, the linker group L is -NHSO₂-, -CH₃NHSO₂-, -NHSO₂CH₂-, and the radical found on the phenylene unit is an open-chain or cyclic guanidine unit, a cyclic guanidine unit such as, for example, a 4,5-dihydro-1H-imidazol-2-ylamino unit being particularly preferred.

The novel conjugates according to Claim 1 can be prepared by linkage of the toxophore to the linking unit and subsequent linkage to the moiety addressing α_{ν}β₃ integrin receptors. However, it is also possible to first connect the moiety addressing α_{ν}β₃ integrin receptors to the linking unit and then to bind the toxophore to the linking unit.

The combination of the individual units of the conjugates according to the invention can preferably be carried out by means of functional groups which can be reacted with one another and, as a result, can be linked by conventional processes known to the person skilled in the art. For example carboxyl functions can be reacted with amino functions with formation of an amide bond. It is also possible to synthesize the linking unit stepwise on one of the two radicals to be connected, i.e. the toxophore or the moiety addressing α_{ν}β₃ integrin receptors, by conventional processes known to the person skilled in the art and then to link the finished linking unit to the radical which is still to be bound.

The present invention in particular relates to a process for the preparation of conjugates according to formula (I), comprising
[A] the reaction of a compound from the group of compounds of the formulae (III) which has a free or optionally activated carboxyl function,
   with a compound of the formula (Ia) which has a free primary or secondary amino group

   CT-AA1-AA2-AA3-AA4-Sp (Ia)

   in which all radicals have the meaning indicated in Claim 5,
   in the presence of a base;
   or
[B] the reaction of a compound from the group of compounds of the formulae (III) which has a free primary or secondary amino function,
   with a carbonic acid derivative such as, for example, phosgene, thiophosgene or a chloroformic acid ester, if appropriate in the presence of a base,
   followed by the reaction with a compound of the formula (Ia) which has a free primary or secondary amino group

   CT-AA1-AA2-AA3-AA4-Sp (Ia)

   in which all radicals have the meaning indicated in Claim 5,
   and
   if appropriate the removal of protective groups and/or derivatization of nitrogen atoms present at preferred points of time in the preparation process and/or conversion of the compound obtained into the free acid and/or conversion of the compound obtained into one of its physiological salts by reaction with an inorganic or organic base or acid; or
[C] the reaction of a cytotoxic compound or of a cytostatic or of a cytostatic derivative CT which contains a free primary or secondary amino group,
   with a carbonic acid derivative such as, for example, phosgene, thiophosgene or a chloroformic acid ester in the presence of a base,
   followed by the reaction with a compound from the group of compounds of the formulae (III) which has a free primary or secondary amino function,
   and
   if appropriate the removal of protective groups and/or derivatization of nitrogen atoms present at preferred points of time in the preparation process and/or conversion of the compound obtained into the free acid and/or conversion of the compound obtained into one of its physiological salts by reaction with an inorganic or organic base or acid;
   or
[D] the reaction of a compound from the group of compounds of the formulae (III) which contains a free primary or secondary amino function,
   with a compound of the formula (Ia) which contains a free or optionally activated carboxyl function

   CT-AA1-AA2-AA3-AA4-Sp (Ia)
in which all radicals have the meaning indicated in Claim 5,
in the presence of a base;
and
if appropriate the removal of protective groups and/or derivatization of nitrogen atoms present at preferred points in time in the preparation process and/or conversion of the compound obtained into the free acid and/or conversion of the compound obtained into one of its physiological salts by reaction with an inorganic or organic base or acid.

According to a preferred embodiment, all steps of the preparation process are carried out on a solid phase.

In variant [A] of the preparation process according to the invention, a moiety addressing α_{ν}β₃ integrin receptors from the group of radicals of the formulae (III) is linked via its free carboxyl function to the amino function of a toxophore-linking unit conjugate (Ia) with formation of an amide bond. This reaction can be carried out by conventional methods known to the person skilled in the art (cf., for example, J. March, Advanced organic chemistry, 3rd ed., Wiley, p. 370 ff.). It is preferred according to the invention to activate the carboxyl function of the moiety addressing α_{ν}β₃ integrin receptors and then to react with the compound (Ia) in an organic solvent in the presence of a base.

For the activation of the carboxyl group, the coupling reagents known in peptide chemistry can be used, such as are described, for example, in Jakubke/Jeschkeit: Aminosäuren, Peptide, Proteine [Amino acids, Peptides, Proteins]; Verlag Chemie 1982 or Tetrahedr. Lett. 34, 6705 (1993). Examples mentioned are N-carboxylic acid anhydrides, acid chlorides or mixed anhydrides, adducts with carbodiimides, e.g.
N,N'-diethyl-, N,N-diisopropyl- or N,N-dicyclohexylcarbodiimide, N-(3-dimethylaminopropyl)N'-ethyl-carbodiimide hydrochloride, N-cyclohexyl-N'-(2-morpholinoethyl)-carbodiimide metho-p-toluenesulphonate, or carbonyl compounds such as carbonyldiimidazole, or 1,2-oxazolium compounds such as 2-ethyl-5-phenyl-1,2-oxazolium-3-sulphate or 2-tert-butyl-5-methyl-isoxazolium perchlorate, or acylamino compounds such as 2-ethoxy-1-ethoxycarbonyl-1,2-dihydroquinoline or propane-phosphonic anhydride or isobutyl chloroformate or benzotriazolyloxy-tris-(dimethyl-amino)-phosphonium hexafluorophosphate, 1-hydroxybenzotriazole or N-hydroxysuccinimide esters. It is furthermore proposed to employ the acid components in the form of a Leuchs' anhydride.

Variant [A] of the above preparation process according to the invention can be carried out under various pressure and temperature conditions, for example 0.5 to 2 bar and preferably under normal pressure, or -30 to +100°C and preferably -10 to +80°C, in suitable solvents such as dimethylformamide (DMF), tetrahydrofuran (THF), dichloromethane, chloroform, lower alcohols, acetonitrile, dioxane, water or in mixtures of the solvents mentioned. As a rule, reaction in DMF, dichloromethane, THF, dioxane/water or THF/dichloromethane at room temperature or with ice-cooling and under normal pressure is preferred.

Bases which can be employed in variant [A] of the preparation process according to the invention are, for example, triethylamine, ethyl-diisopropylamine, pyridine, N,N-dimethylaminopyridine or other bases conventionally used in steps of this type such as, for example, Hünig's base.

In variant [B] of the process according to the invention, a moiety addressing α_{ν}β₃ integrin receptors from the group of radicals of the formulae, (III) is reacted via its free amino function first with a carbonic acid derivative with formation of a corresponding isocyanate, isothiocyanate or carbamate, which is then linked to the amino function of a toxophore-linking unit conjugate (Ia) with formation of the conjugate (I).

The reaction of the moiety addressing α_{ν}β₃ integrin receptors from the group of radicals of the formulae (III) via its free amino function with a carbonic acid derivative can be carried out by conventional methods known to the person skilled in the art (cf., for example, J. March, Advanced organic chemistry, 3rd ed., Wiley, p. 370 ff.). According to the invention, the reaction is preferably carried out with phosgene or a substitute for phosgene such as, for example, trichloromethyl chloroformate, thiophosgene or a chloroformic acid ester in a solvent such as dimethylformamide (DMF) or a mixture of dioxane and water (1:1) or of tetrahydrofuran (THF) and dichloromethane (DCM) (1:1) at room temperature or with cooling, preferably at room temperature, and stirring for approximately 10 minutes up to approximately 3 hours, if appropriate in the presence of a base.

The subsequent reaction of the isocyanate, isothiocyanate or carbamate thus obtained with the amino function of a toxophore-linking unit conjugate (Ia) with formation of a corresponding thiourea or urea bond can be carried out by conventional methods known to the person skilled in the art (cf., for example, J. March, Advanced organic chemistry, 3rd ed., Wiley, p. 802 ff.).

According to the invention, the carbamate or thiocyanate or isothiocyanate is preferably reacted with the amino function of the compound (Ia) at room temperature with stirring for approximately 1 to 5 hours, preferably approximately 2 to 3 hours, in the presence of a base in a solvent such as dimethylformamide (DMF).

Bases which can be employed in variant [B] of the preparation process according to the invention are, for example, triethylamine, ethyldiisopropylamine, pyridine, N,N-dimethylaminopyridine or other bases conventionally used in steps of this type, such as, for example, Hünig's base.

In variant [C] of the process according to the invention, an amino function of a cytotoxic compound or of a cytostatic or of a cytostatic derivative CT is first reacted with a carbonic acid derivative with formation of a corresponding isocyanate, isothiocyanate or carbamate, which is then reacted with an amino function of a moiety addressing α_{ν}β₃ integrin receptors from the group of radicals of the formulae (III) with formation of the conjugate (I).

The reaction of the amino function of a cytotoxic compound or of a cytostatic or of a cytostatic derivative CT with a carbonic acid derivative can be carried out by conventional methods known to the person skilled in the art (cf., for example, J. March, Advanced organic chemistry, 3rd ed., Wiley, p. 370 ff.). According to the invention, the reaction with phosgene or a substitute for phosgene such as, for example, trichloromethyl chloroformate, thiophosgene or a chloroformic acid ester is preferably carried out in a solvent such as dimethylformamide (DMF) or a mixture of dioxane and water (1:1) or of tetrahydrofuran (THF) and dichloromethane (DCM) (1:1) at room temperature or with cooling, preferably at room temperature, and stirring for approximately 10 minutes up to approximately 3 hours, if appropriate in the presence of a base.

The subsequent reaction of the isocyanate, isothiocyanate or carbamate thus obtained with the amino function of a moiety addressing, α_{ν}β₃ integrin receptors from the group of radicals of the formulae (III) with formation of a corresponding thiourea or urea bond can be carried out by conventional methods known to the person skilled in the art (cf., for example, J. March, Advanced organic chemistry, 3rd ed., Wiley, p. 802 ff.).

According to the invention, the carbamate or thiocyanate or isothiocyanate is preferably reacted with the amino function of a moiety addressing α_{ν}β₃ integrin receptors from the group of radicals of the formulae, (III) at room temperature with stirring for approximately 1 to 5 hours, preferably approximately 2 to 3 hours, in the presence of abase in a solvent such as dimethylformamide (DMF).

Bases which can be employed in variant [C] of the preparation process according to the invention are, for example, triethylamine, ethyldiisopropylamine, pyridine, N,N-dimethylaminopyridine or other bases conventionally used in steps of this type, such as, for example, Hünig's base.

In variant [D] of the preparation process according to the invention, a moiety addressing α_{ν}β₃ integrin receptors from the group of radicals of the formulae (III) is linked via its free amino function to the carboxyl function of a toxophore-linking unit conjugate (Ia) with formation of an amide bond. This reaction can be carried out by conventional methods known to the person skilled in the art (cf., for example, J. March, Advanced organic chemistry, 3rd ed., Wiley, p. 370 ff). It is preferred according to the invention to activate the carboxyl function of the compound (Ia) and then to react it with a moiety addressing α_{ν}β₃ integrin receptors from the group of radicals of the formulae (III) in an organic solvent in the presence of a base.

For activation of the carboxyl group, the coupling reagents known in peptide chemistry can be used, such as are described, for example, in Jakubke/Jeschkeit: Aminosäuren. Peptide, Proteine [Amino acids, Peptides, Proteins]; Verlag Chemie 1982 or Tetrahedr. Lett. 34, 6705 (1993). Examples mentioned are N-carboxylic anhydrides, acid chlorides or mixed anhydrides, adducts with carbodiimides, e.g. N,N-diethyl-, N,N'-diisopropyl- or N,N'-dicyclohexylcarbodiimide, N-(3-dimethylaminopropyl)-N'-ethyl-carbodiimide hydrochloride, N-cyclohexyl-N'-(2-morpholinoethyl)-carbodiimide metho-p-toluenesulphonate, or carbonyl compounds such as carbonyldiimidazole, or 1,2-oxazolium compounds such as 2-ethyl-5-phenyl-1,2-oxazolium-3-sulphate or 2-tert-butyl-5-methyl-isoxazolium perchlorate, or acylamino compounds such as 2-ethoxy-1-ethoxycarbonyl-1,2-dihydroquinoline, or propane-phosphonic anhydride, or isobutyl chloroformate, or benzotriazolyloxy-tris-(dimeth-ylamino)-phosphonium hexafluorophosphate, 1-hydroxybenzotriazole or N-hydroxysuccinimide esters. It is furthermore proposed to employ the acid components in the form of a Leuchs' anhydride. Variant [D] of the above preparation process according to the invention can be carried out under various pressure and temperature conditions, for example 0.5 to 2 bar and preferably under normal pressure, or -30 to +100°C and preferably -10 to +80°C, in suitable solvents such as dimethylformamide (DMF), tetrahydrofuran (THF), dichloromethane, chloroform, lower alcohols, acetonitrile, dioxane, water or in mixtures of the solvents mentioned. As a rule, reaction in DMF, dichloromethane, THF, dioxane/water or THF/dichloromethane at room temperature or with ice-cooling and at normal pressure is preferred.

Bases which can be employed in variant [D] of the preparation process according to the invention are, for example, triethylamine, ethyldiisopropylamine, pyridine, N,N-dimethylaminopyridine or other bases conventionally used in steps of this type such as, for example, Hünig's base.

The compounds obtained according to the process explained above can furthermore be derivatized by removal of protective groups which may be present, further substitution of nitrogen atoms present at preferred positions in the preparation process and/or conversion of the compound obtained into the free acid and/or its physiologically acceptable salts. By way of example, the t-butoxymethoxycarbonyl groups conventionally used as protective groups for nitrogen atoms are removed in acidic medium, for example by addition of trifluoroacetic acid. Suitable alkylating agents for the derivatization of nitrogen atoms in this step are reagents conventionally used for this purpose, using which, for example, a substituted or unsubstituted alkyl or cycloalkyl radical, a substituted or unsubstituted aryl radical or a saturated or unsaturated, optionally substituted heterocyclic radical can be bonded to the appropriate nitrogen atom. With respect to the substituents preferably bonded to the respective nitrogen atoms, reference is made to the above description of the compounds according to the invention. The above reactions and their implementation are well known to the person skilled in the art and are described in detail in standard works such as, for example, Houben-Weyl, Methoden der organischen Chemie [Methods of Organic Chemistry], Georg Thieme Verlag, Stuttgart.

The ester derivatives according to the invention can be converted into the corresponding free carboxylic acids in a conventional manner, such as, for example, by basic ester hydrolysis.

If desired, the compounds according to the invention can be converted into their physiologically acceptable salts. This can be carried out either by reaction with an organic or inorganic base such as, for example, an alkali metal hydroxide or alkaline earth metal hydroxide such as KOH, NaOH, LiOH, Mg(OH)₂ or Ca(OH)₂, as a result of which the terminal carboxyl group is deprotonated and the corresponding carboxylate is formed, or by reaction with an organic or inorganic acid such as, for example, hydrochloric acid, sulphuric acid, phosphoric acid, mandelic acid, oleic acid, linoleic acid or p-toluenesulphonic acid, as a result of which one or more of the nitrogen atoms present are protonated.

The compounds of the formula (Ia) serving as starting substances can be prepared by conventional methods. The linkage of the toxophore to amino acid units can be carried out by conventional methods of peptide chemistry (cf., for example, Jakubke/Jeschkeit: Aminosäuren, Peptide, Proteine [Amino acids, Peptides, Proteins]; Verlag Chemie 1982, Houben-Weyl, Methoden der Organischen Chemie [Methods of Organic Chemistry], Georg Thieme Verlag Stuttgart, Fourth Edition; Volume 15.1 and 15.2, edited by E. Wünsch) and is also described, for example, in WO 96/31532 and WO 98/51703, whose contents are inserted here by means of reference.

If appropriate, a spacer unit Sp should be bonded to an appropriate toxophore-amino acid conjugate or a toxophore or a side-chain modification of amino acids which can be present should be carried out by bonding of a spacer unit Sp'. Possible spacer units Sp according to the present invention are an arylaminocarbonyl or an arylaminothiocarbonyl radical having 7-11 carbon atoms or an alkanedicarboxylic acid radical having 3 to 8 carbon atoms or a carbonyl or a thiocarbonyl radical. Possible side-chain radicals Sp' according to the present invention are an arylaminocarbonyl or an arylaminothiocarbonyl radical having 7-11 carbon atoms.

The bonding of the appropriate arylaminocarbonyl or arylaminothiocarbonyl radicals can be carried out as described above by reaction of the toxophore or of the toxophore-amino acid conjugate with an appropriate aryl isocyanate or aryl isothiocyanate. Reactions of this type are also described, for example, in WO 96/31532.

The bonding of the appropriate carbonyl or thiocarbonyl radicals can be carried out as described above by reaction of the toxophore or of the toxophore-amino acid conjugate with phosgene or a substitute for phosgene such as, for example, trichloromethyl chloroformate or thiophosgene.

The bonding of the appropriate alkanedicarboxylic acid radicals can be carried out by conventional methods known to the person skilled in the art, such as are described, for example, in Houben-Weyl, Methoden der Organischen Chemie [Methods of Organic Chemistry], Georg Thieme Verlag Stuttgart, fourth edition; Volume 15.1 and 15.2, edited by E. Wünsch. For example, free amino functions of the toxophore or of the toxophore-amino acid conjugate can be reacted with appropriate alkanedicarboxylic acids optionally activated as described above or alkanedicarboxylic anhydrides such as succinic or glutaric anhydride in the presence of a base in a solvent such as dichloromethane.

Bases which can be employed here are, for example, triethylamine, ethyldiisopropylamine, pyridine, N,N-dimethylaminopyridine or other bases conventionally used in steps of this type such as, for example, Hünig's base.

Although according to the invention it is preferred to first synthesize the toxophore-linking unit conjugate (1a), it is also possible, of course, to build up the linking unit in series first on the moiety addressing α_{ν}β₃ integrin receptors or to bond it as a whole and then to connect the conjugate thus obtained to the toxophore.

According to a preferred embodiment of the present invention, the synthesis of the compounds according to the invention is carried out on a solid phase such as a polystyrene resin, particularly preferably a commercially available Wang polystyrene resin. The resin is in this case first swollen in a solvent such as dimethylformamide (DMF). The moiety of the formula (III) addressing _{ν 3} integrin receptors is then bonded to the resin via its carboxyl function by standard processes. For example, the bonding of the carboxylic acid to the resin can be carried out in the presence of a base such as pyridine and a reagent activating the carboxyl unit, such as an acid halide, for example dichlorobenzoyl chloride, in a solvent such as dimethylformamide (DMF). However, other reagents conventionally used for this purpose can also be employed. The reaction mixture is stirred at room temperature and normal pressure for at least 2 hours, preferably 12 hours, particularly preferably approximately 24 hours, the carboxylic acid being employed in an excess with respect to the loading of the solid phase, preferably in a two- to throe-fold excess. All reactions described herein can then be carried out on the moiety of the formulae (III) bound to the resin and addressing α_{ν}β₃ integrin receptor, as described here.

According to a preferred embodiment of the present invention, the toxophore is camptothecin or a camptothecin derivative such as 9-aminocamptothecin. The linkage of these toxophores to the linking unit can be carried out via the C20 OH group or, in the case of 9-aminocamptothecin, via the free amino group.

The camptothecin unit used as a starting compound can be present in the 20(R) or in the 20(S) configuration or as a mixture of these two stereoisomeric forms. The 20(S) configuration is preferred.

After linkage of the first amino acid to camptothecin, diastereomer mixtures can be formed. Pure diastereomers of the compounds according to the invention can be prepared by the processes indicated above, for example, by separating the diastereomers in a suitable manner after coupling of the first amino acid unit to the camptothecin and subsequent protective group removal.

The radical of the formula (III) addressing α_{ν}β₃ integrin receptors can be prepared from commercially obtainable starting compounds via the following steps:
The essential steps of the preparation process according to the invention are the reaction of a β-amino acid of the formula (IIIa) 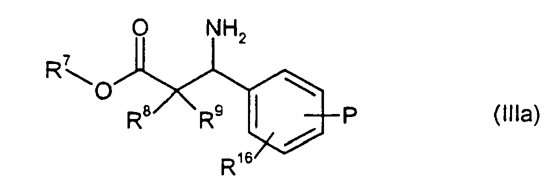
where
- P: s -(CH₂)ₘNO₂, -(CH₂)ₘO-C₁₋₆-alkyl, -(CH₂)ₘSO₂P', -(CH₂)ₘCOP', -(CH₂)ₘCH₂O-C₁₋₆-alkyl, where m is in each case an integer of 0 or 1;
- P': is -OH, -O-C₁₋₆-alkyl,
and the other radicals are as defined above, where R⁷ can additionally be a solid phase conventionally used for carrying out a solid-phase reaction;
with a compound R¹⁰-A to give a compound of the formula (IIIb) 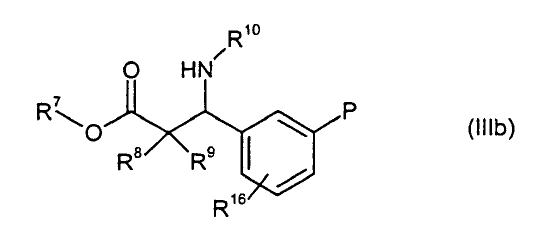 where
- R¹⁰: is -SO₂R^{10'}, -COOR^{10"} or- COR^{10'};
- R^{10'}: and R^{10"} are as defined above;
- A: is -Cl, -Br, -I, -O-triflyl, -O-tosyl, -O-C₁₋₆-alkyl, -O-CO-C₁₋₆-alkyl, -O-CO-O-C₁₋₆-alkyl, -OC(CH₃)=CH₂;
and the other radicals are as defined above;
the conversion of the radical P into the radical Q,
where
- Q: is -(CH₂)ₘNH₂, -(CH₂)ₘOH, -(CH₂)ₘCH₂OH, -(CH₂)ₘSO₂A, -(CH₂)ₘCOA,
- A: is as defined above;
- m: is an integer of 0 or 1;
the reaction of the compound (IIIb) obtained above with a compound of the formula (IIIc) 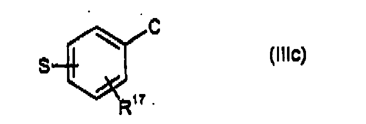 where
- S: is ASO₂(CH₂)ₙ-,
where
n is an integer of 0
A is as defined above; and
C is -NO₂ or 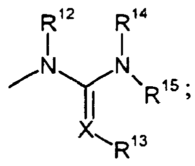
and
X, R¹², R¹³, R¹⁴ and R¹⁵ are as defined above;
to give a compound of the formula (IIId) 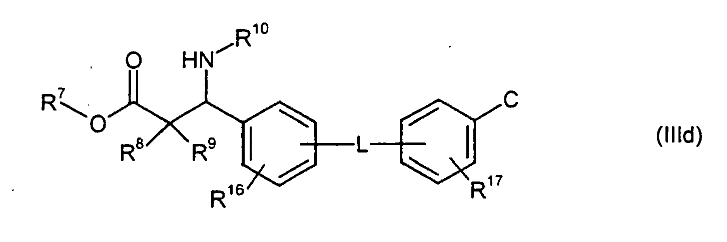 where the radicals are as defined above;
if appropriate the conversion of C, if C is a nitro group, into an optionally cyclic urea, thiourea or guanidine unit with retention of the radical (III); and
if appropriate the removal of protective groups and/or derivatization of nitrogen atoms present at preferred points of time in the preparation process and/or conversion of the compound obtained into the free acid and/or conversion of the compound obtained into one of its physiological salts by reaction with an inorganic or organic base or acid.

The β-amino acid derivatives of the formula (IIIa) are either commercially obtainable or are accessible in a simple manner by standard chemical processes, such as are known to any person skilled in the art and are described in standard works such as Houben-Weyl, Methoden der organischen Chemie [Methods of Organic Chemistry], Georg Thieme-Verlag, Stuttgart. In particular, reference is made to the preparation processes for β-amino acid derivatives described by Rodionow et al., J. Am. Chem. Soc. 51, 1929, 844-846, Kunz et al., Angew. Chem. 101, 1989, 1042-1043 and Ishihara et al., Bull. Chem. Soc. Jpn., 68, 6, 1995, 1721-1730.

According to a preferred embodiment of the present invention, the -amino acid derivatives of the formula (IIIa) are obtained by reaction of malonic acid with a benzaldehyde derivative of the formula (IIIa') 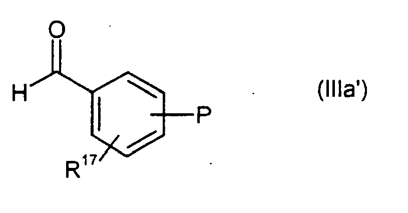 where R¹⁷ and P are as defined above, in the presence of ammonia, ammonium compounds or amines. Instead of malonic acid, an ester, if appropriate with addition of a base conventionally employed for these purposes, such as NaH or a sodium alkoxide, preferably sodium methoxide or sodium ethoxide, can also be used. Preferably, an ammonium compound such as, for example, ammonium acetate is employed as the nitrogen compound.

The benzaldehyde derivatives (IIIa') are either commercially obtainable or are accessible in a simple manner by standard chemical processes, such as are known to any person skilled in the art and are described in standard works such as Houben-Weyl, Methoden der organischen Chemie [Methods of Organic Chemistry], Georg Thieme-Verlag, Stuttgart.

According to a preferred embodiment of the present invention, a nitrobenzaldehyde derivative such as 3- or 4-nitrobenzaldehyde or an alkoxybenzaldehyde derivative such as 3- or 4-methoxybenzaldehyde is employed as the compound of the formula (IIIa').

According to a preferred embodiment of the present invention, the β-amino acid of the formula (IIIa) is obtained by reaction of approximately equimolar amounts of malonic acid, ammonium acetate and 3-nitrobenzaldehyde or 3-methoxybenzaldehyde in a solvent such as isopropanol with heating for a number of hours, preferably 2 to 6 hours, at 50 to 110°C, preferably with reflux of the solvent, in the surrounding atmosphere (i.e. in the air and under normal pressure).

For the following reaction steps, the carboxyl group is blocked by a conventional protective group P. Protective groups of this type are known to the person skilled in the art and do not have to be expressly mentioned here. The carboxyl group is particularly preferably esterified, where P is a C₁₋₆-alkyl such as, for example, methyl, ethyl, propyl, isopropyl, butyl, isobutyl, t-butyl, pentyl, isopentyl, neopentyl, hexyl, a C₃₋₇-cycloalkyl such as, for example, cyclopropyl, cyclopropylmethyl, cyclobutyl, cyclopentyl, cyclohexyl, an aryl such as, for example, phenyl, benzyl, tolyl or a substituted derivative thereof.

Furthermore, the preparation process according to the invention for the radicals of the formula (III) can be carried out on a solid phase.
In this case, the carboxyl radical can be connected to any solid phase conventionally used for reactions of this type, such as a polystyrene resin, for example a Wang polystyrene resin.

According to a preferred embodiment according to the invention, the carboxyl group of the above β-amino acid is esterified by reaction with an alcohol such as ethanol or a polymer conventionally used for carrying out a solid-phase reaction. This can be carried out under conditions known to the person skilled in the art, such as acid catalysis and, if appropriate, addition of a dehydrating agent such as dicyclo-hexyl-carbodiimide. Preferably, however, the β-amino acid is suspended in the appropriate alcohol present in an excess, such as ethanol, HCl is passed through for a period of approximately 30 minutes to approximately 2 hours and the mixture is then heated in a surrounding atmosphere for a number of hours, preferably approximately 1 to 6 hours and particularly preferably approximately 3 to 5 hours, at approximately 50 to approximately 100°C, preferably under reflux of the alcohol.

The carboxyl-protected β-amino acids accessible in this way are reacted with a suitable sulphonating, carbamoylating or acylating reagent in order to obtain the corresponding sulphonamide, carbamate or amide derivatives. The sulphonating reagent used is preferably a sulphonyl chloride of the formula R^{10"}-SO₂Cl or a carbamoyl chloride of the formula R^{10"}-OCOCl, where R^{10"} is a C₁₋₁₀-alkyl such as methyl, ethyl, propyl, isopropyl, butyl, isobutyl, t-butyl, pentyl, isopentyl, neopentyl, hexyl, heptyl, octyl, nonyl, decyl, cyclopropyl, cyclobutyl, cyclopentyl, cyclohexyl, or camphor-10-yl, an aryl such as phenyl, benzyl, tolyl, mesityl or substituted derivatives of these such as -C₆H₂(CH₃)₃, -C₆(CH₃)₅, -CH₂C₆H₂(CH₃)₃, 2-chlorophenyl, 3-chloro-phenyl, 4-chlorophenyl, 3-aminophenyl, 4-aminophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 4-chlorophenylmethyl, 2,4-dichlorophenylmethyl, 2,6-dichlorophenylmethyl, 2-methoxycarbonylphenylmethyl, 3-trifluoromethylphenyl, 4-trifluoromethylphenyl, 3,5-bis(trifluoromethyl)phenyl, 4-trifluoromethoxyphenyl, phenyl-methyl, 2-acetamido-4-methyl-thiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-meth-ylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methyl-benzothiazol-2-yl, N-methoxycarbonyl-piperidin-3-yl, thio-phen-2-yl, isoxazol-5-yl, 2-chloropyridin-3-yl, pyridin-3-yl, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl, 8-quinolinyl, or a heterocyclic analogue of the abovementioned cyclic radicals. Instead of the abovementioned sulphonyl or carbamoyl chlorides, it is also possible to employ the corresponding fluorides, bromides or iodides. As acylating reagent, the appropriate carboxylic acid halides or carboxylic acid anhydrides are reacted with the amino group, the appropriate C₁₋₆-alkyl carboxylic acid chlorides such as methyl-, ethyl-, propyl-, isopropyl-, butyl-, isobutyl-, t-butyl-, pentyl-, isopentyl-, neopentyl-, hexyl-, C₃₋₇-cycloalkyl such as cyclopropyl-, cyclobutyl-, cyclopentyl-, cyclohexyl-, aryl such as phenyl-, benzyl-, tolylcarboxylic acid chlorides or substituted derivatives thereof being preferred according to the invention. For the preparation of the urea or thiourea radicals, the amino group is preferably first reacted with a carbonic acid or thiocarbonic acid derivative such as a chloroformic acid ester or thiophosgene and then with a desired amine. The above reactions and their implementation are well known to the person skilled in the art and are described in detail in standard works such as, for example, Houben-Weyl, Methoden der organischen Chemie [Methods of Organic Chemistry], Georg Thieme Verlag, Stuttgart.

According to a preferred embodiment of the invention, the carboxyl-protected β-amino acid of the formula (IIIa) is treated with an equimolar amount or a slight excess of the appropriate sulphonylating agent, for example phenylsulphonyl chloride, or acylating agent, for example mesitylacetyl chloride, with cooling, preferably at 0°C, in a solvent such as pyridine or dioxane in a surrounding atmosphere in the presence of a base such as an amine, preferably triethylamine or diisopropylethylamine, and the mixture is stirred at this temperature for a period of approximately 10 minutes to approximately 2 hours. In the case of sulphonylation, this is followed by stirring at room temperature for a number of hours, preferably approximately 2 to 6 hours.

Before the synthesis of the linker group L, the radical P of the compound of the formula (IIIb) must be converted into a group Q which can participate in a nucleophilic substitution either as a nucleophilic reagent or as a substrate. If P includes a nitro group, this will be reduced to the corresponding amino group, which according to the present invention can preferably be carried out by addition of tin(II) chloride to a solution of the compound of the formula (IIIb) in a solvent such as ethanol and subsequent heating to approximately 50 to 110°C, preferably under reflux of the solvent, for a number of hours, preferably approximately 1 to 4 hours, in a surrounding at-mosphere. If P includes an ether group, the liberation of the corresponding hydroxyl group is preferably carried out by addition of a Lewis acid such as boron tribromide in a solvent such as dichloromethane with cooling, preferably at -78°C, and subsequent stirring for a number of hours, preferably 6 to 24 hours, at room temperature. If P includes a sulphonic acid or carboxylic acid group, a conversion into the corresponding sulphonyl or carboxylic acid halide is preferably carried out. This can be carried out in a manner known to the person skilled in the art, for example by reaction of the corresponding sulphonic or carboxylic acid with thionyl chloride.

The compound prepared in this way is then reacted with a compound of the formula (IIIc) 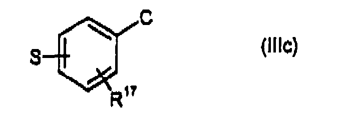 where
- S: is ASO₂(CH₂)ₙ-,
where
n is an integer of 0
A is as defined above; and
- C: is -NO₂ or 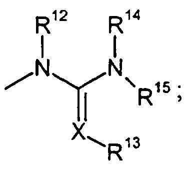 and
- X, R¹², R¹³, R¹⁴ and R¹⁵: are as defined above;
to give a compound of the formula (IIId) 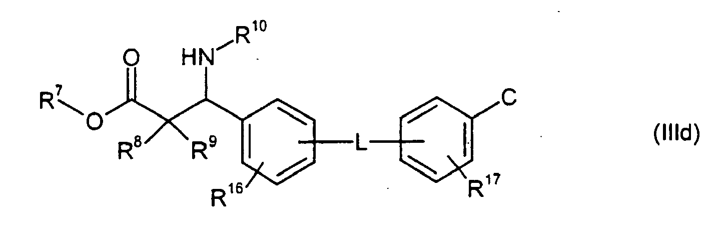 where the radicals are as defined above. This reaction formally represents the substitution of a leaving group in one of the starting compounds by a nucleophilic unit in the other starting compound in each case.

According to a preferred embodiment of the present invention, the reactants are mixed together in approximately equimolar amounts in the presence of a base such as pyridine or sodium hydride and, if appropriate, in a solvent such as, for example, tetrahydrofuran (THF) or dimethylformamide (DMF) in a surrounding atmosphere at room temperature or with cooling, preferably at approximately 0°C, and stirred for a number of hours, preferably approximately 1 h to approximately 24 hours, at room temperature or with cooling, for example at 0°C.

The compounds of the formula (IIId) thus obtained are converted into the radicals of the formula (III) according to the invention by conversion of the terminal nitro group into an open-chain or cyclic guanidine, urea or thiourea unit.

For this, the nitro group is first converted according to the invention into an amino group, preferably by addition of a customary reducing agent such as tin-(II) chloride, if appropriate in the presence of solvents such as ethanol, by stirring the reaction mixture with heating at approximately 50 to 110°C, preferably under reflux of the solvent, in a surrounding atmosphere for approximately 2 hours.

The amino group thus obtained is then converted into a guanidine, urea or thiourea unit. For this, the above amino group is first preferably reacted with a carbonic acid ester or thiocarbonic acid ester derivative in a solvent such as dimethylformamide (DMF) in the presence of mercury-(II) chloride with cooling, preferably at approximately 0°C, and stirring for approximately 10 minutes to approximately 3 hours with cooling, preferably at approximately 0°C, and if appropriate subsequently at room temperature. The carbonic acid ester or thiocarbonic acid ester derivative employed can preferably be phosgene, triphosgene, thiophosgene, chloroformic acid esters or thiopseudourea derivatives, commercially obtainable chloroformic acid esters being preferred for the preparation of the urea derivatives, thiophosgene being preferred for the preparation of the thiourea derivatives and thiopseudourea derivatives being preferred for the preparation of guanidine derivatives.

The carbamates or isothiocyanates formed in this way can be converted into the corresponding urea, thiourea and guanidine derivatives by reaction with appropriate amines. The amines used can be substances of the formula HNRR', where R and R' independently of one another or simultaneously can be hydrogen, a substituted or unsubstituted alkyl or cycloalkyl radical, a substituted or unsubstituted aryl radical, a saturated or unsaturated, optionally substituted heterocyclic radical, an alkylamine radical, an alkylamide radical or can be connected to one another and together with the nitrogen atom can form an optionally substituted heterocyclic ring system which can be saturated or unsaturated and/or can contain further heteroatoms. With respect to the preferred radicals on the amine, reference is made to the above description of the compounds according to the invention. According to the invention, the carbamate or isothiocyanate is preferably reacted with an amine at room temperature with stirring for approximately 1 to 5 hours, preferably approximately 2 to 3 hours, in the presence of an auxiliary base such as diisopropylethylamine in a solvent such as dimethylformamide (DMF). In the case of the preparation of cyclic guanidine derivatives, the corresponding isothiocyanate is preferably first heated in ethanol for a number of hours, preferably approximately 12 to 24 hours, and then heated with a diamine such as diaminoethane in a solvent such as toluene, dimethylformamide (DMF) or a mixture of both.

According to a further preferred embodiment of the present invention, it is also possible to generate the above guanidine, urea or thiourea group on the compound of the formula (IIIc) in the above manner and then to react the compound of the formula (IIIc) thus obtained with the compound of the formula (IIIb) in the manner described above.

The compounds obtained according to the process explained above can furthermore be derivatized by removal of protective groups which may be present, further substitution of nitrogen atoms present at preferred positions in the preparation process and/or conversion of the compound obtained into the free acid and/or its physiologically acceptable salts. For example, the t-butoxymethoxycarbonyl groups conventionally used as protective groups for nitrogen atoms are removed in an acidic medium, for example by addition of trifluoroacetic acid. Suitable alkylating agents for derivatization of nitrogen atoms are reagents conventionally used for this purpose in this step, to which, for example, a substituted or unsubstituted alkyl or cycloalkyl radical, a substituted or unsubstituted aryl radical or a saturated or unsaturated, optionally substituted heterocyclic radical can be bonded to the corresponding nitrogen atom. With respect to the substituents preferably bonded to the respective nitrogen atoms, reference is made to the above description of the compounds according to the invention. The above reactions and their implementation are well known to the person skilled in the art and are described in detail in standard works such as, for example, Houben-Weyl, Methoden der organischen Chemie [Methods of Organic Chemistry], Georg Thieme Verlag, Stuttgart. The ester derivatives according to the invention can be converted into the corresponding free carboxylic acids in a conventional manner, such as, for example, by basic ester hydrolysis.

If desired, the compounds according to the invention can be converted into their physiologically acceptable salts. This can be carried out either by reaction with an organic or inorganic base such as, for example, an alkali metal hydroxide or alkaline earth metal hydroxide such as KOH, NaOH, LiOH, Mg(OH)₂ or Ca(OH)₂, whereby the terminal carboxyl group is deprotonated and the corresponding carboxylate is formed, or by reaction with an organic or inorganic acid such as, for example, hydrochloric acid, sulphuric acid, phosphoric acid, mandelic acid, oleic acid, linoleic acid or p-toluenesulphonic acid, whereby one or more of the above nitrogen atoms are protonated.

The conjugates according to the invention can be used as active compound components for the production of medicaments against carcinomatous disorders. For this, they can be converted into the customary formulations such as tablets, coated tablets, aerosols, pills, granules, syrups, emulsions, suspensions and solutions in a known manner using inert, non-toxic, pharmaceutically suitable excipients or solvent. Preferably, the compounds according to the invention are used here in an amount such that their concentration in the total mixture is approximately 0.5 to approximately 90% by weight, the concentration, inter alia, being dependent on the corresponding indication of the medicament.

The abovementioned formulations are produced, for example, by extending the active compounds with solvents and/or excipients having the above properties, where, if appropriate, additionally emulsifiers or dispersants and, in the case of water as the solvent, alternatively an organic solvent, have to be added.

The medicaments according to the invention can be administered in a customary manner.

The present invention is illustrated below with the aid of non-restricting examples and comparison examples.

### Examples

In the examples below, all quantitative data, if not stated otherwise, relate to percentages by weight. The mass determinations were carried out by high-performance liquid chromatography-mass spectrometry (HPLC-MS) using the electron spray ionization (ESI) method or by FAB or MALDI mass spectroscopy.

### List of the abbreviations used

- HPLC: - high-performance liquid chromatography
- RP: - reverse phase
- ACN: - acetonitrile
- DMF: - dimethylformamide
- DCM: - dichloromethane
- THF: - tetrahydrofuran
- DIEA: - diisopropylethylamine (Hünig's base)
- NMP: - N-methylpyrrolidone
- TFA: - trifluoroacetic acid
- Fmoc: - 9-fluorenylmethoxycarbonyl
- RT: - room temperature
- MTBE: - methyl tert-butyl ether
- Boc: - tert-butyloxycarbonyl
- TLC: - thin-layer chromatography
- DMAP: - dimethylaminopyridine
- DMSO: - dimethyl sulphoxide

### I. Preparation of camptothecin conjugates

### 1.1. 20-O-L-Valyl-camptothecin trifluoroacetate

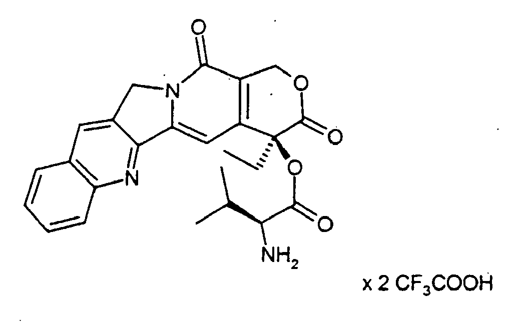

A suspension of 10 g (28.7 mmol) of 20(S)-camptothecin in 500 ml of absolute dichloromethane is treated with stirring with 14 g (2 eq.) of N-(tert-butoxycarbonyl)-valine-N-carboxyanhydride and 1 g of 4-(N,N-dimethylamino)-pyridine. After heating under reflux for 4 days, the mixture is concentrated in vacuo. The residue is stirred with 100 ml of MTBE for 20 min. 200 ml of petroleum ether are then added and the mixture is filtered. 14.9 g of the Boc-protected intermediate compound are obtained, which can contain small amounts of D-valine epimer which, however, can be removed without problems after removal of the protective group.

11.65 g of this Boc-protected intermediate compound are then stirred at 5°C for 1 h in a mixture of 300 ml of dichloromethane and 70 ml of anhydrous trifluoroacetic acid. After concentrating in vacuo to a small volume, the product is precipitated with diethyl ether and thoroughly washed with diethyl ether. The product is again precipitated from dichloromethane/methanol using diethyl ether. If appropriate, the crude product is again taken up in 40 ml of methanol, and the solution is treated with 120 ml of MTBE and cooled to 0°C. The precipitate is filtered off and 9.4 g (80%) of 20-O-(valyl)-camptothecin trifluoroacetate are obtained after drying. [TLC: acetonitrile/water (20: 1); R_{f} = 0.39].

### I.2 -[L-Valyl-camptothecin]-benzyl L-glutamate trifluoroacetate

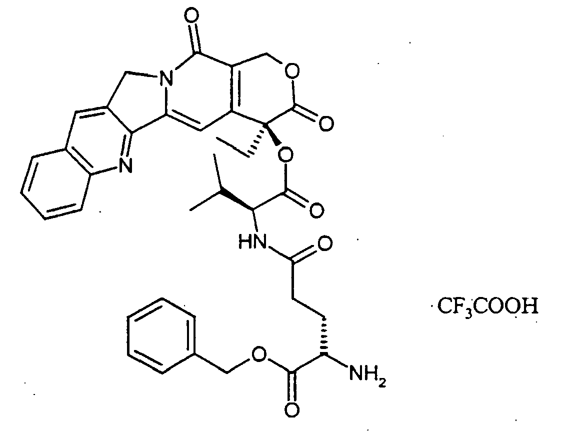

400 mg (1.185 mmol) of benzyl N-tert-butoxycarbonyl-glutamate are dissolved in 40 ml of DMF and treated with 273 mg (1.2 eq) of N-(3-dimethylaminopropyl)-N'-ethylcarbodiimide hydrochloride and with 240 mg (1.5 eq) of hydroxybenzotriazole. After 1 h, 665 mg (1.185 mmol) of the compound from Example I.1 and 811 µl of Hünig's base are added. The coupling reaction is complete after 2 h. The reaction mixture is concentrated in vacuo. The residue is taken up in dichloromethane, the mixture is extracted twice with water and then the organic phase is dried and concentrated again. The residue is then taken up in dichloromethane/methanol, a little ether is added and it is then precipitated with petroleum ether. This purification process is repeated and the intermediate is filtered off and dried (yield: 752 mg = 83 %).

100 mg (0.13 mmol) of this Boc-protected intermediate compound are then stirred at room temperature for 1 h with 10 ml of dichloromethane and 1 ml of anhydrous trifluoroacetic acid. After concentrating in vacuo, the product is taken up in dichloromethane/methanol, precipitated with diethyl ether and thoroughly washed with diethyl ether. The product is again precipitated from dichloromethane/methanol using diethyl ether. The precipitate is filtered off and 85 mg (84%) of the target compound are obtained after drying.

[TLC: acetonitrile/water 10:1 Rf = 0.4]. Analogously, the following, partially protected camptothecin-peptide conjugates were prepared by reaction of appropriate camptothecin-amino acid conjugates with further partially protected amino acids. If appropriate, protective groups are removed according to known methods:

| Example | Compound | R_{f} value |
|---|---|---|
| I.3 | 20-O-[L-Histidyl-L-valyl]-camptothecin trifluoroacetate | 0.4 ¹⁾ |
| I.4 | 20-O-{N^{ε}-[Fluorenyl-9-methoxycarbonyl]-L-lysyl-L-valyl}-camptothecin trifluoroacetate | 0.4 ²⁾ |
| I.5 | 20-O-[L-Glutamic acid L-valyl]-camptothecin | 0.28 ¹⁾ |
| I.6 | 20-O-(Glutaryl-glycyl-L-valyl)-camptothecin | 0,7 ¹⁾ |
| I.7 | 20-O-(Glutaryl-glycyl-L-leucyl)-camptothecin | 0.25 ³⁾ |
| I.8 | 20-O-(Glutaryl-L-leucyl-glycyl-L-leucyl)-camptothecin | 0.68 ¹⁾ |
| I.9 | 20-O-(Glutaryl-glycyl-L-leucyl-L-valyl)-camptothecin | 0.4 ⁴⁾ |
| I.10 | 20-O-(Glutaryl-L-prolyl-L-leucyl-glycyl-L-leucyl)-camptothecin | 0.2 ³⁾ |
| ¹⁾ Acetonitrile/water/glacial acetic acid 5:1:0.2 | | |
| ²⁾ Dichloromethane/methanol 10:1 | | |
| ³⁾ Acetonitrile/water 10:1 | | |
| ⁴⁾ Dichloromethane/methanol/glacial acetic acid 10:1/0.1 | | |

### 1.11 20-O-Succinylcamptothecin

1 g (2.9mmol) of camptothecin is initially introduced into 50 ml of dichloromethane and treated with 100 mg of dimethylaminopyridine (DMAP) and 600 mg of succinic anhydride. After a reaction time of 44 h, equal amounts of DMAP and succinic anhydride are again added and the mixture is stirred for a further 48 h. It is purified by flash chromatography on silica gel (acetonitrile --> acetonitrile/water (20:1)). The corresponding fractions are concentrated and the product is precipitated from dichloromethane/methanol using ether. Yield: 160 mg (13%). [TLC: (acetonitrile/water (10:1); R_{f} = 0.6].

### I.12. 20-O-[N^{α}-(Phenylamino-thiocarbonyl)-L-lysyl-L-valyl]-camptothecin

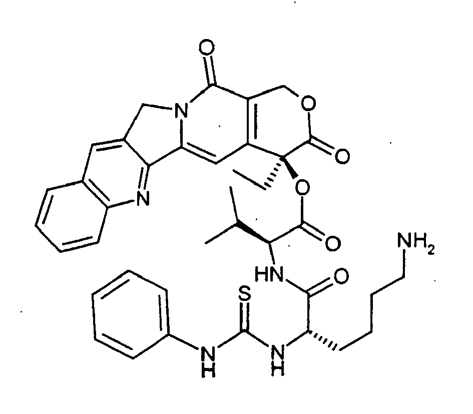

666 mg (0.73 mmol) of the compound from Example 1.4 are dissolved in 40 ml of DMF and treated with 500 µl of Hünig's base. 87 µl (1 eq) of commercially obtainable phenyl isothiocyanate are then added and the mixture is stirred overnight. It is then concentrated and the residue is precipitated twice from dichloro-methane/methanol using ether. The precipitate is filtered off and after drying 614 mg (90%) of the protected intermediate are obtained. [TLC: (acetonitrile) R_{f} = 0.66].

614 mg (0.658 mmol) of this intermediate compound are dissolved in 20 ml of DMF and treated with 2 ml of piperidine. After stirring at RT for 45 minutes, the mixture is concentrated. The residue is precipitated twice from dichloromethane/methanol using ether. It is filtered off and 365 mg (78%) of the target compound are obtained after drying. [TLC: (acetonitrile/water/glacial acetic acid 5:1:0.2) R_{f} = 0.46].

### II. Preparation of non-peptide integrin ligands or intermediates suitable for linkage

### II. 1 Ethyl-3-amino-(3-(3-[N,N'-bis-t-butoxycarbonyl-guanidino]-benzene-sulphonylamino)-phenyl]-3-propanoic acid

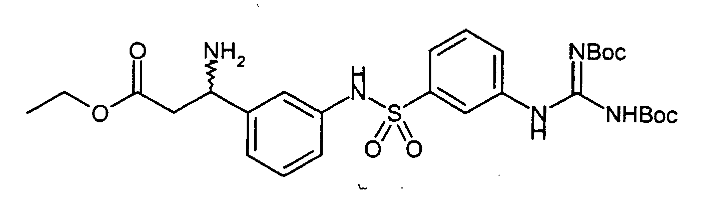

### II. 1a: 3-Amino-3-nitrophenylpropionic acid hydrochloride

3-Nitrobenzaldehyde (151 g), ammonium acetate (94 g) and malonic acid (127 g) were heated under reflux in isopropanol (1 1) for 5 h. The solution was filtered and the precipitate was washed with hot isopropanol (0.71). The crude product was dried in vacuo (yield: 146 g). ¹H-NMR (D₄-MeOH): 3.09 (m, 2 H), 4.88 (m, 1 H), 7.74 (t, 1 H), 7.90 (d, 1 H), 8.33 (d, 1 H), 8.43 (s, 1 H).

### II.1b: 3-Amino-3-(3-nitrophenyl)-propionate hydrochloride

3-Amino-3-nitrophenylpropionic acid hydrochloride from II.1a (60 g) were suspended in 660 ml of ethanol and gaseous HCl was passed in for 1 h. The reaction mixture was then heated under reflux for 4 h and was cooled and concentrated. A white solid was obtained (yield: 62 g). ¹H-NMR (D₄-MeOH): 1.22 (t, 3 H), 3.12 (dd, 1 H), 3.20 (dd, 1 H), 4.18 (q, 2 H), 4.95 (t, 1 H), 7.77 (t, 1 H), 7.94 (d, 1 H), 8.35 (d, 1 H), 8.43 (s, 1 H).

### II.1c: Ethyl 3-allyloxycarbonylamino-3-(3-nitrophenyl)-propionate

Diisopropylethylamine (66 ml) and allyl chloroformate (22 ml) in 150 ml of methylene chloride were added at 0°C to a solution of ethyl 3-amino-3-(3-nitrophenyl)-propionate hydrochloride from II.1b (47 g) in 350 ml of methylene chloride. After a reaction time of 30 min, HCl (1N, 100 ml) was added. The organic phase was separated off, washed with water, dried over MgSO₄ and concentrated. A white solid was obtained (yield: 56.4 g).
¹H-NMR (CDCl₃): 1.19 (t, 3 H), 2.91 (d, 2 H), 4.09 (q, 2 H), 4.57 (m, 2 H), 5.18-5.26 (m, 3 H), 5.92 (m, 1 H), 6.04 (m, 1 H), 7.52 (t, 1 H), 7.68 (d, 1 H), 8.14 (d, 1 H), 8.20 (s, 1 H).

### II.1d: : Ethyl 3-allyloxycarbonylamino-3-(3-aminophenyl)-propionate

Tin(II) chloride (64.6 g) was added to a solution of ethyl 3-allyloxycarbonylamino-3-(3-nitrophenyl)-propionate from II.1c (18.8 g) in 245 ml of ethanol, and the reaction mixture was heated under reflux for 2 h. After cooling the solution, it was adjusted to pH = 7 using 2N NaOH, briefly heated, cooled again and filtered. It was then extracted with dichloromethane, and the org. phase was dried (MgSO₄) and concentrated. 10.9 g of product were obtained.
¹H-NMR (CDCl₃): 1.18 (t, 3 H), 2.80 (m, 2 H), 4.08 (q, 2 H), 4.56 (m, 2 H), 5.06 (m, 1 H), 5.20 (d, 1 H), 5.30 (d, 1 H), 5.70 (m, 1 H), 5.90 (m, 1 H), 6.57 (d, 1 H), 6.62 (s, 1 H), 6.68 (d, 1 H), 7.11 (t, 1 H).

### II.1e: Ethyl 3-allyloxycarbonylamino-3-(3-[3-nitrophenylsulphonylamino]-phenyl)-propionate

3-Nitrobenzenesulphonyl chloride (9.9 g) was added at 0°C to a solution of ethyl 3-allyloxycarbonylamino-3-(3-aminophenyl)-propionate from II.1d (10.9 g) in 100 ml of pyridine. After a reaction time of 2 h at 0°C, the mixture was concentrated, treated with 1 N HCl (150 ml) and extracted with dichloromethane. After drying (MgSO₄), the solvent was removed, and 16.8 g of product were obtained. ¹H-NMR (CDCl₃): 1.18 (t, 3 H), 2.78 (m, 2 H), 4.06 (q, 2 H), 4.53 (m, 2 H), 5.04 (q, 1 H), 5.18-5.35 (m, 2 H), 5.80-5.95 (m, 2 H), 6.79 (d, 2 H), 7.00 (d, 1 H), 7.03 (s, 1 H), 7.11 (d, 1 H), 7.24 (t, 1 H), 7.65 (t, 1 H), 8.03 (d, 1 H), 8.38 (d, 1 H), 8.61 (s, 1 H).

### II.1f: Ethyl 3-allyloxycarbonylamino-3-(3-[3-aminophenylsulphonylamino]-phenyl)-propionate

Tin(II) chloride (39.7 g) was added to a solution of ethyl 3-allyloxycarbonylamino-3-(3-[3-nitrophenylsulphonylamino]-phenyl)-propionate from II.1e (16.8 g) in 155 ml of ethanol and the reaction mixture was heated under reflux for 2 h. After cooling, the solution was adjusted to pH = 7 using 2N NaOH, briefly heated, cooled again and filtered. It was then extracted with dichloromethane, and the org. phase was dried (MgSO₄) and concentrated. 7.3 g of product were obtained. ¹H-NMR (CDCl₃): 1.12 (t, 3 H), 2.73 (m, 2 H), 3.91 (s, 2 H), 4.02 (q, 2 H), 4.49 (m, 2 H), 5.01 (q, 1 H), 5.15 (d, 1 H), 5.24 (d, 1 H), 5.75 (m, 1 H), 5.84 (m, 1 H), 6.47 (s, I H), 6.69 (d, 1 H), 6.81 (d, 1 H), 6.88 (s, 1 H), 6.99 (d, 1 H), 7.03 (d, 1 H), 7.08-7.15 (m, 3 H).

### II.1g: Ethyl 3-allyloxycarbonylamino-3-(3-(3-[N,N'-Bis-t-butoxycarbonyl-guani-dino]-phenylsulphonylamino)-phenyl)-propionate

Triethylamine (6.5 ml), 1,3-bis(t-butoxycarbonyl)-2-methyl-2-isothiourea (8.23 g) and mercuric chloride (7.7 g) were added at 0°C to a solution of ethyl 3-allyloxycarbonylamino-3-(3-[3-aminophenylsulphonylamino]-phenyl)-propionate from II.1f (10.5 g) in 320 ml of DMF. After a reaction time of 30 min at 0°C, the mixture was stirred at room temperature for a further 1.5 h. The precipitate was removed by filtration and the solution was concentrated. After chromatography (methylene chloride/methanol (40:1)), 13.5 g of product were obtained. ¹H-NMR (CDCl₃): 1.16 (t, 3 H), 1.51 (s, 9 H), 1.56 (s, 9 H), 2.76 (m, 2 H), 4.05 (q, 2 H), 4.53 (m, 2 H), 5.05 (m, 1 H), 5.21 (m, 1 H), 5.30 (m, 1 H), 5.73 (m, 1 H), 5.90 (m, 1 H), 6.83 (s, 1 H), 7.01-7.09 (m, 3 H), 7.19 (t, 1 H), 7.36 (m, 2 H), 7.79 (d, 1 H), 8.12 (s, 1 H), 10.25 (s, 1 H), 11.80 (s, 1 H).

### II.1: Ethyl-3-amino-(3-(3-[N,N'-bis-t-butoxycarbonyl-guanidino]-benzenesulphonyl-amino)-phenyl]-3-propanoic acid

Acetic acid (1.6 ml), bistriphenylphosphinepalladium dichloride (110 mg) and tributyltin hydride (3.5 g) were added to a solution of II.1g in methylene chloride (150 ml). After 2.5 h, bistriphenylphosphinepalladium dichloride (110 mg) and tributyltin hydride (3.5 g) were added again and the mixture was stirred for 24 h. The solution was treated with satd. NaHCO₃, extracted with methylene chloride, dried over MgSO₄ and concentrated. After chromatography (methylene chloride/methanol), 4.8 g of product were obtained.
¹H-NMR (CDCl₃): 1.23 (t, 3 H), 1.51 (s, 9 H), 1.56 (s, 9 H), 2.54 (m, 2 H), 4.12 (q, 2 H), 4.33 (dd, 1 H), 7.05 (d, 1 H), 7.11 (m, 2 H), 7.21 (t, 1 H), 7.37 (t, 1 H), 7.42 (d, 2 H), 7.90 (d, 1 H), 8.03 (s, 1 H), 10.25 (s, 1 H), 11.80 (s, 1 H).

### II.12 3-(4-Aminobenzenesulphonylamino)-3-[3-(propylaminocarbonylamino-phenyl-sulphonylamino)-phenyl]-propionic acid

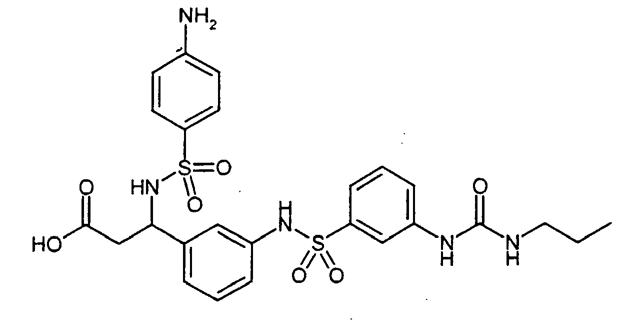

1.2 g of polystyrene Wang resin (loading 1.08 mmol/g) are swollen in DMF. The solvent is filtered off with suction and a solution of 841 mg of (3R,S)-3-(9-fluorenyl-methoxycarbonylamino)-3-(3-nitrophenyl)-propionic acid (amino acid reagent, prepared by protecting the free amino function of 3-amino-3-(3-nitrophenylpropionic acid with FMCO in a conventional and known manner) in 15 ml of DMF is added. After shaking at room temperature for 15 min, the suspension is treated with 350 µl of pyridine and 540 mg of 2,6-dichlorobenzoyl chloride. It is shaken overnight at room temperature. The resin is then washed with DMF, MeOH and DCM.

The resin is treated with a solution of 5400 mg of tin(II) chloride dihydrate in 12 ml of NMP and shaken overnight at room temperature. The resin is then washed with NMP, MeOH, THF and DCM.

The resin is treated with a solution of 450 µl of DIEA in 500 µl of THF and a solution of 430 mg of 3-nitrobenzenesulphonyl chloride in 500 µl of THF. It is shaken overnight at room temperature. The resin is then washed with DMF, MeOH and THF.

The resin is treated with a solution of 5400 mg of tin(II) chloride dihydrate in 12 ml of NMP and shaken overnight at room temperature. The resin is then washed with NMP, MeOH, THF and DCM.

The resin is treated with a solution of 500 µl of DIEA in 12 ml of THF/DCM 1:1 and a solution of 2757 mg of 4-nitrophenylchloroformic acid ester in 12 ml of THF/DCM 1:1. After shaking at room temperature for 45 min, it is washed with THF and DMF and a solution of 943 mg of propylamine and 2780 µl of DIEA in 20 ml of NMP is added. After shaking for 10 h, the resin is washed with DMF, MeOH, THF and DCM.

The resin is treated with a solution of 450 µl of DIEA in 500 µl of THF and a solution of 430 mg of 4-nitrobenzenesulphonyl chloride in 500 µl of THF. It is shaken overnight at room temperature. The resin is then washed with DMF, MeOH and THF.

The resin is treated with a solution of 5400 mg of tin(II) chloride dihydrate in 12 ml of NMP and shaken overnight at room temperature. The resin is then washed with NMP, MeOH, THF and DCM.

For the removal of the product, the resin is shaken for 1 h with 12 ml of TFA/DCM and filtered off, and the filtrate is concentrated in vacuo.

### II.13 3-Amino-3-[3-(propylaminocarbonylamino-phenyl-sulphonylamino)-phenyl]-propionic acid

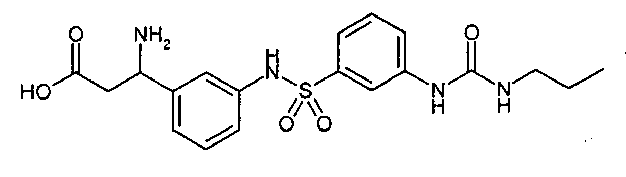

1.2 g of polystyrene Wang resin (loading 1.08 mmol/g) are swollen in DMF. The solvent is filtered off with suction and a solution of 841 mg of (3R,S)-3-(9-fluorenyl-methoxycarbonylamino)-3-(3-nitrophenyl)-propionic acid (amino acid reagent) in 15 ml of DMF are added. After shaking at room temperature for 15 min, the suspension is treated with 350 µl of pyridine and 540 mg of 2,6-dichlorobenzoyl chloride. It is shaken overnight at room temperature. The resin is then washed with DMF, MeOH and DCM.

The resin is treated with a solution of 5400 mg of tin(II) chloride dihydrate in 12 ml of NMP and shaken overnight at room temperature. The resin is then washed with NMP, MeOH, THF and DCM.

The resin is treated with a solution of 450 µl of DIEA in 500 µl of THF and a solution of 430 mg of 3-nitrobenzenesulphonyl chloride in 500 µl of THF. It is shaken overnight at room temperature. The resin is then washed with DMF, MeOH and THF.

The resin is treated with a solution of 5400 mg of tin(II) chloride dihydrate in 12 ml of NMP and shaken overnight at room temperature. The resin is then treated with NMP, MeOH, THF and DCM.

The resin is treated with a solution of 500 µl of DIEA in 12 ml of THF/DCM 1:1 and a solution of 2757 mg of 4-nitrophenylchloroformic acid ester in 12 ml of THF/DCM 1:1. After shaking at room temperature for 45 min, it is washed with THF and DMF and a solution of 943 mg of propylamine and 2780 µl of DIEA in 20 ml of NMP is added. After shaking for 10 h, the resin is washed with DMF, MeOH, THF and DCM.

For the removal of the product, the resin is shaken for 1 h with 12 ml of TFA/DCM and filtered off, and the filtrate is concentrated in vacuo.

### II.14 3-(4-Aminobenzeneaminocarbonylamino)-3-[3-(guanidino-phenyl-sulphonyl-amino)-phenyl]-propionic acid trifluoroacetic acid

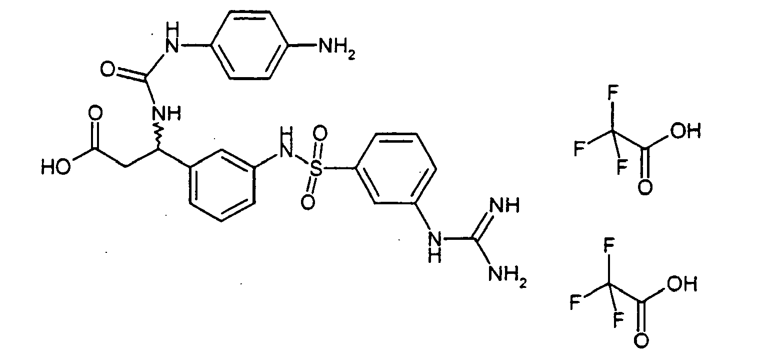

100 mg (0.165 mmol) of the compound II.1 are stirred with 1 equivalent of 4-nitrophenyl isocyanate for 3 h in 10 ml of DMF. The mixture is concentrated, and the residue is taken up in dichloromethane and precipitated using pentane. The intermediate a (106 mg; 83%) is dried.

120 mg (0.156 mmol) of the intermediate a are dissolved in methanol and hydrogenated over palladium/carbon. The catalyst is separated off, the solution is concentrated and the residue is lyophilized from dioxane/water (86 mg; 75% of intermediate b).

76 mg (0.1027 mmol) of the intermediate b are taken up in methanol and treated with 154 µl (3 eq.) of a 2M lithium hydroxide solution. After 6 h, a further 51 µl of the lithium hydroxide solution are added and the mixture is stirred for 2 days. It is concentrated, precipitated from dichloromethane using ether and intermediate c is thus obtained.

10 mg of the precipitated intermediate c are then taken up in 4 ml of dichloromethane and treated with 0.5 ml of trifluoroacetic acid. After 1.5 h, the mixture is concentrated and the residue is precipitated from dichloromethane/methanol using ether. 3 mg (30%) of the target compound are obtained. [ESI-MS: m/e = 512 (M+H)⁺].

### II.16 3-(4-Aminophenylaminocarbonylamino)-3-[3-(propylaminocarbonyl-amino-phenyl-sulphonylamino)-phenyl]-propionic acid

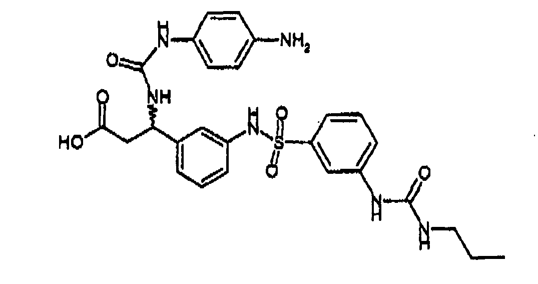

70 mg (0.166 mmol) of the compound II.13 are stirred with 54 mg (2 eq) of 4-nitrophenyl isocyanate for 1 h in 10 ml of DMF. The mixture is concentrated and the residue is purified by flash chromatography on silica gel using dichloromethane/methanol/ammonia 17% strength (15:2:0.2). After precipitation from dichloromethane/methanol using ether, the intermediate a (29 mg; 30%) is obtained.

This is dissolved in methanol and hydrogenated over palladium/carbon. The catalyst is separated off, the solution is concentrated and the residue is lyophilized from dioxane/water. 18 mg (74%) of the target compound are obtained.

### 3. Conjugates with linkage via the side chain of the non-peptide integrin ligand

### Example 3.1

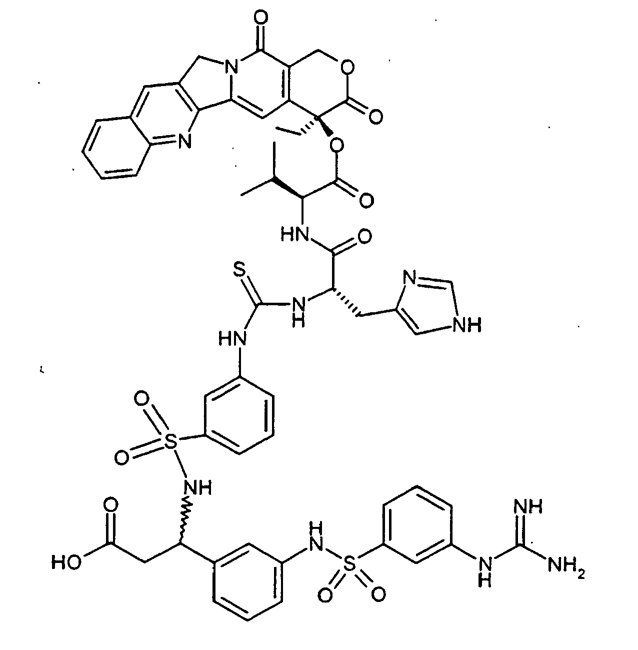

300 mg (0.5 mmol) of starting material II.1 are stirred with 80 µl of pyridine and 166 mg (1.5 eq) of m-nitrobenzenesulphonyl chloride overnight in 10 ml of dichloromethane. A further 200 ml of dichloromethane and 200 ml of water are added and the mixture is shaken thoroughly. The organic phase is collected and concentrated, and the residue is chromatographed on silica gel using dichloro-methane/methanol 99:1. After drying, 274 mg (70%) of the corresponding nitro-benzenesulphonamide are obtained.

265 mg (0.33 mmol) of the nitrobenzenesulphonamide are taken up in 30 ml of methanol and hydrogenated over palladium/carbon. After precipitating, the corresponding aminobenzenesulphonamide is obtained.

192 mg (0.25 mmol) of the corresponding aminobenzenesulphonamide are dissolved in 20 ml of methanol and the solution is stirred overnight with 504 µl (4 eq) of a 2M lithium hydroxide solution. It is concentrated, distilled off twice with dichloromethane, and, after precipitation from methanol using ether, 180 mg of the corresponding free carboxylic acid are obtained.

180 mg (0.25 mmol) of this free carboxylic acid are dissolved in 15 ml of dioxane/water (1:1) and treated with 38.5 µl of thiophosgene. After stirring at room temperature for 15 min, 172 µl (4 eq) of Hünig's base are added, and the mixture is stirred for a further 10 min and then concentrated. The residue is taken up in dichloromethane, redistilled twice and then precipitated from dichloromethane using ether. 208 mg of the corresponding isothiocyanate are obtained as a Hünig's base salt.

The Boc protective group is then removed in dichloromethane using 4 ml of trifluoroacetic acid and the unprotected guanidine is precipitated from dichloromethane/methanol using ether. 80 mg of the corresponding guanidine are obtained.

30 mg (0.037 mmol) of the corresponding guanidine are dissolved in 3 ml of DMF and then treated with 24 mg (0.8 eq) of the starting material 1.3 and with 26 µl of Hünig's base. After stirring at room temperature for 20 min, the mixture is concentrated and the residue is stirred first with dichloromethane and then with methanol. Methanol is added until dissolution is complete and the mixture is precipitated using ether.

20 mg (47%) of the target product are obtained.

[TLC: (acetonitrile/water/glacial acetic acid (5:1:0.2); R_{f} = 0.19]. [FAB-MS: m/e = 1159 (M+H)⁺].

### Example 3.2

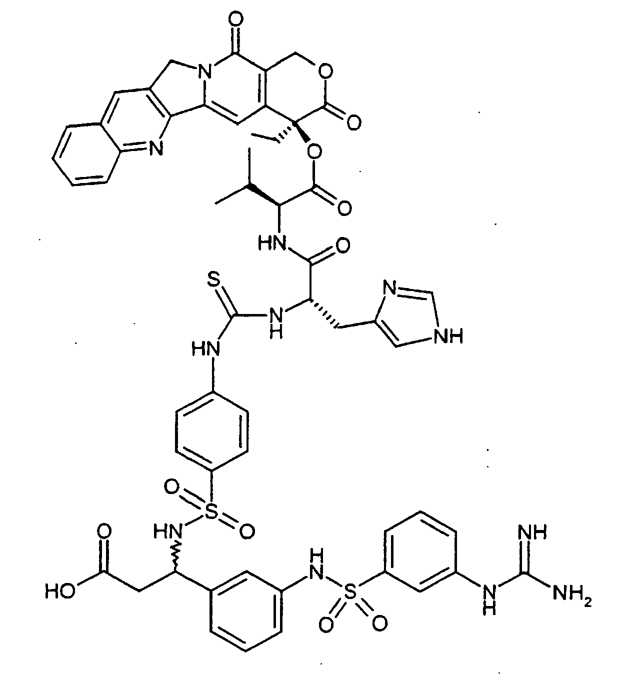

The synthesis is carried out analogously to Example 3.1 with the exception that in the first step p-nitrobenzenesulphonyl chloride is employed instead of m-nitrobenzenesulphonyl chloride.

[TLC: (acetonitrile/water/glacial acetic acid (5:1:0.2); R_{f}= 0.18]. [FAB-MS: m/e = 1159 (M+H)⁺].

### Example 3.3

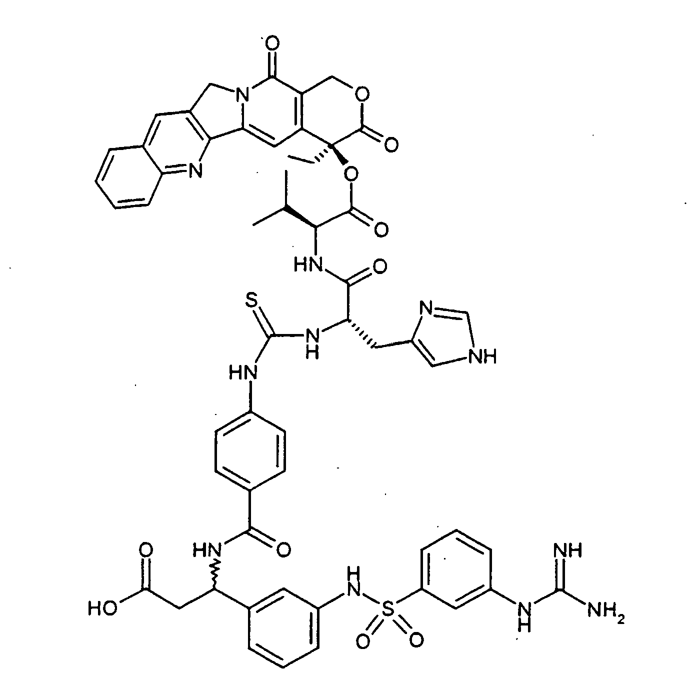

The synthesis is carried out analogously to Example 3.1 with the exception that in the first step starting material II.1 is coupled to p-aminobenzoic acid in the presence of N-(3-dimethylaminopropyl)-N'-ethylcarbodiimide hydrochloride/hydroxybenzotriazole according to standard conditions instead of to m-nitro-benzenesulphonyl chloride. [TLC final product: (dichloromethane/methanol/ammonia (17% strength) (15:6:0.6); R_{f}= 0.25]. [FAB-MS: m/e = 1123 (M+H)⁺].

### Example 3.4

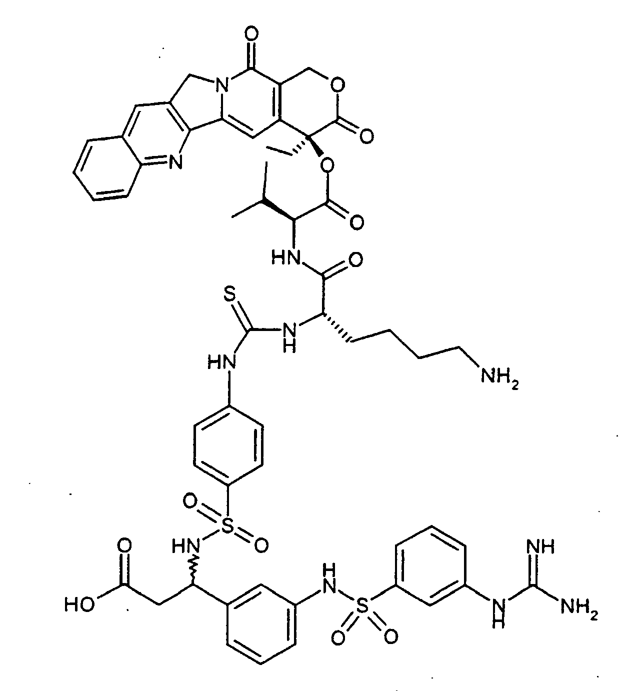

The synthesis is carried out analogously to Example 3.2 with the exception that in the last reaction step reaction takes place with starting material 1.4 instead of with starting material 1.3 and the Fmoc protective group is then detached according to standard conditions using piperidine. [TLC final product: (dichloromethane/- methanol/ammonia (17% strength) (10:10:1); R_{f}= 0.16]. [FAB-MS: m/e = 1150 (M+H)⁺].

### Example 3.5

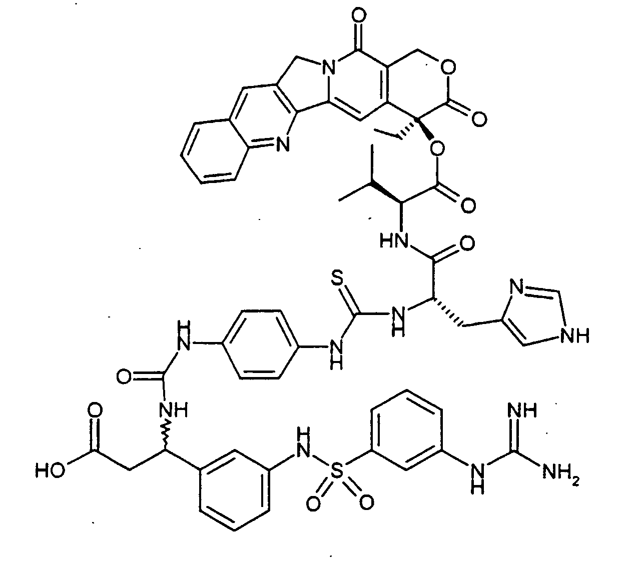

52 mg (0.073 mmol) of the Boc-protected starting material II.14 (intermediate c) are treated with 7.8 µl of thiophosgene in 5 ml of dioxane/water (1:1) and the mixture is stirred for 30 min. 37 µl of Hünig's base are then added and the mixture is concentrated.

The residue is taken up in dichloromethane and treated with 1 ml of trifluoroacetic acid. After 3 h, the mixture is concentrated and the residue is precipitated from dichloromethane/methanol using ether.

The residue obtained is dissolved in DMF as described in Example 3.1 in the last stage and then reacted with the starting material 1.3 in the presence of Hünig's base. Yield: 53%. [TLC final product: (dichloromethane/methanol/ammonia (17% strength) (15:8:0.8); R_{f}= 0.26]. [FAB-MS: m/e = 1138 (M+H)⁺].

### Example 3.12

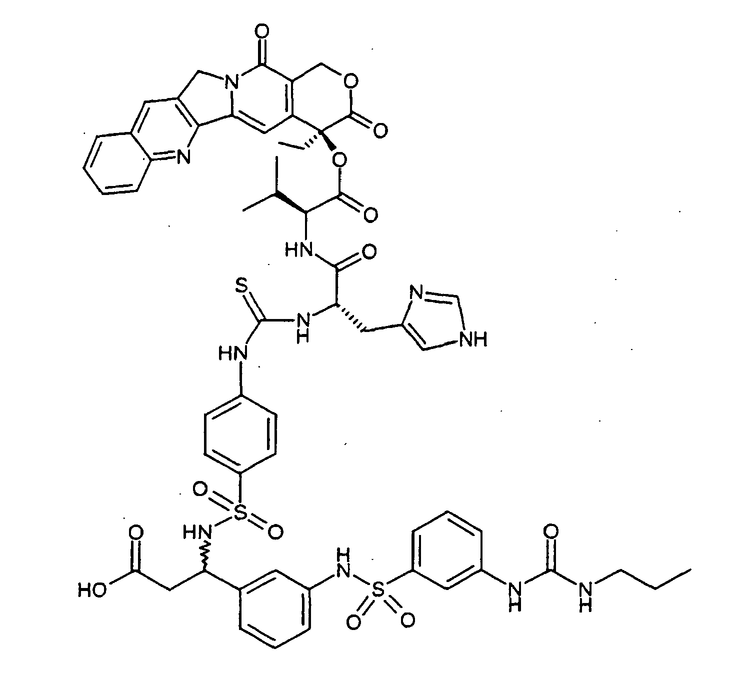

Preparation is carried out in analogy to Example 3.6 from the starting materials II.12 and I3.

[TLC final product: (dichloromethane/methanol/ammonia (17% strength) (15:3:0.3); R_{f}= 0.19]. [FAB-MS: m/e = 1202 (M+H)⁺].

### Example 3.13

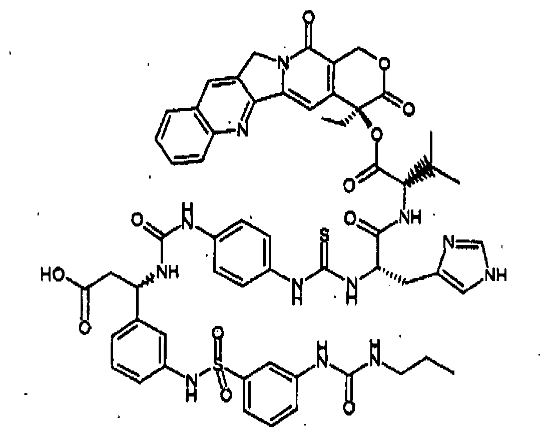

Preparation is carried out in analogy to Example 3.6 from the starting materials II.16 and I.3. [TLC final product: (dichloromethane/methanol/ammonia (17% strength) (15:4:0,5); R_{f} = 0.36]. [FAB.MS: m/e = 1181 (M+H)⁺].

### Example 3.16

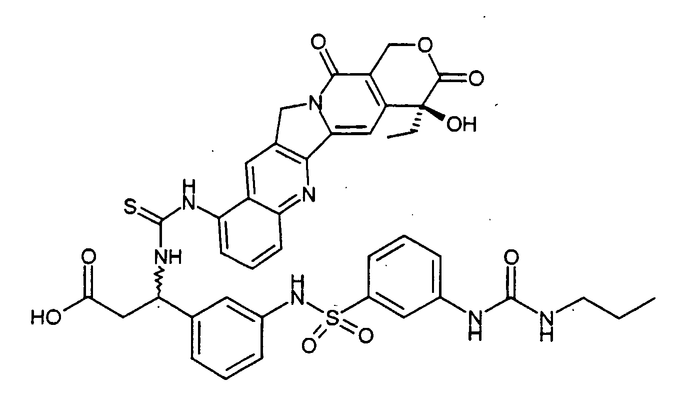

The preparation is carried out in analogy to Example 3.15 from the starting materials II.13 and 9-aminocamptothecin ([prepared according to Wani et al. (J.Med.Chem. 29 (1986), 2358)] [TLC final product: (dichloromethane/methanol/ammonia (17% strength) (15:4:0.5); R_{f}= 0.38]. [ESI-MS: m/e = 826 (M+H)⁺].

### Example 3.17

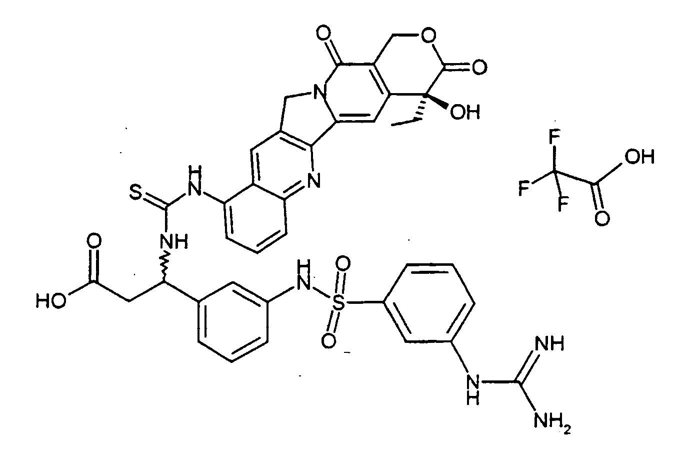

First, 200 mg of the starting material II.1 are dissolved in 10 ml of methanol and treated with 825 µl of a 2M lithium hydroxide solution and the mixture is stirred overnight. After completion of the reaction, it is concentrated and the product is precipitated from dichloromethane/methanol using ether. In addition to the ester cleavage, one of the two Boc protective groups is also detached here. The thiourea is then prepared from this intermediate and 9-aminocamptothecin ([prepared according to Wani et al. (J.Med.Chem. 29 (1986), 2358)] in analogy to Example 3.15. In the last step, the remaining Boc group is then detached using trifluoroacetic acid in dichloromethane.

[TLC final product: acetonitrile/water/glacial acetic acid (5:1:0.2); R_{f}= 0.5]. [MALDI-MS: m/e = 783 (M+H)⁺].

### Example 3.18

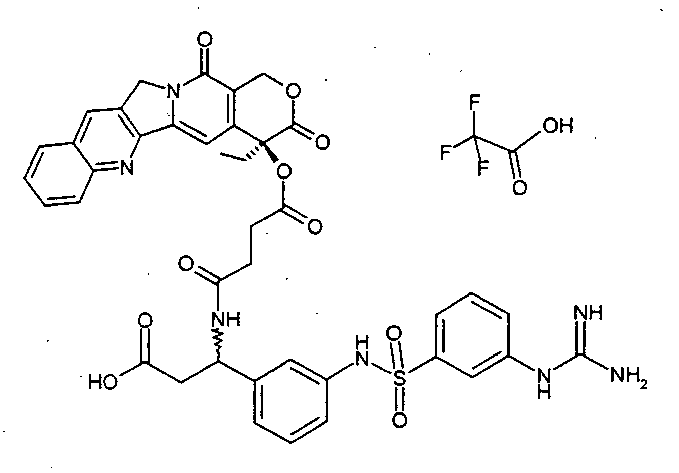

First, 200 mg of the starting material II.1 are dissolved in 10 ml of methanol and treated with 825 µl of a 2M lithium hydroxide solution and the mixture is stirred overnight. After completion of the reaction, it is concentrated and the product is precipitated from dichloromethane/methanol using ether. In addition to the ester cleavage, one of the two Boc protective groups is also detached here (intermediate a).

60 mg (0.134 mmol) of the starting material 1.11 are dissolved in 5 ml of DMF and then treated with 31 mg (1.2 eq) of N-(3-dimethylaminopropyl)-N'-ethylcarbodiimide hydrochloride and 27 mg (1.5 eq) of hydroxybenzotriazole. The mixture is stirred at room temperature for 30 min and 64 mg of the intermediate a and 69 µl of Hilnig's base are then added. After stirring at room temperature for 4 h, the mixture is concentrated and the residue is stirred with 5 ml of water. The aqueous phase is concentrated and the residue is chromatographed on silica gel (dichloromethane/methanol/ammonia (17% strength) 15:1:0.1 -> 15:2:0.2). The corresponding fractions are combined and precipitated from dichloromethane/ methanol using ether (intermediate b). Yield: 31mg (26%) [TLC of intermediate b: (dichloromethane/methanol/ammonia (17% strength) (15:4:0.5); R_{f}= 0.44].

In the last step, the remaining Boc group is then detached from 30 mg of intermediate b using 1 ml oftrifluoroacetic acid in 5 ml of dichloromethane. 30 mg (100%) of the target compound are obtained. [TLC final product: acetonitrile/water/glacial acetic acid (5:1:0.2); R_{f}= 0.45]. [FAB-MS: m/e = 808 (M+H)⁺].

### Example 3.19

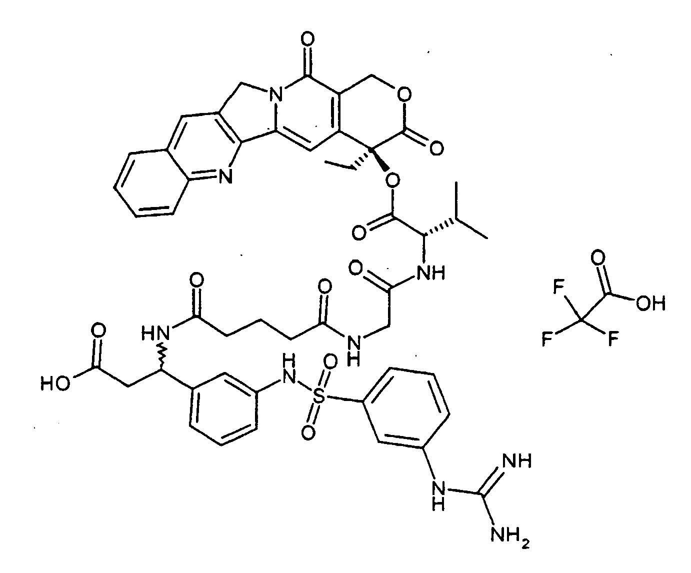

First, 200 mg of the starting material II.1 are dissolved in 10 ml of methanol and treated with 825 µl of a 2M lithium hydroxide solution and the mixture is stirred overnight. After completion of the reaction, it is concentrated and the product is precipitated from dichloromethane/methanol using ether. In addition to the ester cleavage, one of the two Boc protective groups is also detached here (intermediate a).

68 mg (0.11 mmol) of the compound starting material 1.6 are dissolved in 5 ml of DMF and then treated with 25 mg (1.2 eq) of N-(3-dimethylaminopropyl)-N'-ethylcarbodiimide hydrochloride and 22 mg (1.5 eq) of hydroxybenzoiriazole. The mixture is stirred at room temperature for 30 min and 52 mg of the intermediate a and 38 µl of Hünig's base are then added. After stirring at room temperature for 2 days, the mixture is concentrated and the residue is precipitated from dichloromethane/methanol using ether. The residue is chromatographed on silica gel (acetonitrile/water/glacial acetic acid (10:1:0.05). The appropriate fractions are combined, the solvent is removed by evaporation and the residue is precipitated from dichloromethane/methanol using ether (intermediate b).
Yield: 29 mg (24%) [TLC intermediate b: acetonitrile/water/glacial acetic acid (10:1:0.1); R_{f}= 0.25].

In the last step, the remaining Boc group is then detached from 25 mg of intermediate b fusing 1 ml of trifluoroacetic acid in 5 ml of dichloromethane. After repeated precipitation from dichloromethane/methanol using ether, 18 mg (72%) of the target compound are obtained.

[TLC: acetonitrile/water/glacial acetic acid (5:1:0.2); R_{f}= 0.38] [MALDI-MS: m/e = 978 (M+H)⁺].

### Example 3.20.

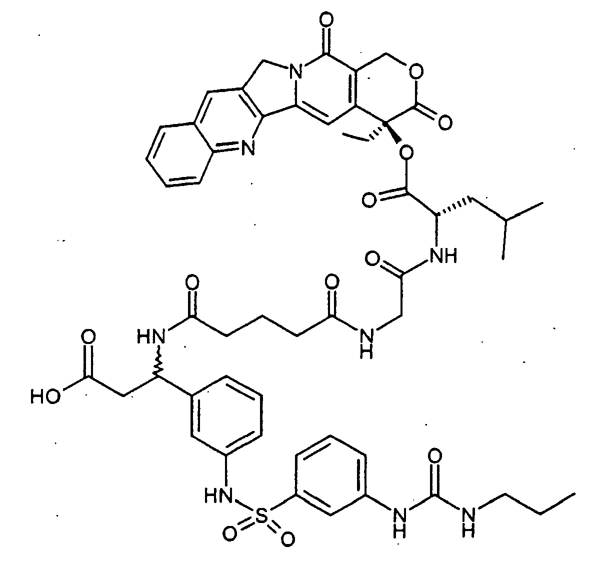

125 mg (0.2 mmol) of the compound starting material 1. 11 are dissolved in 5 ml of DMF and then treated with 42 mg (0.22 mmol) of N-(3-dimethylaminopropyl)-N'-ethylcarbodiimide hydrochloride and 34 mg (0.3 mmol) of N-hydroxysuccinimide. The mixture is stirred at room temperature for 2 h and then concentrated and the residue is precipitated twice from dichloromethane using ether.

10 mg (0.014 mmol) of the activated intermediate are treated with 5.8 mg of the starting material 11.13 and with 4.8 µl (0.028 mmol) of Hünig's base in 4 ml of DMF and the mixture is stirred at room temperature for 4 h. It is then concentrated and the residue is chromatographed on silica gel (dichloromethane/methanol/ammonia (17% strength) 15:2:0.2 -> 15:3:0.3). The appropriate fractions are combined and precipitated from dichloromethane/methanol using ether. Yield: 6 mg (42%) [TLC: dichloromethane/methanol/ammonia (17% strength) (15:3:0.3) R_{f} = 0.14] [ESI-MS: m/e = 1035 (M+H)⁺].

### Example 3.21

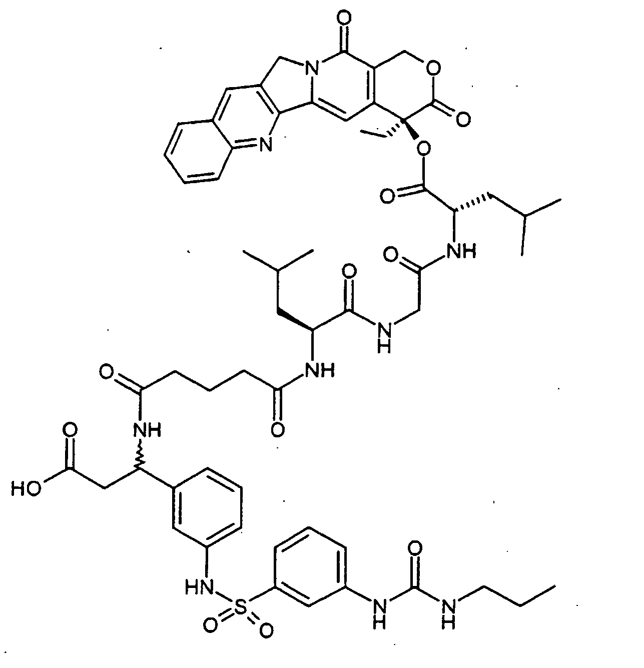

The synthesis is carried out analogously to Example 3.20 starting from the carboxy starting material 1.8 and the betaine starting material II.13.

[TLC: dichloromethane/methanol/ammonia (17% strength) (15:3:0.3) R_{f} = 0.18] [ESI-MS: m/e = 1148 (M+H)⁺].

### Example 3.22

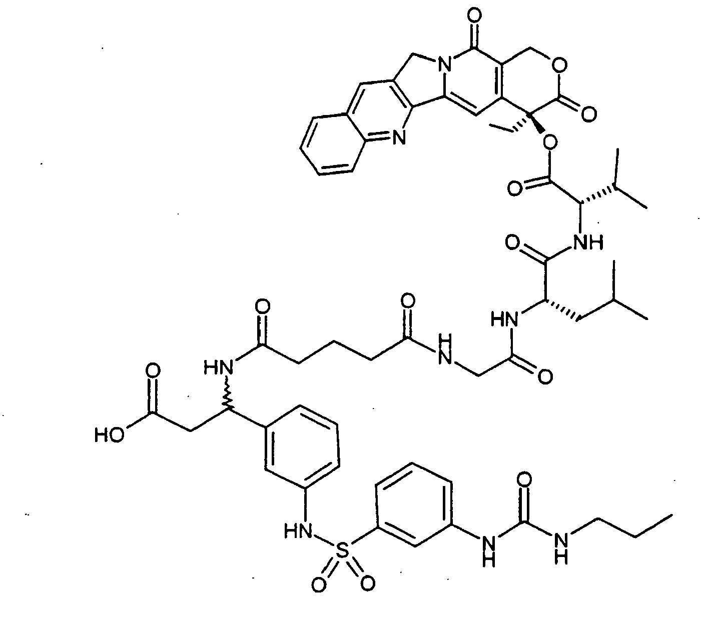

The synthesis is carried out analogously to Example 3.20 starting from the carboxy starting material 1.9 and the betaine starting material II.13.

[TLC: dichloromethane/methanol/ammonia (17% strength) (15:4:0.5) R_{f}= 0.42] [ESI-MS: m/e = 1134 (M+H)⁺].

### Example 3.23

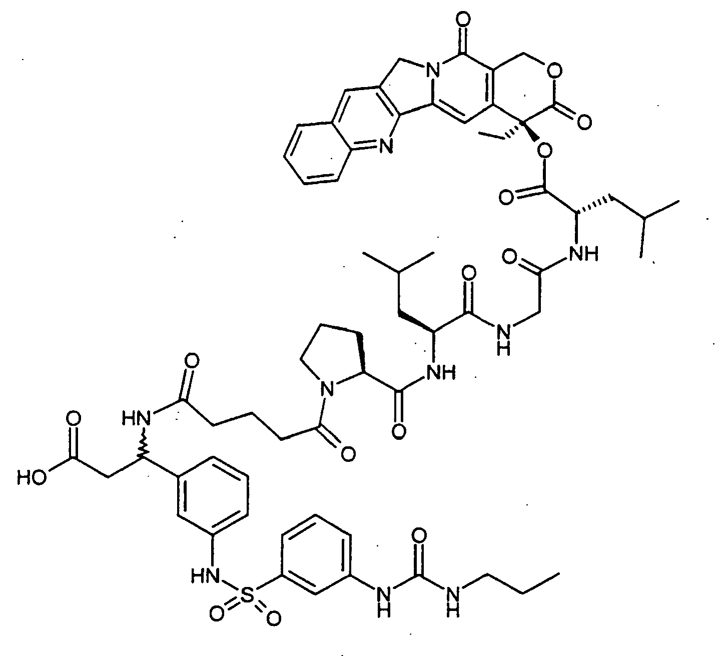

The synthesis is carried out analogously to Example 3.20 starting from the carboxy starting material I.10 and the betaine starting material II.13.

[TLC: dichloromethane/methanol/ammonia (17% strength) (15:3:0.3); R_{f}= 0.24] [ESI-MS: m/e = 1245 (M+H)⁺].

### Biological Tests

### A: α_{ν}β₃ Binding test

α_{ν}β₃ from human A375 cells was purified analogously to a procedure which was described by Wong et al. (Molecular Pharmacology, 50, 529-537 (1996)). In each case, 10 µl of α_{ν}β₃ (5 ng) in TBS pH 7.6, 2 mM CaCl₂, 1 mM MgCl₂, 1% n-octyl-glucopyranoside (Sigma); 10 µl of test substance in TBS pH 7.6, 0.1% DMSO and 45 µl of TBS pH 7.6, 2 mM CaCl₂, 1 mM MgCl₂, 1mM MnCl₂ were incubated at. room temperature for 1 h. In each case, 25 µl of WGA SPA beads (Amersham, 4 mg/ml) and 10 µl of echistatin (0.1 µCi, Amersham, chloramine-T labelled) were then added. After 16 hours at room temperature, the samples were measured in a scintillation measuring apparatus (Wallac 1450). The test results are shown in Table 1 below.

**Table 1: IC₅₀ values of the binding to the α_{ν}β₃ receptor [nM]**

| **Example** | **IC₅₀ [nM]** |
|---|---|
| 3.1 | 147 |
| 3.2 | 45 |
| 3.3 | 45 |
| 3.4 | 65 |
| 3.5 | 36 |
| 3.12 | 390 |
| 3.13 | 14 |
| 3.16 | 51 |
| 3.17 | 66 |
| 3.18 | 79 |
| 3.19 | 48 |
| 3.20 | 265 |
| 3.21 | 549 |
| 3.22 | 531 |
| 3.23 | 355 |

### B: Growth inhibition test for the determination of the cytotoxic properties on various tumour cell lines:

The human large intestine cell lines SW 480 and HT29 (ATCC No. CCL 228 and HTB38), the human breast cell lines MDA-MB 231, MCF-7 and BT20 (ATCC No. HTB-, 26, 22 and 23) and the mouse melanoma cell line B16F10 (CRL 6475) were grown to confluence in Roux dishes in RPMI 1640 medium with addition of 10% FCS. They were then trypsinized and taken up in RPMI plus 10% FCS to a cell count of 50,000 cells or, for B16F10, 20,000 cells per ml. 100 µl of cell suspension/well were added to a 96 microwell plate and incubated at 37°C for 1 day in a CO₂ incubator. A further 100 µl of RPMI medium and 1 µl of DMSO were then added with the test substances. The growth was cheeked after day 6. For this, 25 µl of MTT solution (3-(4,5-dimethylthiazol-2-yl)-2,5-diphenyltrazolium bromide) was added to each well at a starting concentration of 5 mg/ml of H₂0. The plate was incubated at 37°C for 5 hours in a CO₂ incubator. The medium was then aspirated and 100 µl of ipropanol/well were added. After shaking with 100 µl of H₂O for 30 min, the extinction was measured at 595 nm using a Multiplate Reader (BIO-RAD 3550.UV).

The cytostatic action is indicated in Table 2 as an IC₅₀ value, in each case for the individual cell lines.

**Table 2: IC₅₀ values of the cytotoxic action on tumour cell lines [nM]**

| **Example** | **SW480** | **HT 29** | **B16F10** | **BT20** | **MCF7** | **MDA-MB 231** |
|---|---|---|---|---|---|---|
| 3.1 | 100 | 400 | 1000 | 1000 | 800 | 500 |
| 3.2 | 400 | 200 | 1000 | 200 | 200 | 200 |
| 3.3 | 300 | 90 | --- | 150 | 100 | 100 |
| 3.4 | 200 | 200 | 500 | 700 | 700 | 300 |
| 3.5 | 200 | 150 | 900 | 500 | 150 | 150 |
| 3.12 | 300 | 150 | 250 | 150. | 100 | 150 |
| 3.13 | 200 | 90 | 200 | 150 | 100 | 200 |
| 3.16 | 500 | 100 | 1000 | 700 | 300 | 1000 |
| 3.17 | 800 | 600 | 5000 | 5000 | --- | 3000 |
| 3.18 | 300 | 180 | 1000 | 900 | 600 | 500 |
| 3.19 | 1500 | 500 | b000 | 4000 | 5000 | 2000 |

### C. In-vivo inhibition or tumour growth using a nude mouse model

### Material:

In all in-vivo experiments for investigating the inhibition of tumour growth, athymic nude mice (NMRI nu/nu strain) were used. The tumour was developed by serial passage in nude mice. The human origin of the tumour was confirmed by isoenzymatic and immunohistochemical methods.

### Experimental set-up:

The tumour was implanted subcutaneously in both flanks of nu/nu nude mice 6 to 8 weeks old The treatment was started, depending on the doubling time, as soon as the tumours had reached a diameter of 5- 7 mm. The mice were assigned to the treatment group or the control group (5 mice per group having 8-10 assessable tumours) by randomization. The individual tumours of the control group all grew progressively.

The size of the tumours was measured in two dimensions by means of a slide gauge, The tumour volume, which correlated well with the cell count, was then used for all assessments. The volume was calculated according to the formula "length x breadth x breadth / 2" ([a x b²] / 2, a and b represent two diameters arranged at right angles).

The values of the relative tumour volume (RTV) were calculated for each individual tumour by dividing the tumour size on day X with the tumour size on day 0 (at the time of randomization). The average values of the RTV were then used for the further assessment.

The inhibition of the increase of the tumour volume (tumour volume of the test group/control group, T/C, in per cent) was the final measured value.

### Treatment:

The compounds can be administered with a daily or an intermittent therapy schedule through a couple of days either by intraperitoneal, intravenious, oral or subcutaneous route.

The compound of example 3.8 inhibited tumor growth of the subcutaneously growing human breast cancer xenograft MX1 with an optimal T/C of 15.9 at a dose of 40 mg/kg given on day 1-3 and 14-16.

### D. CSF-induced proliferation of hemopoietic stem cells

Bone marrow cells are flushed out of the femur of mice. 10⁵ cells are incubated in McCoy 5A medium (0.3% agar) together with recombinant murine GM-CSF (Genzyme; parent cell colony formation) and the substances (10⁻⁴ to 100 µg/ml) at 37°C and 7% CO₂. 7 days later, the colonies (<50 cells) and clusters (17-50 cells) are counted.

A series of compounds exhibits a drastically reduced toxicity against stem cells in vitro compared to camptothecin.

| **Example** | **IC₅₀[ng/ml]** |
|---|---|
| 3.19 | 200 |
| 3.20 | 100 |
| 3.21 | 80 |
| 3.22 | 6 |
| 3.23 | 6 |
| 3.24 | 40 |
| 3.25 | 10 |
| 3.5 | 20 |
| 3.19 | 20 |
| 3.13 | 40 |
| 3.19 | 30 |
| Camptothecin | 0.3 |

## Claims

1. Conjugate **characterized by** the formula (I)
CT-AA1-AA2-AA3-AA4-Sp-IA (I)
in which
CT denotes a cytotoxic radical or a radical of a cytostatic or of a cytostatic derivative, which can additionally carry a hydroxyl, carboxyl or amino group,
AA1 is absent or is an amino acid in the D or L configuration, which can optionally carry protective groups or a radical Sp',
AA2 is absent or is an amino acid in the D or L configuration, which can optionally carry protective groups or a radical Sp',
AA3 is absent or is an amino acid in the D or L configuration, which can optionally carry protective groups or a radical Sp',
AA4 is absent or is an amino acid in the D or L configuration, which can optionally carry protective groups or a radical Sp',
in which
Sp' is an arylaminocarbonyl or an arylaminothiocarbonyl radical having 7-11 carbon atoms,
Sp is absent, is an arylaminocarbonyl or an arylaminothiocarbonyl radical having 7-11 carbon atoms or is an alkanedicarboxylic acid radical having 3 to 8 carbon atoms or a carbonyl or a thiocarbonyl radical,
with the proviso that at least one of the radicals AA1 to AA4 and/or Sp is present,
IA is a non-peptide radical addressing an α_{ν}β₃ integrin receptor, which is
a radical of the formula (III) 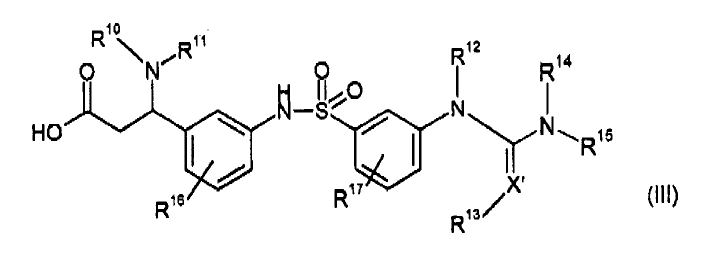 in which
R¹⁰ is -SO₂R^{10'}, COOR^{10'}, -COR¹⁰', -CONR^{10'}₂ or -CS-NR^{10'}2, or represents a direct bond via which the radical of the formula (III) is optionally bonded to the rest of the conjugate;
R^{10'} independently of one another is hydrogen, a substituted or unsubstituted alkyl or cycloalkyl radical, a substituted or unsubstituted aryl radical or a saturated or unsaturated, optionally substituted heterocyclic radical, via which the radical of the formula (III) is optionally bonded to the rest of the conjugate;
R^{10"} is a substituted or unsubstituted alkyl or cycloalkyl radical, a substituted or unsubstituted aryl radical or a saturated or unsaturated, optionally substituted heterocyclic radical, via which the radical of the formula (III) is optionally bonded to the rest of the conjugate;
R¹¹ is hydrogen, a substituted or unsubstituted alkyl or cycloalkyl radical or a substituted or unsubstituted aryl radical,
R¹⁶ is hydrogen, CN, a substituted or unsubstituted alkyl or cycloalkyl radical, a substituted or unsubstituted alkoxy radical or a halogen atom;
R¹⁷ is hydrogen, CN, a substituted or unsubstituted alkyl or cycloalkyl radical, a substituted or unsubstituted alkoxy radical or a halogen atom;
R¹² is hydrogen, a substituted or unsubstituted alkyl or cycloalkyl radical, a substituted or unsubstituted aryl radical, a saturated or unsaturated, optionally substituted heterocyclic radical;
X' is N, O or S;
R¹³ is absent, is -H, a substituted or unsubstituted alkyl or cycloalkyl radical, -NO₂, -CN, -COR^{13'}, -COOR^{13'};
R^{13'} is hydrogen, a substituted or unsubstituted alkyl or cycloalkyl radical, a substituted or unsubstituted aryl radical or a saturated or unsaturated, optionally substituted heterocyclic radical which can be saturated or unsaturated and/or can contain further heteroatoms;
R¹⁴ is hydrogen, a substituted or unsubstituted alkyl or cycloalkyl radical, a substituted or unsubstituted aryl radicals, a saturated or unsaturated, optionally substituted heterocyclic radical;
R¹⁵ is hydrogen, a substituted or unsubstituted alkyl or cycloalkyl radical, a substituted or unsubstituted aryl radical, a saturated or unsaturated, optionally substituted heterocyclic radical,
and their physiologically acceptable salts and stereoisomers.

2. Conjugate according to Claim 1, **characterized in that**
CT is camptothecin or 9-aminocamptothecin, which can be linked to the rest of the conjugate via the C20-OH group or, in the case of 9-aminocamptothecin, via the free amino group;
AA1 is absent or is a naturally occurring amino acid in the D or L configuration, which is selected from the group consisting of glycine, alanine, valine, leucine, isoleucine and phenylalanine;
AA2 is absent or is a naturally occurring amino acid in the D or L configuration, which is selected from the group consisting of lysine, glutamate, histidine, glycine, arginine, ornithine and leucine, and can optionally carry protective groups or a radical Sp',
AA3 is absent or is a naturally occurring amino acid in the D or L configuration, which is selected from the group consisting of glycine, alanine, valine, leucine, isoleucine and phenylalanine;
AA4 is absent or is a naturally occurring amino acid in the D or L configuration, which can optionally carry protective groups or a radical Sp',
in which
Sp' is a phenylaminocarbonyl or a phenylaminothiocarbonyl radical,
Sp is absent, is a phenylaminocarbonyl or a phenylaminothiocarbonyl radical or is an alkanedicarboxylic acid radical having 3 to 6 carbon atoms or a carbonyl or a thiocarbonyl radical,
with the proviso that at least one of the radicals AA1 to AA4 and/or Sp is present,
IA is a non-peptide radical of the formula (III) addressing an α_{ν}β₃ integrin receptor,
in which
R¹⁰ is SO₂R^{10'}, -COOR^{10"}, -COR^{10'}, -CONR^{10'}₂ or -CSNR^{10'}₂ or represents a direct bond, via which the radical of the formula (III) is optionally bonded to the rest of the conjugate;
R^{10'} is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -C₆H₂(CH₃)₃, -C₆(CH₃)₅, -CH₂C₆H₂(CH₃)₃, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 4-chlorophenylmethyl, 2,4-dichloro-phenyl-methyl, 2,6-dichlorophenylmethyl, 3-aminophenyl, 4-aminophenyl, 2-methoxycarbonylphenylmethyl, 3-trifluoromethylphenyl, 4-trifluoromethylphenyl, 3,5-bis(trifluoro-methyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluoro-phenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-tritluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methylbenzothiazol-2-yl, N-meth-oxycarbonyl-piperidin-3-yl, thiophen-2-yl, isoxazol-5-yl, ethoxy, 2-chloropyridin-3-yl, pyridin-3-yl, benzyloxy, 5-methylisoxazol-3-yl, 1-adamantyl, 4-chlorophenoxymethyl, 2,2-dimethylethenyl, 2-chloropyridine-5-methyl, 5,7-dimethyl-1,3,4-triazaindolizin-2-yl, (S)-camphan-1-yl, (R)-camphan-1-yl or 8-quinolinyl;
R^{10"} is a C₁₋₆-alkyl radical, a C₃₋₇-cycloalkyl radical, a substituted or unsubstituted aryl radical or a saturated or unsaturated, optionally substituted heterocyclic radical, via which the radical of the formula (III) is optionally bonded to the rest of the conjugate;
R¹¹ is hydrogen, methyl, ethyl, propyl, isopropyl, butyl, isobutyl, t-butyl, pentyl, isopentyl, neopentyl, hexyl, cyclopropyl, cyclo-propylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄₋alkyl, C₁₋₄-dialkyl-amino-C₁₋₄-alkyl, amino-C₁₋₄-alkyl, C₁₋₄-alkyloxy-C₁₋₄-alkyl, dialkylamino-C₁₋₄-alkyl, amino-C₁₋₄-alkyl, C₁₋₄-alkyloxy-C₁₋₄-alkyl or 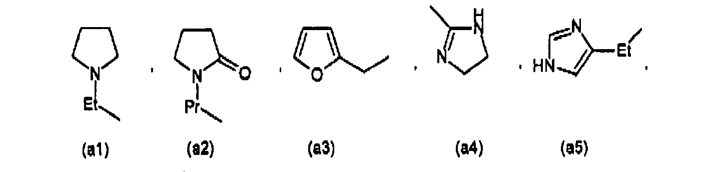 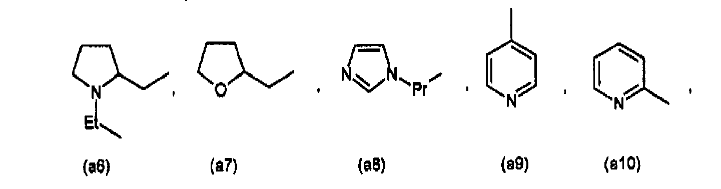 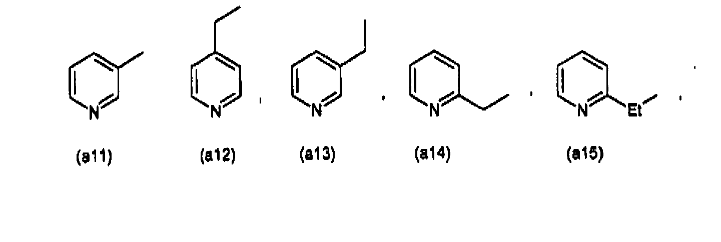 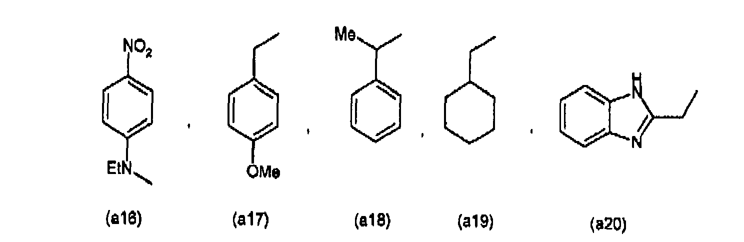 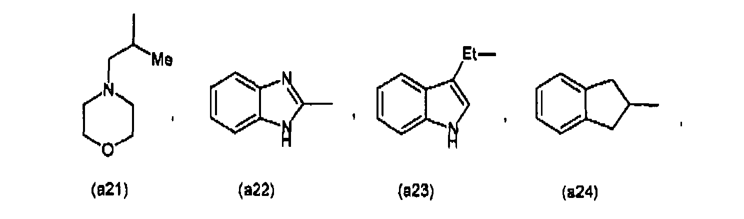 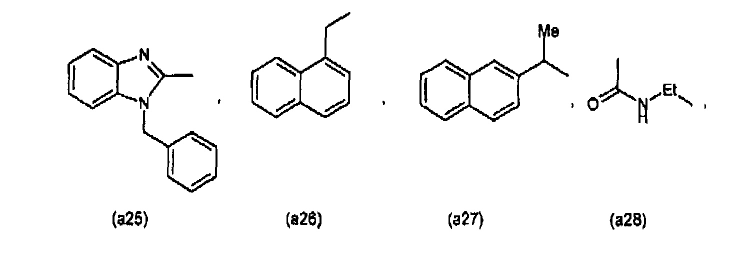 R¹⁶ is hydrogen, CN, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, methoxy, trifluoromethoxy, ethoxy, propoxy, butoxy, pentoxy or hexoxy, fluorine, chlorine, bromine or iodine;
R¹⁷ is hydrogen, CN, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, methoxy, trifluoromethoxy, ethoxy, propoxy, butoxy, pentoxy or hexoxy, fluorine, chlorine, bromine or iodine;
R¹² is hydrogen, methyl, ethyl, propyl, isopropyl, butyl, isobutyl, t-butyl, pentyl, isopentyl, neopentyl, hexyl, cyclopropyl, cyclo-propylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkyl-amino-C₁₋₄-alkyl, amino-C₁₋₄-alkyl, C₁₋₄-alkyloxy-C₁₋₄-alkyl, one of the radicals (a1) to (a28);
X' is N, O or S;
R¹³ is absent, is -II, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, -NO₂, -CN, -COR^{7'}, -COOR^{7'};
R^{13'} is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof;
R¹⁴ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethyleyclohexyl, 5-methyl-2-hexyl, phenyl, benzyl, tolyl or a substituted derivative thereof, C₁₋₄-alkylamino-C₁₋₄-alkyl, C₁₋₄-dialkyl-amino-C₁₋₄-alkyl, amino-C₁₋₄-alkyl, C₁₋₄-alkyloxy-C₁₋₄-alkyl, one of the radicals (a1) to (a28); and
R¹⁵ is hydrogen, methyl, ethyl, propyl, isopropyl, butyl, isobutyl, t-butyl, pentyl, isopentyl, neopentyl, hexyl, cyclopropyl, cyclo-propylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, C₁₋₄-alkylamino-C₁₋₄-alkyl, C₁₋₄-dialkyl-amino-C₁₋₄-alkyl, amino-C₁₋₄-alkyl, C₁₋₄-alkyloxy-C₁₋₄-alkyl, one of the radicals (a1) to (a28).
and their physiologically acceptable salts and stereoisomers.

3. Process for the preparation of conjugates according to Claim 1, comprising
[A] the reaction of a compound of the formulae (III), which has a free or optionally activated carboxyl function,
with a compound of the formula (Ia) which has a free primary or secondary amino group
CT-AA1-AA2-AA3-AA4-Sp (Ia)
in which all radicals have the meaning indicated in Claim 1,
in the presence of a base;
or
[B] the reaction of a compound of the formulae (III) which has a free
primary or secondary amino function, with a carbonic acid derivative such as, for example, phosgene, thiophosgene or a chloroformic acid ester, if appropriate in the presence of a base,
followed by the reaction with a compound of the formula (Ia) which has a free primary or secondary amino group
CT-AA1-AA2-AA3-AA4-Sp (Ia)
in which all radicals have the meaning indicated in Claim 1, and
if appropriate the removal of protective groups and/or derivatization of nitrogen atoms present at preferred points of time in the preparation process and/or conversion of the compound obtained into the free acid and/or conversion of the compound obtained into one of its physiological salts by reaction with an inorganic or organic base or acid;
or
[C] the reaction of a cytotoxic compound or of a cytostatic or of a cytostatic derivative CT which contains a free primary or secondary amino group,
with a carbonic acid derivative such as, for example, phosgene, thiophosgene or a chloroformic acid ester in the presence of a base,
followed by the reaction with a compound of the formulae (III) which has a free primary or secondary amino function,
and
if appropriate the removal of protective groups and/or derivatization of nitrogen atoms present at preferred points of time in the preparation process and/or conversion of the compound obtained into the free acid and/or conversion of the compound obtained into one of its physiological salts by reaction with an inorganic or organic base or acid;
or
[D] the reaction of a compound of the formulae (III) which contains a free primary or secondary amino function,
with a compound of the formula (Ia) which contains a free or optionally activated carboxyl function
CT-AA1-AA2-AA3-AA4-Sp (Ia)
in which all radicals have the meaning indicated in Claim 1,
in the presence of a base;
and
if appropriate the removal of protective groups and/or derivatization of nitrogen atoms present at preferred points in time in the preparation process and/or conversion of the compound obtained into the free acid and/or conversion of the compound obtained into one of its physiological salts by reaction with an inorganic or organic base or acid.

4. Process according to Claim 3, **characterized in that** all steps of the process are carried out on a solid phase.

5. Medicament, comprising at least one of the conjugates according to one of Claims 1 to 2.

6. Use of compounds according to one of Claims 1 to 2 for the production of medicaments for the treatment of carcinomatous disorders.

## Patentansprüche

1. Konjugat gemäß Formel (I)
CT-AA1-AA2-AA3-AA4-Sp-IA (I)
worin
CT für einen zytotoxischen Rest oder einen Rest eines Zytostatikums oder eines Derivates eines Zytostatikums steht, der zusätzlich eine Hydroxy-, Carboxyl- oder Aminogruppe tragen kann,
AA1 fehlt oder für eine Aminosäure in der D oder L Konfiguration steht, die gegebenenfalls Schutzgruppen oder einen Rest Sp' tragen kann,
AA2 fehlt oder für eine Aminosäure in der D oder L Konfiguration steht, die gegebenenfalls Schutzgruppen oder einen Rest Sp' tragen kann,
AA3 fehlt oder für eine Aminosäure in der D oder L Konfiguration steht, die gegebenenfalls Schutzgruppen oder einen Rest Sp' tragen kann,
AA4 fehlt oder für eine Aminosäure in der D oder L Konfiguration steht, die gegebenenfalls Schutzgruppen oder einen Rest Sp' tragen kann,
worin
Sp' für einen Arylaminocarbonylrest oder einen Arylaminothiocarbonylrest bestehend aus 7-11 Kohlenstoffatomen steht ,
Sp fehlt, für einen Arylaminocarbonylrest oder einen Arylaminothiocarbonylrest bestehend aus 7-11 Kohlenstoffatomen oder einen Alkandicarbonsäurerest bestehend aus 3 bis 8 Kohlenstoffatomen oder einen Carbonylrest oder einen Thiocarbonylrest steht,
mit der Vorgabe, dass mindestens ein Rest AA1 bis AA4 und/oder Sp vorhanden ist,
IA für ein nicht-peptidischen Rest der Formel (III) steht, der einen α_{ν}β₃-Integrinrezeptor adressiert, 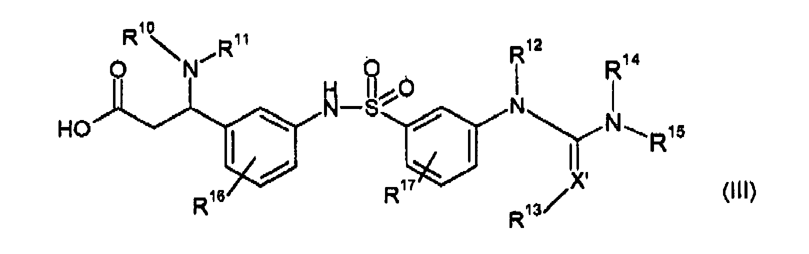 worin
R¹⁰ für -SO₂R^{10'}, -COOR^{10"}, -COR^{10'}, -CONR^{10'}₂ oder -CS-NR^{10'}₂, oder für eine direkte Bindung steht, über die der Rest der Formel (III) gegebenenfalls an den Rest des Konjugates gebunden ist;
R^{10'} unabhängig voneinander für Wasserstoff, einen substituierten oder unsubstituierten Alkyl- oder Cycloalkylrest, einen substituierten oder unsubstituierten Arylrest oder einen gesättigten oder ungesättigten, gegebenenfalls substituierten heterocyclischen Rest steht, über den der Rest der Formel (III) gegebenenfalls an den Rest des Konjugates gebunden ist;
R^{10"} für einen substituierten oder unsubstituierten Alkyl- oder Cycloalkylrest, einen substituierten oder unsubstituierten Arylrest oder einen gesättigten oder ungesättigten, gegebenenfalls substituierten heterocyclischen Rest steht, über den der Rest der Formel (III) gegebenenfalls an den Rest des Konjugates gebunden ist;
R¹¹ für Wasserstoff, einen substituierten oder unsubstituierten Alkyl- oder Cycloalkylrest oder einen substituierten oder unsubstituierten Arylrest steht,
R¹⁶ für Wasserstoff, CN, einen substituierten oder unsubstituierten Alkyl- oder Cycloalkylrest oder einen substituierten oder unsubstituierten Alkoxyrest oder einem Halogenatom steht;
R¹⁷ für Wasserstoff, CN, einen substituierten oder unsubstituierten Alkyl- oder Cycloalkylrest oder einen substituierten oder unsubstituierten Alkoxyrest oder einem Halogenatom steht;
R¹² für Wasserstoff, einen substituierten oder unsubstituierten Alkyl- oder Cycloalkylrest, einen substituierten oder unsubstituierten Arylrest oder einen gesättigten oder ungesättigten, gegebenenfalls substituierten heterocyclischen Rest steht;
X' für N, O oderr S steht;
R¹³ fehlt, für Wasserstoff, einen substituierten oder unsubstituierten Alkyl- oder Cycloalkylrest, -NO₂, -CN, - COR^{13'}, oder -COOR^{13'} steht;
R^{13'} für Wasserstoff, einen substituierten oder unsubstituierten Alkyl- oder Cycloalkylrest, einen substituierten oder unsubstituierten Arylrest oder einen gesättigten oder ungesättigten, gegebenenfalls substituierten heterocyclischen Rest steht, der weitere Heteroatome enthalten kann;
R¹⁴ für Wasserstoff, einen substituierten oder unsubstituierten Alkyl- oder Cycloalkylrest, einen substituierten oder unsubstituierten Arylrest oder einen gesättigten oder ungesättigten, gegebenenfalls substituierten heterocyclischen Rest steht;
R¹⁵ für Wasserstoff, einen substituierten oder unsubstituierten Alkyl- oder Cycloalkylrest, einen substituierten oder unsubstituierten Arylrest oder einen gesättigten oder ungesättigten, gegebenenfalls substituierten heterocyclischen Rest steht,
und dessen physiologisch akzeptablen Salze und Stereoisomeren.

2. Konjugat gemäß Anspruch 1, **dadurch gekennzeichnet, dass**
CT für Camptothecin oder 9-Aminocamptothecin steht, das über die C20-OH-Gruppe oder im Fall des 9-Aminocamptothecins über die freie Aminogruppe an den Rest des Konjugates gebunden ist;
AA1 fehlt oder für eine natürlich vorkommende Aminosäure in der D oder L Konfiguration ausgewählt aus der Gruppe bestehend aus Glycin, Alanin, Valin, Leucin, Isoleucin und Phenylalanin steht;
AA2 fehlt oder für eine natürlich vorkommende Aminosäure in der D oder L Konfiguration ausgewählt aus der Gruppe bestehend aus Lysin, Glutamat, Histidin, Glycin, Arginin, Ornithin und Leucine steht, und die gegebenenfalls Schutzgruppen oder einen Rest Sp' tragen kann,
AA3 fehlt oder für eine natürlich vorkommende Aminosäure in der D oder L Konfiguration ausgewählt aus der Gruppe bestehend aus Glycin, Alanin, Valin, Leucin, Isoleucin und Phenylalanin steht;
AA4 fehlt oder für eine natürlich vorkommende Aminosäure in der D oder L Konfiguration steht, die gegebenenfalls Schutzgruppen oder einen Rest Sp' tragen kann,
worin
Sp' für einen Phenylaminocyrbonylrest oder einen Phenylaminothiocarbonylrest steht,
Sp fehlt, für einen Phenylaminocyrbonylrest oder einen Phenylaminothiocarbonylrest oder einen Alkandicarbonsäurerest bestehend aus 3 bis 6 Kohlenstoffatomen oder einen Carbonylrest oder einen Thiocarbonylrest steht,
mit der Vorgabe, dass mindestens ein Rest AA1 bis AA4 und/oder Sp vorhanden ist,
IA für ein nicht-peptidischen Rest der Formel (III) steht, der einen α_{ν}β₃-Integrinrezeptor adressiert,
worin
R¹⁰ für SO₂R^{10'}, -COOR^{10"}, -COR^{10'}, -CONR^{10'}₂ oder -CSNR^{10'}₂ oder für eine direkte Bindung steht, über die der Rest der Formel (III) gegebenenfalls an den Rest des Konjugates gebunden ist;
R^{10'} für Wasserstoff, Methyl, Ethyl, Propyl, Isopropyl, Butyl, Isobutyl, t-Butyl, Pentyl, Isopentyl, Neopentyl, Hexyl, Cyclopropyl, Cyclobutyl, Cyclopentyl, Cyclohexyl, Cycloheptyl, Phenyl, Benzyl, Tolyl oder ein substituiertes Derivat davon, -C₆H₂(CH₃)₃, -C₆(CH₃)₅, -CH₂C₆H₂(CH₃)₃, 2-Chlorphenyl, 3-Chlorphenyl, 4-Chlorphenyl, 2,3-Dichlorphenyl, 2,4-Dichlorphenyl, 3,4-Dichlorphenyl, 2,5-Dichlorphenyl, 3,5-Dichlorphenyl, 2,6-Dichlorphenyl, 4-Chlorphenylmethyl, 2,4-Dichlorphenylmethyl, 2,6-Dichlorphenylmethyl, 3-Aminophenyl, 4-Aminophenyl, 2-Methoxycarbonylphenylmethyl, 3-Trifluormethylphenyl, 4-Trifluormethylphenyl, 3,5-Bis(trifluoromethyl)phenyl, 4-Trifluormethoxyphenyl, Phenylmethyl, 2-Acetamido-4-methylthiazol-5-yl, Phenylethyl, 1-Phenylpropyl, (S)-(+)-Camphor-10-yl, (R)-(-)-Camphor-10-yl, 2-Phenylethenyl, 2-Thiophenyl, 4-Methoxyphenyl, 3,5-Dimethoxyphenyl, 3-Methylphenyl, 4-Methylphenyl, 4-t-Butylphenyl, 4-Propyl-phenyl, 2,5-Dimethylphenyl, 2-Methoxy-5-methylphenyl, 2,3,5,6-Tetramethylphenyl, 1-Naphthyl, 2-Naphthyl, 4-Fluorphenyl, 2,4-Difluorphenyl, 2-Chlor-6-methylphenyl, 2-Chlor-4-fluorphenyl, 2,5-Dimethoxyphenyl, 3,4-Dimethoxyphenyl, 3-Chlor-6-methoxyphenyl, 2-Trifluormethylphenyl, 2-Alkylsulphonylphenyl, 2-Arylsulphonylphenyl, 3-(N-Acetyl-6-methoxy)aniline, 4-Acetamidophenyl, 2,2,2-Trifluorethyl, 5-Chlor-3-methylbenzothiazol-2-yl, N-Methoxycarbonyl-piperidin-3-yl, Thiophen-2-yl, Isoxazol-5-yl, Ethoxy, 2-Chlorpyridin-3-yl, Pyridin-3-yl, Benzyloxy, 5-Methylisoxazol-3-yl, 1-Adamantyl, 4-Chlorphenoxymethyl, 2,2-Dimethylethenyl, 2-Chloropyridine-5-methyl, 5,7-Dimethyl-1,3,4-triazaindolizin-2-yl, (S)-Camphan-1-yl, (R)-Camphan-1-yl oder 8-Chinolinyl steht;
R^{10"} für einen C₁₋₆-Alkylrest, einen C₃₋₇-Cycloalkylrest, einen substituierten oder unsubstituierten Arylrest oder einen gesättigten oder ungesättigten gegebenenfalls substituierten heterocyclischen Rest steht, über den der Rest der Formel (III) gegebenenfalls an den Rest des Konjugates gebunden ist;
R¹¹ für Wasserstoff, Methyl, Ethyl, Propyl, Isopropyl, Butyl, Isobutyl, t-Butyl, Pentyl, Isopentyl, Neopentyl, Hexyl, Cyclopropyl, Cyclopropylmethyl, Cyclobutyl, Cyclopentyl, Cyclohexyl, Cycloheptyl, 4-Methylcyclohexyl, 3,3,5-Trimethylcyclohexyl, 5-Methyl-2-hexyl, Phenyl, Benzyl, Tolyl oder ein substituiertes Derivat davon, C₁₋₄-Alkylamino-C₁₋₄-alkyl, C₁₋₄-Dialkyl-amino-C₁₋₄-alkyl, Amino-C₁₋₄-alkyl, C₁₋₄-Alkyloxy-C₁₋₄-alkyl, Dialkylamino-C₁₋₄-alkyl, Amino-C₁₋₄-alkyl, C₁₋₄-Alkyloxy-C₁₋₄-alkyl oder 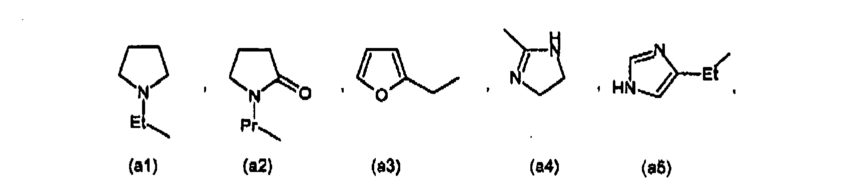 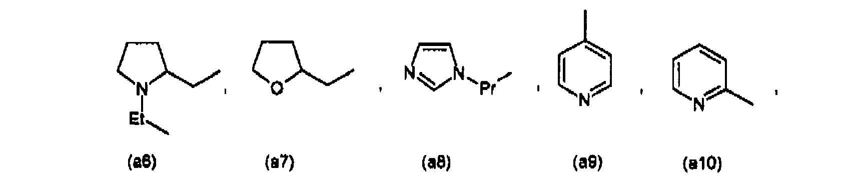 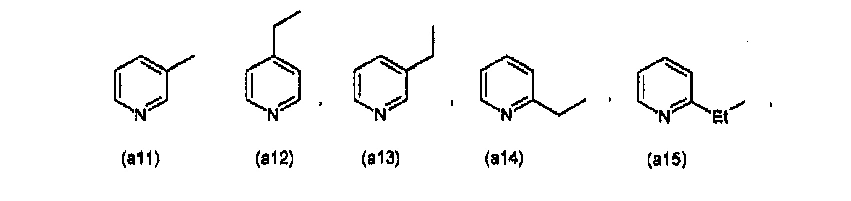 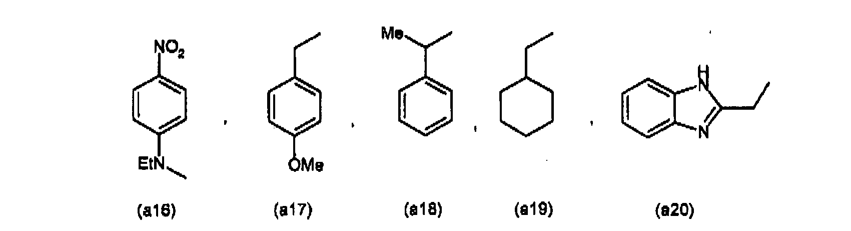 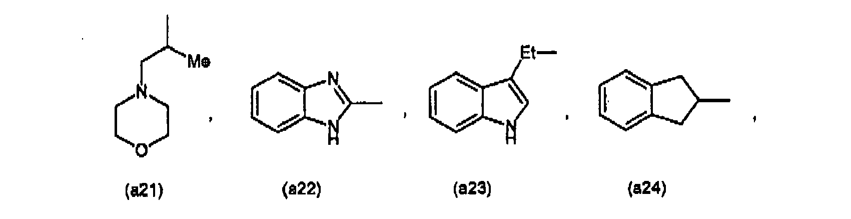 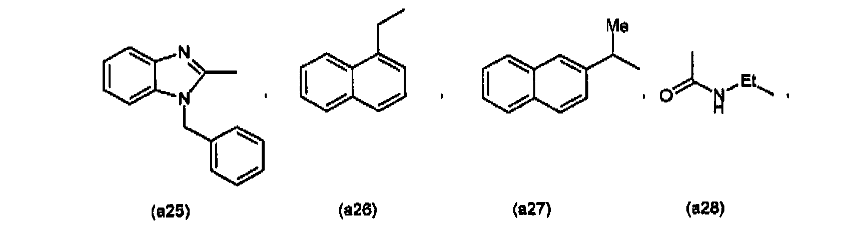 steht,
R¹⁶ für Wasserstoff, CN, Methyl, Ethyl, Propyl, Isopropyl, Butyl, Isobutyl, t-Butyl, Pentyl, Isopentyl, Neopentyl, Hexyl, Cyclopropyl, Cyclobutyl, Cyclopentyl, Cyclohexyl, Cycloheptyl, Methoxy, Trifluoromethoxy, Ethoxy, Propoxy, Butoxy, Pentoxy oder Hexoxy, Fluor, Chlor, Brom oder Iod steht;
R¹⁷ für Wasserstoff, CN, Methyl, Ethyl, Propyl, Isopropyl, Butyl, Isobutyl, t-Butyl, Pentyl, Isopentyl, Neopentyl, Hexyl, Cyclopropyl, Cyclobutyl, Cyclopentyl, Cyclohexyl, Cycloheptyl, Methoxy, Trifluormethoxy, Ethoxy, Propoxy, Butoxy, Pentoxy oder Hexoxy, Fluor, Chlor, Brom oder Iod steht;
R¹² für Wasserstoff, Methyl, Ethyl, Propyl, Isopropyl, Butyl, Isobutyl, t-Butyl, Pentyl, Isopentyl, Neopentyl, Hexyl, Cyclopropyl, Cyclopropylmethyl, Cyclobutyl, Cyclopentyl, Cyclohexyl, Cycloheptyl, 4-Methylcyclohexyl, 3,3,5-Trimethylcyclohexyl., 5-Methyl-2-hexyl, Phenyl, Benzyl, Tolyl oder ein substituiertes Derivat davon, C₁₋₄-Alkylamino-C₁₋₄-alkyl, C₁₋₄-Dialkyl-amino-C₁₋₄-alkyl, Amino-C₁₋₄-alkyl, C₁₋₄-Alkyloxy-C₁₋₄-alkyl, oder einen der Rest (a1) bis (a28);
X' für N, O oder S steht;
R¹³ fehlt, für Wasserstoff, Methyl, Ethyl, Propyl, Isopropyl, Butyl, Isobutyl, t-Butyl, Pentyl, Isopentyl, Neopentyl, Hexyl, Cyclopropyl, Cyclobutyl, Cyclopentyl, Cyclohexyl, Cycloheptyl, -NO₂, -CN, -COR^{7'}, oder -COOR^{7'} steht;
R^{13'} für Wasserstoff, Methyl, Ethyl, Propyl, Isopropyl, Butyl, Isobutyl, t-Butyl, Pentyl, Isopentyl, Neopentyl, Hexyl, Cyclopropyl, Cyclobutyl, Cyclopentyl, Cyclohexyl, Cycloheptyl, Phenyl, Benzyl, Tolyl oder einem substituierten Derivat davon steht;
R¹⁴ für Wasserstoff, Methyl, Ethyl, Propyl, Isopropyl, Butyl, Isobutyl, t-Butyl, Pentyl, Isopentyl, Neopentyl, Hexyl, Cyclopropyl, Cyclopropylmethyl, Cyclobutyl, Cyclopentyl, Cyclohexyl, Cycloheptyl, 4-Methylcyclohexyl, 3,3,5-Trimethylcyclohexyl, 5-Methyl-2-hexyl, Phenyl, Benzyl, Tolyl oder einem substituierten Derivat davon, C₁₋₄-Alkylamino-C₁₋₄-alkyl, C₁₋₄-Dialkyl-amino-C₁₋₄-alkyl, Amino-C₁₋₄-alkyl, C₁₋₄-Alkyloxy-C₁₋₄-alkyl, oder einem Rest (a1) oder (a28) steht; und
R¹⁵ für Wasserstoff, Methyl, Ethyl, Propyl, Isopropyl, Butyl, Isobutyl, t-Butyl, Pentyl, Isopentyl, Neopentyl, Hexyl, Cyclopropyl, Cyclopropylmethyl, Cyclobutyl, Cyclopentyl, Cyclohexyl, Cycloheptyl, 4-Methylcyclohexyl, 3,3,5-Trimethylcyclohexyl, 5-Methyl-2-hexyl, Phenyl, Benzyl, Tolyl oder einem substituierten Derivat davon, C₁₋₄-Alkylamino-C₁₋₄-alkyl, C₁₋₄Dialkyl-amino-C₁₋₄-alkyl, Amino-C₁₋₄-alkyl, C₁₋₄-Alkyloxy-C₁₋₄-alkyl, oder einem Rest (a1) bis (a28) steht,
und dessen physiologisch akzeptablen Salze und Stereoisomeren.

3. Verfahren zur Herstellung eines Konjugates gemäß Anspruch 1, durch
[A] Reaktion einer Verbindung der Formel (III), die eine freie oder gegebenenfalls aktivierte Carboxylfunktion besitzt, mit einer Verbindung der Formel (Ia), die eine freie primäre oder sekundäre Aminogruppe besitzt,
CT-AA1-AA2-AA3-AA4-Sp (Ia),
in der alle Reste die in Anspruch 1 angegebene Bedeutung haben, in Gegenwart einer Base;
oder
[B] Reaktion eine Verbindung der Formel (III), die eine freie primäre oder sekundäre Aminofunktion hat,
mit einem Carbonsäurederivat wie Phosgen, Thiophosgen oder einem Chlorameisensäureester, gegebenenfalls in Gegenwart einer Base,
gefolgt durch die Reaktion mit einer Verbindung der Formle (Ia), die eine freie primäre oder sekundäre Aminogruppe hat
CT-AA1-AA2-AA3-AA4-Sp (Ia)
in der alle Reste die in Anspruch 1 angegebene Bedeutung haben, und
gegebenenfalls Entfernung der Schutzgruppe und/oder Derivatisierung der Stickstoffatome, die an bevorzugten Stellen des
Herstellungsprozesses auftreten, und/oder Konversion der entstandenen Verbindungen in die freie Säure und/oder Konversion der entstandenen Verbindungen in eines ihrer physiologisch akzeptablen Salze durch Reaktion mit einer anorganischen oder organischen Base oder Säure;
oder
[C] Reaktion einer zytotoxischen Verbindung oder eines Zytostatikums oder eines Derivates eines Zytostatikums CT, das eine freie primäre oder sekundäre Aminogruppe enthält,
mit einem Carbonsäurederivat wie beispielsweise Phosgen, Thiophosgen oder einem Chlorameisensäureester in Gegenwart einer Base,
gefolgt von der Reaktion mit einer Verbindung der Formel (III), die eine freie primäre oder sekundäre Aminofunktion besitzt, und
gegebenenfalls Entfernung der Schutzgruppe und/oder Derivatisierung der Stickstoffatome, die an bevorzugten Stellen des
Herstellungsprozesses auftreten, und/oder Konversion der entstandenen Verbindungen in die freie Säure und/oder Konversion der entstandenen Verbindungen in eines ihrer physiologisch akzeptablen Salze durch Reaktion mit einer anorganischen oder organischen Base oder Säure;
oder
[D] Reaktion einer Verbindung der Formel (III), die eine freie primäre oder sekundäre Aminofunktion enthält,
mit einer Verbindung der Formel (Ia), die eine freie oder gegebenenfalls aktivierte Carboxylfunktion hat,
CT-AA1-AA2-AA3-AA4-Sp (Ia)
in der alle Reste die in Anspruch 1 angegebene Bedeutung haben,
in Gegenwart einer Base;
und
gegebenenfalls Entfernung der Schutzgruppe und/oder Derivatisierung der Stickstaffatome, die an bevorzugten Stellen des
Herstellungsprozesses auftreten, und/oder Konversion der entstandenen Verbindungen in die freie Säure und/oder Konversion der entstandenen Verbindungen in eines ihrer physiologisch akzeptablen Salze durch Reaktion mit einer anorganischen oder organischen Base oder Säure.

4. Verfahren gemäß Anspruch 3, **dadurch** gekennzeichet, dass alle Verfahrensstufen an der festen Phase durchgeführt werden.

5. Medikament umfassend mindestens ein Konjugat gemäß Anspruch 1 oder 2.

6. Verwendung einer Verbindung gemäß Anspruch 1 oder 2 zur Herstellung eines Medikaments zur Behandlung von karzinomatösen Erkrankungen.

## Revendications

1. Composé **caractérisé par** la formule (I)
CT-AA1-AA2-AA3-AA4-Sp-IA (I)
dans laquelle
CT dénote un radical cytotoxique ou un radical d'un cytostatique ou d'un dérivé cytostatique, qui peut comporter additionnellement un groupe hydroxyle, carboxyle ou amine
AA1 est absent ou est un acide aminé de configuration D ou L, portant de façon optionnelle des groupements protecteurs ou un radical Sp'
AA2 est absent ou est un acide aminé de configuration D ou L, portant de façon optionnelle des groupements protecteurs ou un radical Sp'
AA3 est absent ou est un acide aminé de configuration D ou L, portant de façon optionnelle des groupements protecteurs ou un radical Sp'
AA4 est absent ou est un acide aminé de configuration D ou L, portant de façon optionnelle des groupements protecteurs ou un radical Sp'
dans lequel
Sp' est un radical arylaminocarbonyle ou un arylaminothiocarbonyle ayant 7-11 atomes de carbones
Sp est absent, est un radical arylaminocarbonyle ou un arylaminothiocarbonyle ayant 7-11 atomes de carbone ou est un radical acide alkanedicarboxylique ayant 3 à 8 atomes de carbone ou un radical carbonyle ou thiocarbonyle
à condition qu'au moins un des radicaux AA1 à AA4 et/ou Sp soit présent,
IA est un radical non peptidique se liant au récepteur α_{ν}β₃ intégrine, qui est un radical de formule (III) 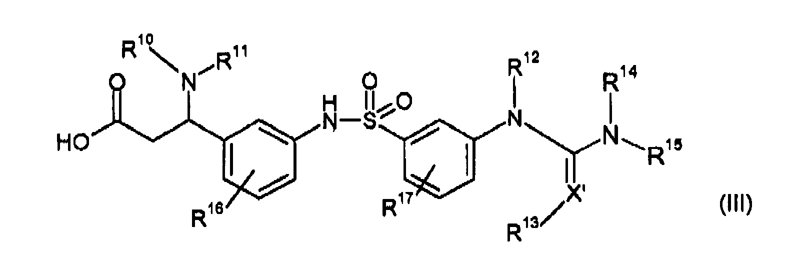 dans laquelle
R¹⁰ est -SO₂R^{10'}, -COOR^{10"}, -COR^{10'}, -CONR^{10'}₂ or -CS-NR^{10'}₂, ou représente une liaison directe par laquelle le radical de formule (III) est lié de façon optionnelle au reste du composé;
R^{10'} est, indépendamment l'un de l'autre, hydrogène, un radical alkyle substitué ou non-substitué ou cycloalkyle, un radical aryle substitué ou non-substitué ou un radical hétérocycle saturé ou insaturé, substitué de façon optionnelle, par lequel le radical de formule (III) est lié de facon optionelle au reste du composé;
R^{10"} est un radical alkyle substitué ou non-substitué ou cycloalkyle, un radical aryle substitué ou non-substitué ou un radical hétérocycle saturé ou insaturé, substitué de façon optionnelle, par lequel le radical de formule (III) est lié de facon optionnelle au reste du composé;
R¹¹ est hydrogène, radical alkyle substitué ou non-substitué ou cycloalkyle ou un radical aryle substitué ou non-substitué,
R¹⁶ est hydrogène, CN, radical alkyle substitué ou non-substitué ou cycloalkyle, un radical alkoxy substitué ou non-substitué ou un atome halogène,
R¹⁷ est hydrogène, CN, radical alkyle substitué ou non-substitué ou cycloalkyle, un radical alkoxy substitué ou non-substitué ou un atome halogène,
R¹² est hydrogène, un radical alkyle substitué ou non-substitué ou cycloalkyle, un radical aryle substitué ou non-substitué ou un radical hétérocycle saturé ou insaturé, substitué de façon optionnelle,
X' est N, O ou S;
R¹³ est absent H, un radical alkyle substitué ou non-substitué ou cycloalkyle, -NO₂, -CN, -COR^{13'}, -COOR^{13'};
R^{13'} est hydrogène, un radical alkyle substitué ou non-substitué ou cycloalkyle, un radical aryle substitué ou non-substitué ou un radical hétérocycle substitué de façon optionnelle qui peut être saturé ou insaturé, ou peut contenir des hétéroatomes supplémentaires;
R¹⁴ est hydrogène, un radical alkyle substitué ou non-substitué ou cycloalkyle, un radical aryle substitué ou non-substitué ou un radical hétérocycle saturé ou insaturé, substitué de façon optionnelle,
R¹⁵ est hydrogène, un radical alkyle substitué ou non-substitué ou cycloalkyle, un radical aryle substitué ou non-substitué ou un radical hétérocycle saturé ou insaturé, substitué de façon optionnelle, et leur sels et stéréoisomères physiologiquement acceptables,

2. Composé selon la revendication 1, charactérisé par
CT est camptothécine ou 9-aminocamptothécine, qui peut être lié au reste du composé par un groupe C20-OH ou dans le cas de la 9-aminocamptothécine, par le groupement amine libre;
AA1 est absent ou un acide aminé naturel de configuration D ou L, sélectionné du groupe se composant de glycine, alanine, valine, leucine, isoleucine et phénylalanine
AA2 est absent ou un acide aminé naturel de configuration D ou L, sélectionné du groupe se composant de lysine, glutamate, histidine, glycine, arginine, ornithine et leucine, and et peut porter des groupements protecteurs ou un radical Sp',
AA3 est absent ou un acide aminé naturel de configuration D' ou L, sélectionné du groupe se composant de glycine, alanine, valine, leucine, isoleucine et phénylalanine
AA4 est absent ou un acide aminé naturel de configuration D ou L, qui peut éventuellement porter des groupements protecteurs ou un radical Sp',
dans lequel
Sp' est un radical phénylaminocarbonyle ou phénylaminothio-carbonyle,
Sp est absent, est un radical phénylaminocarbonyle ou phénylaminothio-carbonyle, ou un radical acide alkanedicarboxylique ayant 3 à 6 atomes de carbones ou un radical carbonyle ou thiocarbonyle,
à condition qu'au moins un des radicaux AA1 à AA4 et / ou Sp soit présent,
IA est un radical non peptidique de formule (III) se liant au récepteur α_{ν}β₃ intégrine,
dans lequel
R¹⁰ est SO₂R^{10'}, -COOR^{10"}, -COR^{10'}, -CONR^{10'}₂ or -CSNR^{10'}₂ ou représente une liaison directe, par laquelle le radical de formule (III) est lié de façon optionnelle au reste du composé
R^{10'} est hydrogène, méthyle, éthyle, propyle, isopropyle, butyle, isobutyle, t-butyle, pentyle, isopentyle, neopentyle, hexyle, cyclopropyle, cyclo-butyle, cyclopentyle, cyclohexyle, cycloheptyle, phenyle, benzyle, tolyle ou un de leur dérivés substitués, -C₆H₂(CH₃)₃, -C₆(CH₃)₅, -CH₂C₆H₂(CH₃)₃, 2-chlorophényle, 3-chlorophényle, 4-chlorophényle, 2,3-dichlorophényle, 2,4-dichlorophényl, 3,4-dichlorophényl, 2,5-dichlorophényle, 3,5-dichlorophényle, 2,6-dichlorophényle, 4-chlorophénylméthyle, 2,4-dichloro-phényl-méthyle, 2,6-dichlorophénylméthyle, 3-aminophényle, 4-amino-phényle, 2-méthoxycarbonylphénylméthyle, 3-trifluorométhylphényle, 4-trifluorométhylphényle, 3,5-bis(trifluorométhyl)phényle, 4-trifluorométhoxyphényle, phénylméthyle, 2-acétamido-4-méthylthiazol-5-yle, phényléthyle, 1-phénylpropyle, (S)-(+)-camphor-10-yle, (R)-(-)-camphor-10-yle, 2-phényléthènyle, 2-thiophényle, 4-méthoxyphényle, 3,5-diméthoxyphényle, 3-méthylphényle, 4-méthylphényle, 4-t-butylphényle, 4-propyl-phényle, 2,5-diméthylphényle, 2-methoxy-5-méthylphényle, 2,3,5,6-tétraméthylphényle, 1-naphthyle, 2-naphthyle, 4-fluoro-phenyle, 2,4-difluorophényle, 2-chloro-6-méthylphényle, 2-chloro-4-fluorophényle, 2,5-diméthoxyphényle, 3,4-diméthoxyphényle, 3-chloro-6-méthoxyphényle, 2-trifluorométhylphényle, 2-alkylsulphonylphényle, 2-arylsulphonylphényle, 3-(N-acétyl-6-methoxy)aniline, 4-acetamidophényle, 2,2,2-tri-fluoroéthyle, 5-chloro-3-méthylbenzothiazol-2-yle, N-méth-oxycarbonyl-piperidin-3-yle, thiophén-2-yle, isoxazol-5-yle, éthoxy, 2-chloropyridin-3-yle, pyridin-3-yle, benzyloxy, 5-méthylisoxazol-3-yle, 1-adamantyle, 4-chlorophénoxyméthyle, 2,2-diméthyléthènyl, 2-chloropyridine-5-méthyle, 5,7-diméthyl-1,3,4-triazaindolizin-2-yle, (S)-camphan-1-yle, (R)-camphan-1-yle or 8-quinolinyle;
R^{10"} est un radical C1-6 alkyle, a radical C3-7 cycloalkyle, un radical aryle substitué ou non-substitué ou un radical hétérocycle saturé ou insaturé, substitué de façon optionnelle, par lequel le radical de formule (III) est optionnellement relié au composé;
R¹¹ est hydrogène, méthyle, éthyle, propyle, isopropyle, butyle, isobutyle, t-butyle, pentyle, isopentyle, néopentyle, hexyle, cyclopropyle, cyclo-propylméthyle, cyclobutyle, cyclopentyle, cyclohexyle, cyclo-heptyle, 4-méthylcyclohexyle, 3,3,5-triméthylcyclohexyle, 5-méthyl-2-hexyle, phényle, benzyle, tolyle ou un de leur derivé substitué, C₁₋₄-alkylamino-C₁₋₄-alkyle, C₁₋₄-dialkyl-amino-C₁₋₄-alkyle, amino-C₁₋₄-alkyle, C₁₋₄-alkyloxy-C₁₋₄-alkyle, dialkylamino-C₁₋₄-alkyle, amino-C₁₋₄-alkyle, C₁₋₄-alkyloxy-C₁₋₄-alkyle or 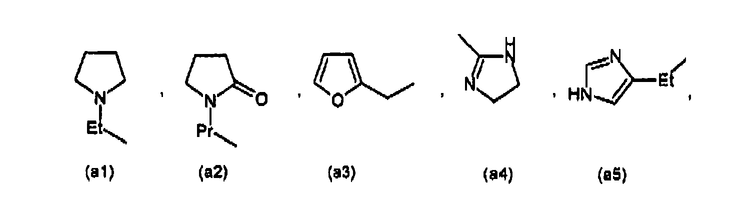 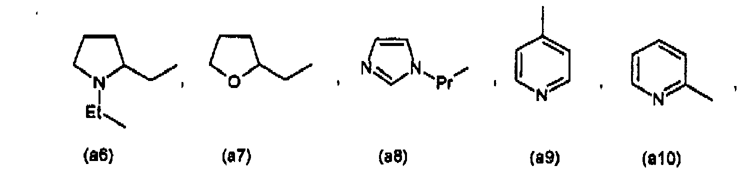 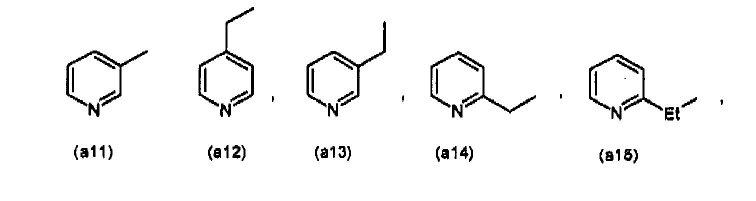 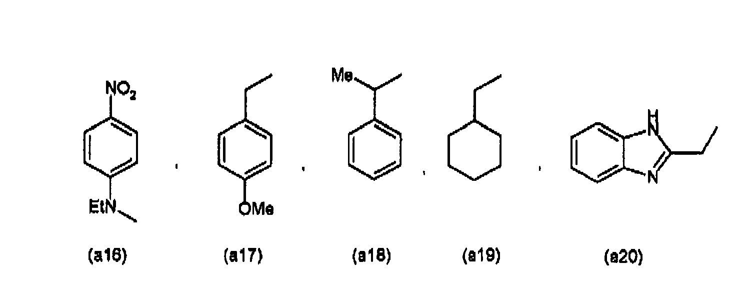 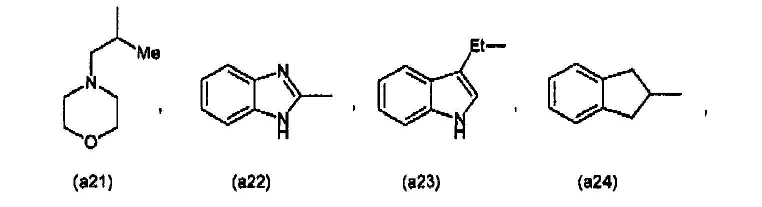 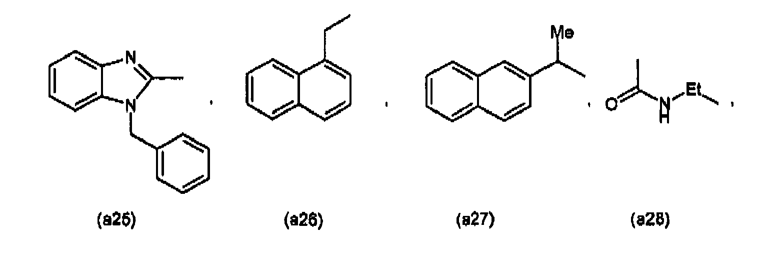 R¹⁶ est hydrogène, CN, méthyle, éthyle, propyle, isopropyle, butyle, isobutyle, t-butyle, pentyle, isopentyle, néopentyle, hexyle, cyclo-propyle, cyclobutyle, cyclopentyle, cyclohexyle, cycloheptyle, methoxy, trifluorométhoxy, éthoxy, propoxy, butoxy, pentoxy or hexoxy, fluoro, chloro, bromo or iodo
R¹⁷ est hydrogène, CN, méthyle, éthyle, propyle, isopropyle, butyle, isobutyle, t-butyle, pentyle, isopentyle, néopentyle, hexyle, cyclo-propyle, cyclobutyle, cyclopentyle, cyclohexyle, cycloheptyle, methoxy, trifluorométhoxy, éthoxy, propoxy, butoxy, pentoxy or hexoxy, fluoro, chloro, bromo or iodo
R¹² est hydrogène, méthyle, éthyle, propyle, isopropyle, butyle, isobutyle, t-butyle, pentyle, isopentyle, neopentyle, hexyle, cyclopropyle, cyclo-propylmethyel, cyclobutyle, cyclopentyle, cyclohexyle, cyclo-heptyle, 4-methylcyclohexyle, 3,3,5-triméthylcyclohexyle, 5-méthyl-2-hexyle, phényle, benzyle, tolyle ou un de leur dérivé substitué, C₁₋₄-alkylamino-C₁₋₄-alkyle, C₁₋₄-dialkyl-amino-C₁₋₄-alkyle, amino-C₁₋₄-alkyle, C₁₋₄-alkyloxy-C₁₋₄-alkyle, un des radicaux (a1) à (a28);
X' est N, O, ou S;
R¹³ est absent, -H, méthyle, éthyle, propyle, isopropyle, butyle, isobutyle, t-butyle, pentyle, isopentyle, néopentyle, hexyle, cyclo-propyle, cyclobutyle, cyclopentyle, cyclohexyle, cycloheptyle, -NO₂, -CN, -COR^{7'}, -COOR^{7'};
R^{13'} est hydrogène, méthyle, éthyle, propyle, isopropyle, butyle, isobutyle, t-butyle, pentyle, isopentyle, neopentyle, hexyle, cyclopropyle, cyclo-butyle, cyclopentyle, cyclohexyle, cycloheptyle, phenyle, benzyle, tolyle ou un de leur dérivé substitué;
R¹⁴ est hydrogène, méthyle, éthyle, propyle, isopropyle, butyle, isobutyle, t-butyle, pentyle, isopentyle, neopentyle, hexyle, cyclopropyle, cyclopropylmethyle, cyclobutyle, cyclopentyle, cyclohexyle, cycloheptyle, 4-méthylcyclohexyle, 3,3,5-tri-méthylcyclohexyle, 5-méthyl-2-hexyle, phényl, benzyle, tolyle ou un de leur dérivé substitué, C₁₋₄-alkylamino-C₁₋₄-alkyle, C₁₋₄-dialkyl-amino-C₁₋₄-alkyle, amino-C₁₋₄-alkyle, C₁₋₄-alkyloxy-C₁₋₄-alkyle, un des radicaux (a1) à (a28);
R¹⁵ est hydrogène, méthyle, éthyle, propyle, isopropyle, butyle, isobutyle, t-butyle, pentyle, isopentyle, neopentyle, hexyle, cyclopropyle, cyclopropylmethyle, cyclobutyle, cyclopentyle, cyclohexyle, cycloheptyle, 4-méthylcyclohexyle, 3,3,5-tri-méthylcyclohexyle, 5-méthyl-2-hexyle, phényl, benzyle, tolyle ou un de leur dérivé substitué, C₁₋₄-alkylamino-C₁₋₄-alkyle, C₁₋₄-dialkyl-amino-C₁₋₄-alkyle, amino-C₁₋₄-alkyle, C₁₋₄-alkyloxy-C₁₋₄-alkyle, un des radicaux (a1) à (a28); et leur sels et stéréoisomères physiologiquement acceptables.

3. Procédé de préparation des composés selon la revendication 1, comprenant
Process for the preparation of conjugates according to Claim 1, comprising
[A] la réaction d'un des composés de formule (III), qui a une fonction carboxyle libre ou optionnellement activée,
avec un composé de formule (Ia) qui a un groupe amine primaire ou secondaire libre
CT-AA1-AA2-AA3-AA4-Sp (Ia)
dans lequel tous les radicaux ont la définition indiquée dans la revendication 1,
en présence d'une base,
ou
[B] la réaction d'un composé de formule (III) qui a une fonction amine primaire ou secondaire libre,
avec un dérivé acide carbonique tel que, par exemple, phosgène, thiophosgène, ou un ester de l'acid chloroformique, si nécessaire en présence d'une base,
suivie de la réaction avec composé de formule (Ia) qui a un groupe amine primaire ou secondaire libre
CT-AA1-AA2-AA3-AA4-Sp (Ia)
dans lequel tous les radicaux ont la définition indiquée dans la revendication 1,
et
si nécessaire suppression des groupements protecteurs et / ou derivatization des atomes d'azote présent à des moments privilégiés au cours de la préparation et / ou conversion du composé obtenu en acide libre et / ou conversion du composé obtenu en l'un de ses sels physiologiques par réaction avec une base ou un acide organique ou inorganique;
ou
[C] la réaction d'un composé cytotoxique ou d'un cytostatique ou d'un dérivé cytostatique CT qui contient un groupe amine primaire ou secondaire libre,
avec un dérivé acide carbonique tel que, par exemple, phosgène, thiophosgène, ou un ester de l'acid chloroformique, si nécessaire en présence d'une base,
suivi de la réaction d'un composé de formule (III) qui a une fonction amine primaire ou secondaire libre,
et
si nécessaire suppression des groupements protecteurs et / ou dérivatisation des atomes d'azote présents à des moments privilégiés au cours de la préparation et / ou conversion du composé obtenu en acide libre et / ou conversion du composé obtenu en l'un de ses sels physiologiques par réaction avec une base ou un acide organique ou inorganique;
ou
[D] la réaction d'un composé de formule (III) qui contient une fonction amine primaire ou secondaire libre,
avec un composé de formule (Ia) qui contient une fonction carboxyle libre ou optionnellement activée,
CT-AA1-AA2-AA3-AA4-Sp (Ia)
dans laquelle tous les radicaux ont la définition indiquée dans la revendication 1,
en présence d'une base,
et
si nécessaire suppression des groupements protecteurs et / ou derivatization des atomes d'azote présent à des moments privilégiés au cours de la préparation et / ou conversion du composé obtenu en acide libre et / ou conversion du composé obtenu en l'un de ses sels physiologiques par réaction avec une base ou un acide organique ou inorganique;

4. Procédé suivant la revendication 3, charactérisé par le fait que toutes les étapes de synthèse sont effectuées sur une phase solide.

5. Médicament comprenant au moins un des composés suivant une des revendications 1 à 2.

6. Utilisation des composés suivant une des revendications 1 à 3 pour la production de médicaments pour le traitement de désordres carcinomiques.
